# EUROPEAN PATENT APPLICATION

(11) **EP 2 573 108 A1**
(43) Date of publication of application: **27.03.2013**
(21) Application number: 11182245.8
(22) Date of filing: 21.09.2011
(51) Int. Cl.: C07K 14/47, C12N 9/12

(54) **Methods for identifying compounds capable of inhibiting degradation of PKM zeta**

(71) Applicant: SYGNIS Bioscience GmbH & Co KG, 69120 Heidelberg (DE)
(72) Inventor: The designation of the inventor has not yet been filed
(74) Representative: Lahrtz, Fritz

(57) **Abstract**

The invention, among others relates to methods for identifying compounds capable of inhibiting degradation of PKM zeta, which e.g. comprise the steps of providing a test system for monitoring the degradation of PKM zeta, and determining whether a given test compound leads to a reduced degradation of PKM zeta in said test system; and/or comprise a step of determining whether a given test compound is capable of interfering with the proteasomal degradation of PKM zeta; or comprises a step of determining whether a given test compound is capable of interfering with the binding of a certain peptide, peptide derivative or peptide conjugate thereof to PKM zeta.

SEQUENCE LISTING

<110>  SYGNIS Bioscience GmbH & Co. KG

<120>  Methods for identifying compounds capable of inhibiting
degradation of PKM zeta

<130>  S67832EP

<160>  58

<170>  PatentIn version 3.5

<210>  1
<211>  3342
<212>  DNA
<213>  Homo sapiens


<220>
<221>  CDS
<222>  (1)..(3342)

<400>  1
atg ccc cgg ccg gag ctg ccc ctg ccg gag ggc tgg gag gag gcg cgc        48
Met Pro Arg Pro Glu Leu Pro Leu Pro Glu Gly Trp Glu Glu Ala Arg
1               5                   10                  15

gac ttc gac ggc aag gtc tac tac ata gac cac acg aac cgc acc acc        96
Asp Phe Asp Gly Lys Val Tyr Tyr Ile Asp His Thr Asn Arg Thr Thr
            20                  25                  30

agc tgg atc gac ccg cgg gac agg tac acc aaa ccg ctc acc ttt gct       144
Ser Trp Ile Asp Pro Arg Asp Arg Tyr Thr Lys Pro Leu Thr Phe Ala
        35                  40                  45

gac tgc att agt gat gag ttg ccg cta gga tgg gaa gag gca tat gac       192
Asp Cys Ile Ser Asp Glu Leu Pro Leu Gly Trp Glu Glu Ala Tyr Asp
    50                  55                  60

cca cag gtt gga gat tac ttc ata gac cac aac acc aaa acc act cag       240
Pro Gln Val Gly Asp Tyr Phe Ile Asp His Asn Thr Lys Thr Thr Gln
65                  70                  75                  80

att gag gat cct cga gta caa tgg cgg cgg gag cag gaa cat atg ctg       288
Ile Glu Asp Pro Arg Val Gln Trp Arg Arg Glu Gln Glu His Met Leu
                85                  90                  95

aag gat tac ctg gtg gtg gcc cag gag gct ctg agt gca caa aag gag       336
Lys Asp Tyr Leu Val Val Ala Gln Glu Ala Leu Ser Ala Gln Lys Glu
            100                 105                 110

atc tac cag gtg aag cag cag cgc ctg gag ctt gca cag cag gag tac       384
Ile Tyr Gln Val Lys Gln Gln Arg Leu Glu Leu Ala Gln Gln Glu Tyr
        115                 120                 125

cag caa ctg cat gcc gtc tgg gag cat aag ctg ggc tcc cag gtc agc       432
Gln Gln Leu His Ala Val Trp Glu His Lys Leu Gly Ser Gln Val Ser
    130                 135                 140

ttg gtc tct ggt tca tca tcc agc tcc aag tat gac cct gag atc ctg 480
Leu Val Ser Gly Ser Ser Ser Ser Ser Lys Tyr Asp Pro Glu Ile Leu
145                 150                 155                 160

aaa gct gaa att gcc act gca aaa tcc cgg gtc aac aag ctg aag aga 528
Lys Ala Glu Ile Ala Thr Ala Lys Ser Arg Val Asn Lys Leu Lys Arg
                165                 170                 175

gag atg gtt cac ctc cag cac gag ctg cag ttc aaa gag cgt ggc ttt 576
Glu Met Val His Leu Gln His Glu Leu Gln Phe Lys Glu Arg Gly Phe
            180                 185                 190

cag acc ctg aag aaa atc gat aag aaa atg tct gat gct cag ggc agc 624
Gln Thr Leu Lys Lys Ile Asp Lys Lys Met Ser Asp Ala Gln Gly Ser
        195                 200                 205

tac aaa ctg gat gaa gct cag gct gtc ttg aga gaa aca aaa gcc atc 672
Tyr Lys Leu Asp Glu Ala Gln Ala Val Leu Arg Glu Thr Lys Ala Ile
    210                 215                 220

aaa aag gct att acc tgt ggg gaa aag gaa aag caa gat ctc att aag 720
Lys Lys Ala Ile Thr Cys Gly Glu Lys Glu Lys Gln Asp Leu Ile Lys
225                 230                 235                 240

agc ctt gcc atg ttg aag gac ggc ttc cgc act gac agg ggg tct cac 768
Ser Leu Ala Met Leu Lys Asp Gly Phe Arg Thr Asp Arg Gly Ser His
                245                 250                 255

tca gac ctg tgg tcc agc agc agc tct ctg gag agt tcg agt ttc ccg 816
Ser Asp Leu Trp Ser Ser Ser Ser Ser Leu Glu Ser Ser Ser Phe Pro
            260                 265                 270

cta ccg aaa cag tac ctg gat gtg agc tcc cag aca gac atc tcg gga 864
Leu Pro Lys Gln Tyr Leu Asp Val Ser Ser Gln Thr Asp Ile Ser Gly
        275                 280                 285

agc ttc ggc atc aac agc aac aat cag ttg gca gag aag gtc aga ttg 912
Ser Phe Gly Ile Asn Ser Asn Asn Gln Leu Ala Glu Lys Val Arg Leu
    290                 295                 300

cgc ctt cga tat gaa gag gct aag aga agg atc gcc aac ctg aag atc 960
Arg Leu Arg Tyr Glu Glu Ala Lys Arg Arg Ile Ala Asn Leu Lys Ile
305                 310                 315                 320

cag ctg gcc aag ctt gac agt gag gcc tgg cct ggg gtg ctg gac tca 1008
Gln Leu Ala Lys Leu Asp Ser Glu Ala Trp Pro Gly Val Leu Asp Ser
                325                 330                 335

gag agg gac cgg ctg atc ctt atc aac gag aag gag gag ctg ctg aag 1056
Glu Arg Asp Arg Leu Ile Leu Ile Asn Glu Lys Glu Glu Leu Leu Lys
            340                 345                 350

gag atg cgc ttc atc agc ccc cgc aag tgg acc cag ggg gag gtg gag 1104
Glu Met Arg Phe Ile Ser Pro Arg Lys Trp Thr Gln Gly Glu Val Glu
        355                 360                 365

cag ctg gag atg gcc cgg aag cgg ctg gaa aag gac ctg cag gca gcc 1152
Gln Leu Glu Met Ala Arg Lys Arg Leu Glu Lys Asp Leu Gln Ala Ala
    370                 375                 380

cgg gac acc cag agc aag gcg ctg acg gag agg tta aag tta aac agt          1200
Arg Asp Thr Gln Ser Lys Ala Leu Thr Glu Arg Leu Lys Leu Asn Ser
385                 390                 395                 400

aag agg aac cag ctt gtg aga gaa ctg gag gaa gcc acc cgg cag gtg          1248
Lys Arg Asn Gln Leu Val Arg Glu Leu Glu Glu Ala Thr Arg Gln Val
                405                 410                 415

gca act ctg cac tcc cag ctg aaa agt ctc tca agc agc atg cag tcc          1296
Ala Thr Leu His Ser Gln Leu Lys Ser Leu Ser Ser Ser Met Gln Ser
            420                 425                 430

ctg tcc tca ggc agc agc ccc gga tcc ctc acg tcc agc cgg ggc tcc          1344
Leu Ser Ser Gly Ser Ser Pro Gly Ser Leu Thr Ser Ser Arg Gly Ser
        435                 440                 445

ctg gtt gca tcc agc ctg gac tcc tcc act tca gcc agc ttc act gac          1392
Leu Val Ala Ser Ser Leu Asp Ser Ser Thr Ser Ala Ser Phe Thr Asp
    450                 455                 460

ctc tac tat gac ccc ttt gag cag ctg gac tca gag ctg cag agc aag          1440
Leu Tyr Tyr Asp Pro Phe Glu Gln Leu Asp Ser Glu Leu Gln Ser Lys
465                 470                 475                 480

gtg gag ttc ctg ctc ctg gag ggg gcc acc ggc ttc cgg ccc tca ggc          1488
Val Glu Phe Leu Leu Leu Glu Gly Ala Thr Gly Phe Arg Pro Ser Gly
                485                 490                 495

tgc atc acc acc atc cac gag gat gag gtg gcc aag acc cag aag gca          1536
Cys Ile Thr Thr Ile His Glu Asp Glu Val Ala Lys Thr Gln Lys Ala
            500                 505                 510

gag gga ggt ggc cgc ctg cag gct ctg cgt tcc ctg tct ggc acc cca          1584
Glu Gly Gly Gly Arg Leu Gln Ala Leu Arg Ser Leu Ser Gly Thr Pro
        515                 520                 525

aag tcc atg acc tcc cta tcc cca cgt tcc tct ctc tcc tcc ccc tcc          1632
Lys Ser Met Thr Ser Leu Ser Pro Arg Ser Ser Leu Ser Ser Pro Ser
    530                 535                 540

cca ccc tgt tcc cct ctc atg gct gac ccc ctc ctg gct ggt gat gcc          1680
Pro Pro Cys Ser Pro Leu Met Ala Asp Pro Leu Leu Ala Gly Asp Ala
545                 550                 555                 560

ttc ctc aac tcc ttg gag ttt gaa gac ccg gag ctg agt gcc act ctt          1728
Phe Leu Asn Ser Leu Glu Phe Glu Asp Pro Glu Leu Ser Ala Thr Leu
                565                 570                 575

tgt gaa ctg agc ctt ggt aac agc gcc cag gaa aga tac cgg ctg gag          1776
Cys Glu Leu Ser Leu Gly Asn Ser Ala Gln Glu Arg Tyr Arg Leu Glu
            580                 585                 590

gaa cca gga acg gag ggc aag cag ctg ggc caa gct gtg aat acg gcc          1824
Glu Pro Gly Thr Glu Gly Lys Gln Leu Gly Gln Ala Val Asn Thr Ala
        595                 600                 605

cag ggg tgt ggc ctg aaa gtg gcc tgt gtc tca gcc gcc gta tcg gac          1872
Gln Gly Cys Gly Leu Lys Val Ala Cys Val Ser Ala Ala Val Ser Asp
    610                 615                 620

gag tca gtg gct gga gac agt ggt gtg tac gag gct tcc gtg cag aga 1920
Glu Ser Val Ala Gly Asp Ser Gly Val Tyr Glu Ala Ser Val Gln Arg
625                 630                 635                 640

ctg ggt gct tca gaa gct gct gca ttt gac agt gac gaa tcg gaa gca 1968
Leu Gly Ala Ser Glu Ala Ala Ala Phe Asp Ser Asp Glu Ser Glu Ala
                645                 650                 655

gtg ggt gcg acc cga att cag att gcc ctg aag tat gat gag aag aat 2016
Val Gly Ala Thr Arg Ile Gln Ile Ala Leu Lys Tyr Asp Glu Lys Asn
            660                 665                 670

aag caa ttt gca ata tta atc atc cag ctg agt aac ctt tct gct ctg 2064
Lys Gln Phe Ala Ile Leu Ile Ile Gln Leu Ser Asn Leu Ser Ala Leu
        675                 680                 685

ttg cag caa caa gac cag aaa gtg aat atc cgc gtg gct gtc ctt cct 2112
Leu Gln Gln Gln Asp Gln Lys Val Asn Ile Arg Val Ala Val Leu Pro
    690                 695                 700

tgc tct gaa agc aca acc tgc ctg ttc cgg acc cgg cct ctg gac gcc 2160
Cys Ser Glu Ser Thr Thr Cys Leu Phe Arg Thr Arg Pro Leu Asp Ala
705                 710                 715                 720

tca gac act cta gtg ttc aat gag gtg ttc tgg gta tcc atg tcc tat 2208
Ser Asp Thr Leu Val Phe Asn Glu Val Phe Trp Val Ser Met Ser Tyr
                725                 730                 735

cca gcc ctt cac cag aag acc tta aga gtc gat gtc tgt acc acc gac 2256
Pro Ala Leu His Gln Lys Thr Leu Arg Val Asp Val Cys Thr Thr Asp
            740                 745                 750

agg agc cat ctg gaa gag tgc ctg gga ggc gcc cag atc agc ctg gcg 2304
Arg Ser His Leu Glu Glu Cys Leu Gly Gly Ala Gln Ile Ser Leu Ala
        755                 760                 765

gag gtc tgc cgg tct ggg gag agg tcg act cgc tgg tac aac ctt ctc 2352
Glu Val Cys Arg Ser Gly Glu Arg Ser Thr Arg Trp Tyr Asn Leu Leu
    770                 775                 780

agc tac aaa tac ttg aag aaa cag agc agg gag ctc aag cca gtg gga 2400
Ser Tyr Lys Tyr Leu Lys Lys Gln Ser Arg Glu Leu Lys Pro Val Gly
785                 790                 795                 800

gtc atg gcc cct gcc tca ggg cct gcc agc acg gac gct gtg tct gct 2448
Val Met Ala Pro Ala Ser Gly Pro Ala Ser Thr Asp Ala Val Ser Ala
                805                 810                 815

ctg ttg gaa cag aca gca gtg gag ctg gag aag agg cag gag ggc agg 2496
Leu Leu Glu Gln Thr Ala Val Glu Leu Glu Lys Arg Gln Glu Gly Arg
            820                 825                 830

agc agc aca cag aca ctg gaa gac agc tgg agg tat gag gag acc agt 2544
Ser Ser Thr Gln Thr Leu Glu Asp Ser Trp Arg Tyr Glu Glu Thr Ser
        835                 840                 845

gag aat gag gca gta gcc gag gaa gag gag gag gag gtg gag gag gag 2592
Glu Asn Glu Ala Val Ala Glu Glu Glu Glu Glu Glu Val Glu Glu Glu
    850                 855                 860

gag gga gaa gag gat gtt ttc acc gag aaa gcc tca cct gat atg gat 2640
Glu Gly Glu Glu Asp Val Phe Thr Glu Lys Ala Ser Pro Asp Met Asp
865                 870                 875                 880

ggg tac cca gca tta aag gtg gac aaa gag acc aac acg gag acc ccg 2688
Gly Tyr Pro Ala Leu Lys Val Asp Lys Glu Thr Asn Thr Glu Thr Pro
                885                 890                 895

gcc cca tcc ccc aca gtg gtg cga cct aag gac cgg aga gtg ggc acc 2736
Ala Pro Ser Pro Thr Val Val Arg Pro Lys Asp Arg Arg Val Gly Thr
            900                 905                 910

ccg tcc cag ggg cca ttt ctt cga ggg agc acc atc atc cgc tct aag 2784
Pro Ser Gln Gly Pro Phe Leu Arg Gly Ser Thr Ile Ile Arg Ser Lys
        915                 920                 925

acc ttc tcc cca gga ccc cag agc cag tac gtg tgc cgg ctg aat cgg 2832
Thr Phe Ser Pro Gly Pro Gln Ser Gln Tyr Val Cys Arg Leu Asn Arg
    930                 935                 940

agt gat agt gac agc tcc act ctg tcc aaa aag cca cct ttt gtt cga 2880
Ser Asp Ser Asp Ser Ser Thr Leu Ser Lys Lys Pro Pro Phe Val Arg
945                 950                 955                 960

aac tcc ctg gag cga cgc agc gtc cgg atg aag cgg cct tcc tcg gtc 2928
Asn Ser Leu Glu Arg Arg Ser Val Arg Met Lys Arg Pro Ser Ser Val
                965                 970                 975

aag tcg ctg cgc tcc gag cgt ctg atc cgt acc tcg ctg gac ctg gag 2976
Lys Ser Leu Arg Ser Glu Arg Leu Ile Arg Thr Ser Leu Asp Leu Glu
            980                 985                 990

tta gac ctg cag gcg aca aga acc tgg cac agc caa ctg acc cag gag 3024
Leu Asp Leu Gln Ala Thr Arg Thr Trp His Ser Gln Leu Thr Gln Glu
        995                 1000                1005

atc tcg gtg ctg aag gag ctc aag gag cag ctg gaa caa gcc aag 3069
Ile Ser Val Leu Lys Glu Leu Lys Glu Gln Leu Glu Gln Ala Lys
    1010                1015                1020

agc cac ggg gag aag gag ctg cca cag tgg ttg cgt gag gac gag 3114
Ser His Gly Glu Lys Glu Leu Pro Gln Trp Leu Arg Glu Asp Glu
    1025                1030                1035

cgt ttc cgc ctg ctg ctg agg atg ctg gag aag cgg cag atg gac 3159
Arg Phe Arg Leu Leu Leu Arg Met Leu Glu Lys Arg Gln Met Asp
    1040                1045                1050

cga gcg gag cac aag ggt gag ctt cag aca gac aag atg atg agg 3204
Arg Ala Glu His Lys Gly Glu Leu Gln Thr Asp Lys Met Met Arg
    1055                1060                1065

gca gct gcc aag gat gtg cac agg ctc cga ggc cag agc tgt aag 3249
Ala Ala Ala Lys Asp Val His Arg Leu Arg Gly Gln Ser Cys Lys
    1070                1075                1080

gaa ccc cca gaa gtt cag tct ttc agg gag aag atg gca ttt ttc 3294
Glu Pro Pro Glu Val Gln Ser Phe Arg Glu Lys Met Ala Phe Phe
    1085                1090                1095

acc cgg cct cgg atg aat atc cca gct ctc tct gca gat gac gtc 3339
Thr Arg Pro Arg Met Asn Ile Pro Ala Leu Ser Ala Asp Asp Val
    1100                1105                1110

taa 3342

<210> 2
<211> 1113
<212> PRT
<213> Homo sapiens

<400> 2

Met Pro Arg Pro Glu Leu Pro Leu Pro Glu Gly Trp Glu Glu Ala Arg
1               5                   10                  15

Asp Phe Asp Gly Lys Val Tyr Tyr Ile Asp His Thr Asn Arg Thr Thr
            20                  25                  30

Ser Trp Ile Asp Pro Arg Asp Arg Tyr Thr Lys Pro Leu Thr Phe Ala
        35                  40                  45

Asp Cys Ile Ser Asp Glu Leu Pro Leu Gly Trp Glu Glu Ala Tyr Asp
    50                  55                  60

Pro Gln Val Gly Asp Tyr Phe Ile Asp His Asn Thr Lys Thr Thr Gln
65                  70                  75                  80

Ile Glu Asp Pro Arg Val Gln Trp Arg Arg Glu Gln Glu His Met Leu
                85                  90                  95

Lys Asp Tyr Leu Val Val Ala Gln Glu Ala Leu Ser Ala Gln Lys Glu
            100                 105                 110

Ile Tyr Gln Val Lys Gln Gln Arg Leu Glu Leu Ala Gln Gln Glu Tyr
        115                 120                 125

Gln Gln Leu His Ala Val Trp Glu His Lys Leu Gly Ser Gln Val Ser
    130                 135                 140

Leu Val Ser Gly Ser Ser Ser Ser Ser Lys Tyr Asp Pro Glu Ile Leu
145                 150                 155                 160

Lys Ala Glu Ile Ala Thr Ala Lys Ser Arg Val Asn Lys Leu Lys Arg
                165                 170                 175

Glu Met Val His Leu Gln His Glu Leu Gln Phe Lys Glu Arg Gly Phe
            180                 185                 190

Gln Thr Leu Lys Lys Ile Asp Lys Lys Met Ser Asp Ala Gln Gly Ser
        195                 200                 205

Tyr Lys Leu Asp Glu Ala Gln Ala Val Leu Arg Glu Thr Lys Ala Ile
    210                 215                 220

Lys Lys Ala Ile Thr Cys Gly Glu Lys Glu Lys Gln Asp Leu Ile Lys
225                 230                 235                 240

Ser Leu Ala Met Leu Lys Asp Gly Phe Arg Thr Asp Arg Gly Ser His
                245                 250                 255

Ser Asp Leu Trp Ser Ser Ser Ser Ser Leu Glu Ser Ser Ser Phe Pro
            260                 265                 270

Leu Pro Lys Gln Tyr Leu Asp Val Ser Ser Gln Thr Asp Ile Ser Gly
        275                 280                 285

Ser Phe Gly Ile Asn Ser Asn Asn Gln Leu Ala Glu Lys Val Arg Leu
    290                 295                 300

Arg Leu Arg Tyr Glu Glu Ala Lys Arg Arg Ile Ala Asn Leu Lys Ile
305                 310                 315                 320

Gln Leu Ala Lys Leu Asp Ser Glu Ala Trp Pro Gly Val Leu Asp Ser
                325                 330                 335

Glu Arg Asp Arg Leu Ile Leu Ile Asn Glu Lys Glu Glu Leu Leu Lys
            340                 345                 350

Glu Met Arg Phe Ile Ser Pro Arg Lys Trp Thr Gln Gly Glu Val Glu
        355                 360                 365

Gln Leu Glu Met Ala Arg Lys Arg Leu Glu Lys Asp Leu Gln Ala Ala
    370                 375                 380

Arg Asp Thr Gln Ser Lys Ala Leu Thr Glu Arg Leu Lys Leu Asn Ser
385                 390                 395                 400

Lys Arg Asn Gln Leu Val Arg Glu Leu Glu Glu Ala Thr Arg Gln Val
                405                 410                 415

Ala Thr Leu His Ser Gln Leu Lys Ser Leu Ser Ser Ser Met Gln Ser
            420                 425                 430

Leu Ser Ser Gly Ser Ser Pro Gly Ser Leu Thr Ser Ser Arg Gly Ser
        435                 440                 445

Leu Val Ala Ser Ser Leu Asp Ser Ser Thr Ser Ala Ser Phe Thr Asp
    450                 455                 460

Leu Tyr Tyr Asp Pro Phe Glu Gln Leu Asp Ser Glu Leu Gln Ser Lys
465                 470                 475                 480

Val Glu Phe Leu Leu Leu Glu Gly Ala Thr Gly Phe Arg Pro Ser Gly
                485                 490                 495

Cys Ile Thr Thr Ile His Glu Asp Glu Val Ala Lys Thr Gln Lys Ala
            500                 505                 510

Glu Gly Gly Gly Arg Leu Gln Ala Leu Arg Ser Leu Ser Gly Thr Pro
        515                 520                 525

Lys Ser Met Thr Ser Leu Ser Pro Arg Ser Ser Leu Ser Ser Pro Ser
    530                 535                 540

Pro Pro Cys Ser Pro Leu Met Ala Asp Pro Leu Leu Ala Gly Asp Ala
545                 550                 555                 560

Phe Leu Asn Ser Leu Glu Phe Glu Asp Pro Glu Leu Ser Ala Thr Leu
                565                 570                 575

Cys Glu Leu Ser Leu Gly Asn Ser Ala Gln Glu Arg Tyr Arg Leu Glu
            580                 585                 590

Glu Pro Gly Thr Glu Gly Lys Gln Leu Gly Gln Ala Val Asn Thr Ala
        595                 600                 605

Gln Gly Cys Gly Leu Lys Val Ala Cys Val Ser Ala Ala Val Ser Asp
    610                 615                 620

Glu Ser Val Ala Gly Asp Ser Gly Val Tyr Glu Ala Ser Val Gln Arg
625                 630                 635                 640

Leu Gly Ala Ser Glu Ala Ala Ala Phe Asp Ser Asp Glu Ser Glu Ala
                645                 650                 655

Val Gly Ala Thr Arg Ile Gln Ile Ala Leu Lys Tyr Asp Glu Lys Asn
            660                 665                 670

Lys Gln Phe Ala Ile Leu Ile Ile Gln Leu Ser Asn Leu Ser Ala Leu
        675                 680                 685

Leu Gln Gln Gln Asp Gln Lys Val Asn Ile Arg Val Ala Val Leu Pro
    690                 695                 700

Cys Ser Glu Ser Thr Thr Cys Leu Phe Arg Thr Arg Pro Leu Asp Ala
705                 710                 715                 720

Ser Asp Thr Leu Val Phe Asn Glu Val Phe Trp Val Ser Met Ser Tyr
                725                 730                 735

Pro Ala Leu His Gln Lys Thr Leu Arg Val Asp Val Cys Thr Thr Asp
            740                 745                 750

Arg Ser His Leu Glu Glu Cys Leu Gly Gly Ala Gln Ile Ser Leu Ala
        755                 760                 765

Glu Val Cys Arg Ser Gly Glu Arg Ser Thr Arg Trp Tyr Asn Leu Leu
    770                 775                 780

Ser Tyr Lys Tyr Leu Lys Lys Gln Ser Arg Glu Leu Lys Pro Val Gly
785                 790                 795                 800

Val Met Ala Pro Ala Ser Gly Pro Ala Ser Thr Asp Ala Val Ser Ala
                805                 810                 815

Leu Leu Glu Gln Thr Ala Val Glu Leu Glu Lys Arg Gln Glu Gly Arg
            820                 825                 830

Ser Ser Thr Gln Thr Leu Glu Asp Ser Trp Arg Tyr Glu Glu Thr Ser
        835                 840                 845

Glu Asn Glu Ala Val Ala Glu Glu Glu Glu Glu Glu Val Glu Glu Glu
    850                 855                 860

Glu Gly Glu Glu Asp Val Phe Thr Glu Lys Ala Ser Pro Asp Met Asp
865                 870                 875                 880

Gly Tyr Pro Ala Leu Lys Val Asp Lys Glu Thr Asn Thr Glu Thr Pro
                885                 890                 895

Ala Pro Ser Pro Thr Val Val Arg Pro Lys Asp Arg Arg Val Gly Thr
            900                 905                 910

Pro Ser Gln Gly Pro Phe Leu Arg Gly Ser Thr Ile Ile Arg Ser Lys
        915                 920                 925

Thr Phe Ser Pro Gly Pro Gln Ser Gln Tyr Val Cys Arg Leu Asn Arg
    930                 935                 940

Ser Asp Ser Asp Ser Ser Thr Leu Ser Lys Lys Pro Pro Phe Val Arg
945                 950                 955                 960

Asn Ser Leu Glu Arg Arg Ser Val Arg Met Lys Arg Pro Ser Ser Val
                965                 970                 975

Lys Ser Leu Arg Ser Glu Arg Leu Ile Arg Thr Ser Leu Asp Leu Glu
            980                 985                 990

Leu Asp Leu Gln Ala Thr Arg Thr Trp His Ser Gln Leu Thr Gln Glu
        995                 1000                1005

Ile Ser Val Leu Lys Glu Leu Lys Glu Gln Leu Glu Gln Ala Lys
    1010                1015                1020

Ser His Gly Glu Lys Glu Leu Pro Gln Trp Leu Arg Glu Asp Glu
    1025                1030                1035

Arg Phe Arg Leu Leu Leu Arg Met Leu Glu Lys Arg Gln Met Asp
    1040                1045                1050

Arg Ala Glu His Lys Gly Glu Leu Gln Thr Asp Lys Met Met Arg
    1055                1060                1065

Ala Ala Ala Lys Asp Val His Arg Leu Arg Gly Gln Ser Cys Lys
    1070                1075                1080

Glu Pro Pro Glu Val Gln Ser Phe Arg Glu Lys Met Ala Phe Phe
    1085                1090                1095

Thr Arg Pro Arg Met Asn Ile Pro Ala Leu Ser Ala Asp Asp Val
    1100                1105                1110

<210> 3
<211> 39
<212> DNA
<213> Artificial Sequence

<220>

<223>  nucleic acid encoding artificial peptide


<220>
<221>  CDS
<222>  (1)..(39)

<400>  3
ttt gtt cga aac tcc ctg gag cga cgc agc gtc cgg atg                    39
Phe Val Arg Asn Ser Leu Glu Arg Arg Ser Val Arg Met
1               5                   10


<210>  4
<211>  13
<212>  PRT
<213>  Artificial Sequence

<220>
<223>  Synthetic Construct

<400>  4

Phe Val Arg Asn Ser Leu Glu Arg Arg Ser Val Arg Met
1               5                   10


<210>  5
<211>  45
<212>  DNA
<213>  Artificial Sequence

<220>
<223>  nucleic acid encoding artificial peptide


<220>
<221>  CDS
<222>  (1)..(45)

<400>  5
cca cct ttt gtt cga aac tcc ctg gag cga cgc agc gtc cgg atg            45
Pro Pro Phe Val Arg Asn Ser Leu Glu Arg Arg Ser Val Arg Met
1               5                   10                  15


<210>  6
<211>  15
<212>  PRT
<213>  Artificial Sequence

<220>
<223>  Synthetic Construct

<400>  6

Pro Pro Phe Val Arg Asn Ser Leu Glu Arg Arg Ser Val Arg Met
1               5                   10                  15

<210>  7
<211>  69
<212>  DNA
<213>  Artificial Sequence

<220>
<223>  nucleic acid encoding artificial peptide


<220>
<221>  CDS
<222>  (1)..(69)

<400>  7
gac agc tcc act ctg tcc aaa aag cca cct ttt gtt cga aac tcc ctg          48
Asp Ser Ser Thr Leu Ser Lys Lys Pro Pro Phe Val Arg Asn Ser Leu
1               5                   10                  15

gag cga cgc agc gtc cgg atg                                              69
Glu Arg Arg Ser Val Arg Met
            20


<210>  8
<211>  23
<212>  PRT
<213>  Artificial Sequence

<220>
<223>  Synthetic Construct

<400>  8

Asp Ser Ser Thr Leu Ser Lys Lys Pro Pro Phe Val Arg Asn Ser Leu
1               5                   10                  15


Glu Arg Arg Ser Val Arg Met
            20


<210>  9
<211>  75
<212>  DNA
<213>  Artificial Sequence

<220>
<223>  nucleic acid encoding artificial peptide


<220>
<221>  CDS
<222>  (1)..(75)

<400>  9
gat agt gac agc tcc act ctg tcc aaa aag cca cct ttt gtt cga aac          48
Asp Ser Asp Ser Ser Thr Leu Ser Lys Lys Pro Pro Phe Val Arg Asn
1               5                   10                  15

tcc ctg gag cga cgc agc gtc cgg atg                                      75

Ser Leu Glu Arg Arg Ser Val Arg Met
            20                  25

<210>  10
<211>  25
<212>  PRT
<213>  Artificial Sequence

<220>
<223>  Synthetic Construct

<400>  10

Asp Ser Asp Ser Ser Thr Leu Ser Lys Lys Pro Pro Phe Val Arg Asn
1               5                   10                  15

Ser Leu Glu Arg Arg Ser Val Arg Met
            20                  25

<210>  11
<211>  45
<212>  DNA
<213>  Artificial Sequence

<220>
<223>  nucleic acid encoding artificial peptide

<220>
<221>  CDS
<222>  (1)..(45)

<400>  11
ttt gtt cga aac tcc ctg gag cga cgc agc gtc cgg atg aag cgg               45
Phe Val Arg Asn Ser Leu Glu Arg Arg Ser Val Arg Met Lys Arg
1               5                   10                  15

<210>  12
<211>  15
<212>  PRT
<213>  Artificial Sequence

<220>
<223>  Synthetic Construct

<400>  12

Phe Val Arg Asn Ser Leu Glu Arg Arg Ser Val Arg Met Lys Arg
1               5                   10                  15

<210>  13
<211>  51
<212>  DNA
<213>  Artificial Sequence

<220>
<223>  nucleic acid encoding artificial peptide


<220>
<221>  CDS
<222>  (1)..(51)

<400>  13
cca cct ttt gtt cga aac tcc ctg gag cga cgc agc gtc cgg atg aag          48
Pro Pro Phe Val Arg Asn Ser Leu Glu Arg Arg Ser Val Arg Met Lys
1               5                   10                  15

cgg                                                                      51
Arg




<210>  14
<211>  17
<212>  PRT
<213>  Artificial Sequence

<220>
<223>  Synthetic Construct

<400>  14

Pro Pro Phe Val Arg Asn Ser Leu Glu Arg Arg Ser Val Arg Met Lys
1               5                   10                  15


Arg




<210>  15
<211>  75
<212>  DNA
<213>  Artificial Sequence

<220>
<223>  nucleic acid encoding artificial peptide


<220>
<221>  CDS
<222>  (1)..(75)

<400>  15
gac agc tcc act ctg tcc aaa aag cca cct ttt gtt cga aac tcc ctg          48
Asp Ser Ser Thr Leu Ser Lys Lys Pro Pro Phe Val Arg Asn Ser Leu
1               5                   10                  15

gag cga cgc agc gtc cgg atg aag cgg                                      75
Glu Arg Arg Ser Val Arg Met Lys Arg
            20                  25


<210>  16

<211>  25
<212>  PRT
<213>  Artificial Sequence

<220>
<223>  Synthetic Construct

<400>  16

Asp Ser Ser Thr Leu Ser Lys Lys Pro Pro Phe Val Arg Asn Ser Leu
1               5                   10                  15


Glu Arg Arg Ser Val Arg Met Lys Arg
            20                  25


<210>  17
<211>  81
<212>  DNA
<213>  Artificial Sequence

<220>
<223>  nucleic acid encoding artificial peptide


<220>
<221>  CDS
<222>  (1)..(81)

<400>  17
gat agt gac agc tcc act ctg tcc aaa aag cca cct ttt gtt cga aac       48
Asp Ser Asp Ser Ser Thr Leu Ser Lys Lys Pro Pro Phe Val Arg Asn
1               5                   10                  15

tcc ctg gag cga cgc agc gtc cgg atg aag cgg                           81
Ser Leu Glu Arg Arg Ser Val Arg Met Lys Arg
            20                  25


<210>  18
<211>  27
<212>  PRT
<213>  Artificial Sequence

<220>
<223>  Synthetic Construct

<400>  18

Asp Ser Asp Ser Ser Thr Leu Ser Lys Lys Pro Pro Phe Val Arg Asn
1               5                   10                  15


Ser Leu Glu Arg Arg Ser Val Arg Met Lys Arg
            20                  25


<210>  19
<211>  54

<212>  DNA
<213>  Artificial Sequence

<220>
<223>  nucleic acid encoding artificial peptide


<220>
<221>  CDS
<222>  (1)..(54)

<400>  19
ttt gtt cga aac tcc ctg gag cga cgc agc gtc cgg atg aag cgg cct          48
Phe Val Arg Asn Ser Leu Glu Arg Arg Ser Val Arg Met Lys Arg Pro
1               5                   10                  15

tcc tcg                                                                  54
Ser Ser




<210>  20
<211>  18
<212>  PRT
<213>  Artificial Sequence

<220>
<223>  Synthetic Construct

<400>  20

Phe Val Arg Asn Ser Leu Glu Arg Arg Ser Val Arg Met Lys Arg Pro
1               5                   10                  15


Ser Ser




<210>  21
<211>  60
<212>  DNA
<213>  Artificial Sequence

<220>
<223>  nucleic acid encoding artificial peptide


<220>
<221>  CDS
<222>  (1)..(60)

<400>  21
cca cct ttt gtt cga aac tcc ctg gag cga cgc agc gtc cgg atg aag          48
Pro Pro Phe Val Arg Asn Ser Leu Glu Arg Arg Ser Val Arg Met Lys
1               5                   10                  15

cgg cct tcc tcg                                                          60
Arg Pro Ser Ser
            20

<210>  22
<211>  20
<212>  PRT
<213>  Artificial Sequence

<220>
<223>  Synthetic Construct

<400>  22

Pro Pro Phe Val Arg Asn Ser Leu Glu Arg Arg Ser Val Arg Met Lys
1               5                   10                  15


Arg Pro Ser Ser
            20


<210>  23
<211>  84
<212>  DNA
<213>  Artificial Sequence

<220>
<223>  nucleic acid encoding artificial peptide


<220>
<221>  CDS
<222>  (1)..(84)

<400>  23
gac agc tcc act ctg tcc aaa aag cca cct ttt gtt cga aac tcc ctg      48
Asp Ser Ser Thr Leu Ser Lys Lys Pro Pro Phe Val Arg Asn Ser Leu
1               5                   10                  15

gag cga cgc agc gtc cgg atg aag cgg cct tcc tcg                      84
Glu Arg Arg Ser Val Arg Met Lys Arg Pro Ser Ser
            20                  25


<210>  24
<211>  28
<212>  PRT
<213>  Artificial Sequence

<220>
<223>  Synthetic Construct

<400>  24

Asp Ser Ser Thr Leu Ser Lys Lys Pro Pro Phe Val Arg Asn Ser Leu
1               5                   10                  15


Glu Arg Arg Ser Val Arg Met Lys Arg Pro Ser Ser
            20                  25

<210>  25
<211>  90
<212>  DNA
<213>  Artificial Sequence

<220>
<223>  nucleic acid encoding artificial peptide


<220>
<221>  CDS
<222>  (1)..(90)

<400>  25
gat agt gac agc tcc act ctg tcc aaa aag cca cct ttt gtt cga aac          48
Asp Ser Asp Ser Ser Thr Leu Ser Lys Lys Pro Pro Phe Val Arg Asn
1               5                   10                  15

tcc ctg gag cga cgc agc gtc cgg atg aag cgg cct tcc tcg                  90
Ser Leu Glu Arg Arg Ser Val Arg Met Lys Arg Pro Ser Ser
            20                  25                  30


<210>  26
<211>  30
<212>  PRT
<213>  Artificial Sequence

<220>
<223>  Synthetic Construct

<400>  26

Asp Ser Asp Ser Ser Thr Leu Ser Lys Lys Pro Pro Phe Val Arg Asn
1               5                   10                  15


Ser Leu Glu Arg Arg Ser Val Arg Met Lys Arg Pro Ser Ser
            20                  25                  30


<210>  27
<211>  63
<212>  DNA
<213>  Artificial Sequence

<220>
<223>  nucleic acid encoding artificial peptide


<220>
<221>  CDS
<222>  (1)..(63)

<400>  27
ttt gtt cga aac tcc ctg gag cga cgc agc gtc cgg atg aag cgg cct          48
Phe Val Arg Asn Ser Leu Glu Arg Arg Ser Val Arg Met Lys Arg Pro
1               5                   10                  15

tcc tcg gtc aag tcg                                                    63
Ser Ser Val Lys Ser
            20


<210>  28
<211>  21
<212>  PRT
<213>  Artificial Sequence

<220>
<223>  Synthetic Construct

<400>  28

Phe Val Arg Asn Ser Leu Glu Arg Arg Ser Val Arg Met Lys Arg Pro
1               5                   10                  15


Ser Ser Val Lys Ser
            20


<210>  29
<211>  69
<212>  DNA
<213>  Artificial Sequence

<220>
<223>  nucleic acid encoding artificial peptide


<220>
<221>  CDS
<222>  (1)..(69)

<400>  29
cca cct ttt gtt cga aac tcc ctg gag cga cgc agc gtc cgg atg aag        48
Pro Pro Phe Val Arg Asn Ser Leu Glu Arg Arg Ser Val Arg Met Lys
1               5                   10                  15

cgg cct tcc tcg gtc aag tcg                                            69
Arg Pro Ser Ser Val Lys Ser
            20


<210>  30
<211>  23
<212>  PRT
<213>  Artificial Sequence

<220>
<223>  Synthetic Construct

<400>  30

Pro Pro Phe Val Arg Asn Ser Leu Glu Arg Arg Ser Val Arg Met Lys
1               5                   10                  15

Arg Pro Ser Ser Val Lys Ser
            20

<210>  31
<211>  93
<212>  DNA
<213>  Artificial Sequence

<220>
<223>  nucleic acid encoding artificial peptide

<220>
<221>  CDS
<222>  (1)..(93)

<400>  31
gac agc tcc act ctg tcc aaa aag cca cct ttt gtt cga aac tcc ctg        48
Asp Ser Ser Thr Leu Ser Lys Lys Pro Pro Phe Val Arg Asn Ser Leu
1               5                   10                  15

gag cga cgc agc gtc cgg atg aag cgg cct tcc tcg gtc aag tcg            93
Glu Arg Arg Ser Val Arg Met Lys Arg Pro Ser Ser Val Lys Ser
            20                  25                  30

<210>  32
<211>  31
<212>  PRT
<213>  Artificial Sequence

<220>
<223>  Synthetic Construct

<400>  32

Asp Ser Ser Thr Leu Ser Lys Lys Pro Pro Phe Val Arg Asn Ser Leu
1               5                   10                  15

Glu Arg Arg Ser Val Arg Met Lys Arg Pro Ser Ser Val Lys Ser
            20                  25                  30

<210>  33
<211>  99
<212>  DNA
<213>  Artificial Sequence

<220>
<223>  nucleic acid encoding artificial peptide

<220>
<221>  CDS
<222>  (1)..(99)

<400>  33
gat agt gac agc tcc act ctg tcc aaa aag cca cct ttt gtt cga aac        48

Asp Ser Asp Ser Ser Thr Leu Ser Lys Lys Pro Pro Phe Val Arg Asn
1               5                   10                  15

tcc ctg gag cga cgc agc gtc cgg atg aag cgg cct tcc tcg gtc aag         96
Ser Leu Glu Arg Arg Ser Val Arg Met Lys Arg Pro Ser Ser Val Lys
            20                  25                  30

tcg                                                                     99
Ser

<210>  34
<211>  33
<212>  PRT
<213>  Artificial Sequence

<220>
<223>  Synthetic Construct

<400>  34

Asp Ser Asp Ser Ser Thr Leu Ser Lys Lys Pro Pro Phe Val Arg Asn
1               5                   10                  15

Ser Leu Glu Arg Arg Ser Val Arg Met Lys Arg Pro Ser Ser Val Lys
            20                  25                  30

Ser

<210>  35
<211>  84
<212>  DNA
<213>  Artificial Sequence

<220>
<223>  nucleic acid encoding artificial peptide

<220>
<221>  CDS
<222>  (1)..(84)

<400>  35
ttt gtt cga aac tcc ctg gag cga cgc agc gtc cgg atg aag cgg cct         48
Phe Val Arg Asn Ser Leu Glu Arg Arg Ser Val Arg Met Lys Arg Pro
1               5                   10                  15

tcc tcg gtc aag tcg ctg cgc tcc gag cgt ctg atc                         84
Ser Ser Val Lys Ser Leu Arg Ser Glu Arg Leu Ile
            20                  25

<210>  36
<211>  28
<212>  PRT

<213>  Artificial Sequence

<220>
<223>  Synthetic Construct

<400>  36

Phe Val Arg Asn Ser Leu Glu Arg Arg Ser Val Arg Met Lys Arg Pro
1               5                   10                  15


Ser Ser Val Lys Ser Leu Arg Ser Glu Arg Leu Ile
            20                  25


<210>  37
<211>  90
<212>  DNA
<213>  Artificial Sequence

<220>
<223>  nucleic acid encoding artificial peptide


<220>
<221>  CDS
<222>  (1)..(90)

<400>  37
cca cct ttt gtt cga aac tcc ctg gag cga cgc agc gtc cgg atg aag          48
Pro Pro Phe Val Arg Asn Ser Leu Glu Arg Arg Ser Val Arg Met Lys
1               5                   10                  15

cgg cct tcc tcg gtc aag tcg ctg cgc tcc gag cgt ctg atc                  90
Arg Pro Ser Ser Val Lys Ser Leu Arg Ser Glu Arg Leu Ile
            20                  25                  30


<210>  38
<211>  30
<212>  PRT
<213>  Artificial Sequence

<220>
<223>  Synthetic Construct

<400>  38

Pro Pro Phe Val Arg Asn Ser Leu Glu Arg Arg Ser Val Arg Met Lys
1               5                   10                  15


Arg Pro Ser Ser Val Lys Ser Leu Arg Ser Glu Arg Leu Ile
            20                  25                  30


<210>  39
<211>  114
<212>  DNA
<213>  Artificial Sequence

<220>
<223>  nucleic acid encoding artificial peptide


<220>
<221>  CDS
<222>  (1)..(114)

<400>  39
gac agc tcc act ctg tcc aaa aag cca cct ttt gtt cga aac tcc ctg          48
Asp Ser Ser Thr Leu Ser Lys Lys Pro Pro Phe Val Arg Asn Ser Leu
1               5                   10                  15

gag cga cgc agc gtc cgg atg aag cgg cct tcc tcg gtc aag tcg ctg          96
Glu Arg Arg Ser Val Arg Met Lys Arg Pro Ser Ser Val Lys Ser Leu
            20                  25                  30

cgc tcc gag cgt ctg atc                                                 114
Arg Ser Glu Arg Leu Ile
        35


<210>  40
<211>  38
<212>  PRT
<213>  Artificial Sequence

<220>
<223>  Synthetic Construct

<400>  40

Asp Ser Ser Thr Leu Ser Lys Lys Pro Pro Phe Val Arg Asn Ser Leu
1               5                   10                  15


Glu Arg Arg Ser Val Arg Met Lys Arg Pro Ser Ser Val Lys Ser Leu
            20                  25                  30


Arg Ser Glu Arg Leu Ile
        35


<210>  41
<211>  120
<212>  DNA
<213>  Artificial Sequence

<220>
<223>  nucleic acid encoding artificial peptide


<220>
<221>  CDS
<222>  (1)..(120)

<400>  41
gat agt gac agc tcc act ctg tcc aaa aag cca cct ttt gtt cga aac          48

Asp Ser Asp Ser Ser Thr Leu Ser Lys Lys Pro Pro Phe Val Arg Asn
1               5                   10                  15

tcc ctg gag cga cgc agc gtc cgg atg aag cgg cct tcc tcg gtc aag         96
Ser Leu Glu Arg Arg Ser Val Arg Met Lys Arg Pro Ser Ser Val Lys
            20                  25                  30

tcg ctg cgc tcc gag cgt ctg atc                                         120
Ser Leu Arg Ser Glu Arg Leu Ile
        35                  40

<210>  42
<211>  40
<212>  PRT
<213>  Artificial Sequence

<220>
<223>  Synthetic Construct

<400>  42

Asp Ser Asp Ser Ser Thr Leu Ser Lys Lys Pro Pro Phe Val Arg Asn
1               5                   10                  15

Ser Leu Glu Arg Arg Ser Val Arg Met Lys Arg Pro Ser Ser Val Lys
            20                  25                  30

Ser Leu Arg Ser Glu Arg Leu Ile
        35                  40

<210>  43
<211>  7
<212>  PRT
<213>  Artificial Sequence

<220>
<223>  cell penetration moiety

<400>  43

Arg Arg Arg Arg Arg Arg Arg
1               5

<210>  44
<211>  8
<212>  PRT
<213>  Artificial Sequence

<220>
<223>  cell penetration moiety

<400>  44

Arg Arg Arg Arg Arg Arg Arg Arg
1               5

<210>  45
<211>  9
<212>  PRT
<213>  Artificial Sequence

<220>
<223>  cell penetration moiety

<400>  45

Arg Arg Arg Arg Arg Arg Arg Arg Arg
1               5


<210>  46
<211>  10
<212>  PRT
<213>  Artificial Sequence

<220>
<223>  cell penetration moiety

<400>  46

Arg Arg Arg Arg Arg Arg Arg Arg Arg Arg
1               5                   10


<210>  47
<211>  18
<212>  PRT
<213>  Artificial Sequence

<220>
<223>  cell penetration moiety

<400>  47

Lys Leu Ala Leu Lys Leu Ala Leu Lys Ala Leu Lys Ala Ala Leu Lys
1               5                   10                  15


Leu Ala



<210>  48
<211>  10
<212>  PRT
<213>  Artificial Sequence

<220>
<223>  cell penetration moiety

<400>  48

Pro Arg Pro Leu Pro Phe Pro Arg Pro Gly
1               5                   10

<210>  49
<211>  11
<212>  PRT
<213>  Artificial Sequence

<220>
<223>  cell penetration moiety

<400>  49

Tyr Ala Arg Ala Ala Ala Arg Gln Ala Arg Ala
1               5                   10


<210>  50
<211>  12
<212>  PRT
<213>  Artificial Sequence

<220>
<223>  cell penetration moiety

<400>  50

Arg Arg Gln Arg Arg Thr Ser Lys Leu Met Lys Arg
1               5                   10


<210>  51
<211>  66
<212>  DNA
<213>  Artificial Sequence

<220>
<223>  KIBRA target sequence

<400>  51
gatccgttga agttaaacag caagattcaa gagatcttgc tgtttaactt caaccttttt      60

tggaaa                                                                  66


<210>  52
<211>  21
<212>  DNA
<213>  Artificial Sequence

<220>
<223>  primer

<400>  52
gaaggagctg aaggagcatt t                                                 21


<210>  53
<211>  21
<212>  DNA
<213>  Artificial Sequence

<220>
<223>  primer

<400>  53
cctgaaagac tgcacttctg g                                                21


<210>  54
<211>  25
<212>  DNA
<213>  Artificial Sequence

<220>
<223>  primer

<400>  54
cgctcaccct caagtgggtg gacag                                            25


<210>  55
<211>  23
<212>  DNA
<213>  Artificial Sequence

<220>
<223>  primer

<400>  55
ggcttggaag aggtggccgt tgg                                              23


<210>  56
<211>  21
<212>  DNA
<213>  Artificial Sequence

<220>
<223>  primer

<400>  56
ccacccgggc ctggagacat g                                                21


<210>  57
<211>  20
<212>  DNA
<213>  Artificial Sequence

<220>
<223>  primer

<400>  57
accccaccgt gttcttcgac                                                  20


<210>  58
<211>  20
<212>  DNA
<213>  Artificial Sequence

<220>
<223>  primer

<400>  58
catttgccat ggacaagatg                                                    20

## Description

In a broad sense, the present invention, among others, relates to compounds that are capable of stabilizing protein kinase M zeta (in the following "PKM zeta", "PKMz" or the like) as well as methods for identifying those. PKM zeta is a protein kinase that has been implicated in the fields of cognition, memory and learning such that said compounds are considered beneficial in these fields.

### BACKGROUND OF THE INVENTION

PKM zeta is an atypical protein kinase and a molecule that is involved in memory maintenance *(cf. e.g. Sacktor, 1993; Sacktor et al., 2008).* For example, inhibiting PKM zeta activity is linked to a decrease in the ability to maintain long-term memories and procedural knowledge, while a increase in PKM zeta levels enhances the capabilities for memory storage *(Shema et al., 2007; Shema et al., 2011; Serrano et al., 2008; Kraus et.al., 2010).* PKM zeta is a brain specific variant of and is known to be identical to about the C-terminal half of the atypical protein kinase Cζ ("PKC zeta"). PKM zeta lacks the N-terminal auto-inhibitory domain of PKC zeta and is e.g. generated by an independent promoter within the gene *(Hernandez, et al., 2003).* PKM zeta mRNA is stored locally in dendrites and translated after sufficient synaptic stimulation *(Osten et al., 1996; Muslimov et al., 2004).* PKM zeta is constitutively active after phosphorylation by PDK1 *(Kelly et al., 2007).*

Among numerous other proteins that show a relation to memory and learning is KIBRA (for Kldney BRAin protein). KIBRA is also known as WWC1 (WW domain-containing protein 1) and has come into the focus of neuroscience since it was shown that a SNP of the ninth intron of the KIBRA gene (cf. the KIBRA rs17070145 T allele, rather than the C allele) is associated with human episodic memory performance. In addition, KIBRA has been implicated in human episodic memory performance by multiple genome-wide association studies *(Papassotiropoulos et al., 2006; Schneider et al., 2010).* This function appears biologically plausible as KIBRA interacts with synaptic proteins *(Büther et al., 2004),* localizes to the post-synaptic density, and is expressed in hippocampus and cortex *(Johannsen et al., 2008),* brain regions involved in learning and memory.

Moreover, it has been shown that protein kinase C zeta ("PKC zeta") can interact with KIBRA and ― at least in vitro ― can phosphorylate two serine residues near the KIBRA C-terminus, suggesting that said phosphorylation may regulate the cellular function of KIBRA *(Büther et al., 2004).* Besides, also PKM zeta has been shown to interact with KIBRA (*Yoshihama et al., 2009*)*.*

Protein degradation or proteolysis is known to involve proteases, i.e. protein degrading enzymes or the like, i.e. compounds that enable the breakage of peptide bonds. To this end, the proteasome is a well known protein complex for protein degradation. It is predominantly found in eukaryotes. Protein degradation via the proteasome usually yields peptides of about seven to eight amino acids, which can subsequently be further degraded, such as into individual amino acids. Proteins to be degraded via the proteasome are labeled (i.e. via modification of a lysine residue) for degradation with a small protein called ubiquitin. This is usually effected by enzymes called ubiquitin ligases, particularly ubiquitin E3 ligases. In detail, protein ubiquitination has been found to involve three enzymes, E1, E2 and E3 (referred to herein as E1, E2 and E3 enzymes or ligases, respectively). The first, E1, a ubiquitin-activating enzyme, hydrolyzes ATP and adenylates a ubiquitin molecule, which is then transferred to the second enzyme, E2, a ubiquitinconjugating enzyme, before the third enzyme, E3, a ubiquitin ligase, catalyzes the transfer of ubiquitin from the second enzyme to a target protein to be ubiquitinated. Proteins may be labeled (i.e. ubiquitinated) with varying numbers of ubiquitin molecules. Usually, at least four or more ubiquitin molecules are added for sufficient degradation.

Prior to the invention, little was known how PKM zeta action could be improved and there remains a need for suitable applications such as practical pharmaceutical applications related to PKM zeta, such as applications that make use of or support, respectively, the beneficial action of PKM zeta in the brain. This is particularly so in view of the protein size of PKM zeta and the presence of the blood brain barrier. Besides, there remains a need for compounds stabilizing PKM zeta.

Accordingly, it is one of the objects of the present invention to provide means and substances for stabilizing PKM zeta or supporting the action of PKM zeta, particularly pharmaceutically suitable substances, which may e.g. be applied in order to improve e.g. cognition, memory and/ or learning.

These and other objects are considered to be solved by the subject-matter of the present invention disclosed hereinbelow and in the claims.

### SUMMARY OF THE INVENTION

The invention relates to the subject-matter as defined herein in the claims. Said subject-matter includes a method for identifying a compound capable of inhibiting (proteasomal) degradation of PKM zeta, such as a method comprising the steps of providing a test system for monitoring the degradation of PKM zeta, and determining whether a given test compound leads to a reduced degradation of PKM zeta in said test system, or such as a method comprising the step of determining whether a given test compound is capable of interfering with the binding of certain peptides, peptide derivatives or peptide conjugates to PKM zeta and/or PKC zeta.

In other aspects, the present invention relates to compounds capable of inhibiting degradation of PKM zeta, identified by methods of the invention, so such compounds for use in medicine or for use in particular methods defined herein.

In further aspects, the present invention relates to pharmaceutical composition comprising such compounds. In still other aspects, the invention relates to methods for stabilizing PKM zeta comprising contacting PKM zeta with such compounds.

### SHORT DESCRIPTION OF THE FIGURES

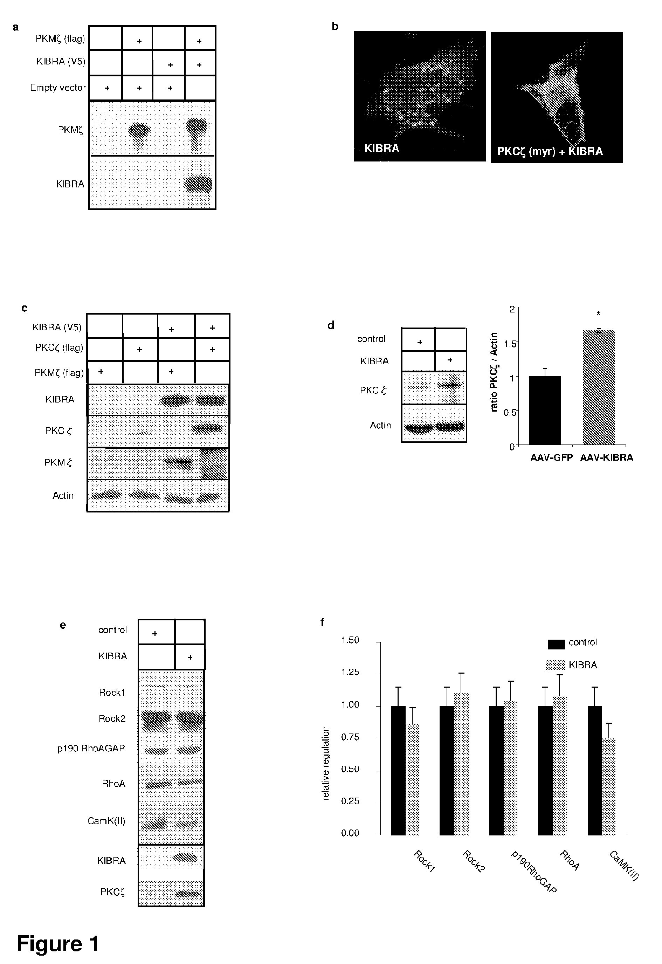

**Fig. 1** depicts that the presence of KIBRA elevates PKC/PKM zeta cellular protein levels: a, Co-immunoprecipitation of KIBRA and PKM zeta in COS-cells with αFLAG beads confirms strong interaction of both proteins b, Co-expression of myristoylated PKC zeta changes localization of a KIBRA-GFP fusion protein in SH-SY5Y cells, suggesting that the interaction is also of relevance in a cellular context c, PKM zeta and PKC zeta expressed alone or co-expressed with KIBRA in COS cells d, Endogenous PKC zeta protein levels are elevated by KIBRA co-expression. Rat primary cortical neurons were infected with either EGFP-control or KIBRA-expressing AAV virus e, f Levels of several protein kinases expressed in COS cells remain unchanged by co-expression of KIBRA. e, Western blot, f, band intensity quantifications (n=3) normalized to actin.

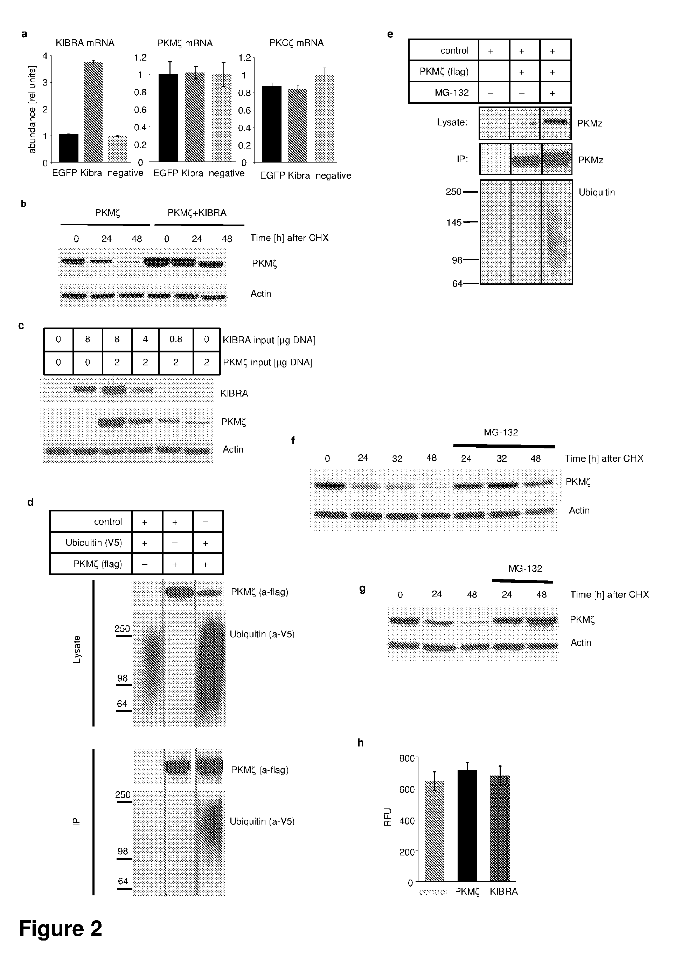

**Fig. 2** illustrates that PKM zeta is subj ect to proteasomal degradation: **a,** Quantification of KIBRA, PKM zeta and PKC zeta mRNA levels in rat primary cortical neurons after infection with KIBRA- or EGFP overexpressing AAV virus or in untreated cells by _{q}PCR. **b,** Time-course of PKM zeta expression in the presence of absence of KIBRA co-expression in COS cells after treatment with the translation inhibitor CHX **c,** Expression levels of PKM zeta co-expressed with different amounts of KIBRA in COS cells **d,** Immunoprecipitation of PKM zeta with or without co-expressed ubiquitin, showing ubiquitinylation of PKM zeta **e,** Pull-down of PKM zeta showing ubiquitinylation by endogenous ubiquitin after treatment with the proteasome inhibitor MG132. **f, g** PKM zeta protein stability with and without proteasome inhibition by MG132 in COS (f) and SHSY-5Y cells **h** Neither KIBRA nor PKM zeta presence has an influence on general proteasome activity as determined by a fluorigenic assay.

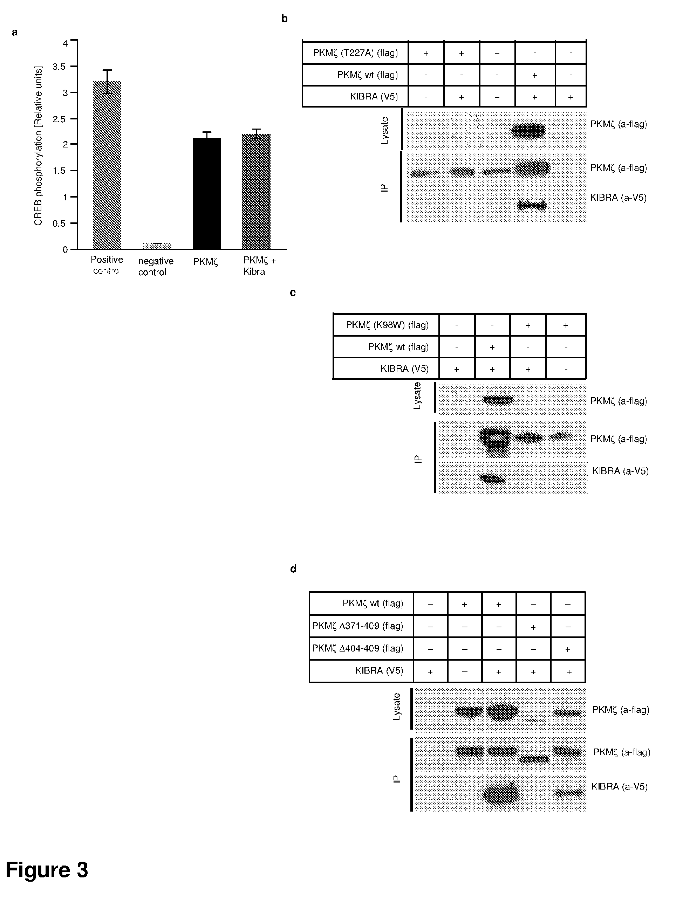

**Fig. 3** shows that KIBRA binds only activated PKM zeta, and that PKM zeta remains active after binding to KIBRA. **a,** CREB peptide phosphorylation assay of either commercially available PKC zeta (positive control) or PKM zeta immunoprecipitated from COS cells when expressed alone or in the presence of KIBRA. Kinase activity in the presence of KIBRA is not diminished (red bar) **b,** Co-IP of KIBRA with wt PKM zeta and the T227A activation-loop mutant using αFLAG beads c, Co-IP of KIBRA with the ATPbinding site mutant of PKM zeta K98W, longer exposure (right) reveals weak expression of PKM zeta K98W in the presence of KIBRA in the lysate while the protein is not detectable before pull-down without KIBRA. **d,** Co-IP of KIBRA with the PKM zeta Δ371-409 deletion construct lacking the C-terminal hydrophobic motif. The C-terminally truncated PKM zeta is only weakly expressed, and does not interact with KIBRA any more. In contrast, the more C-terminal deletion Δ404-409 still interacts with KIBRA.

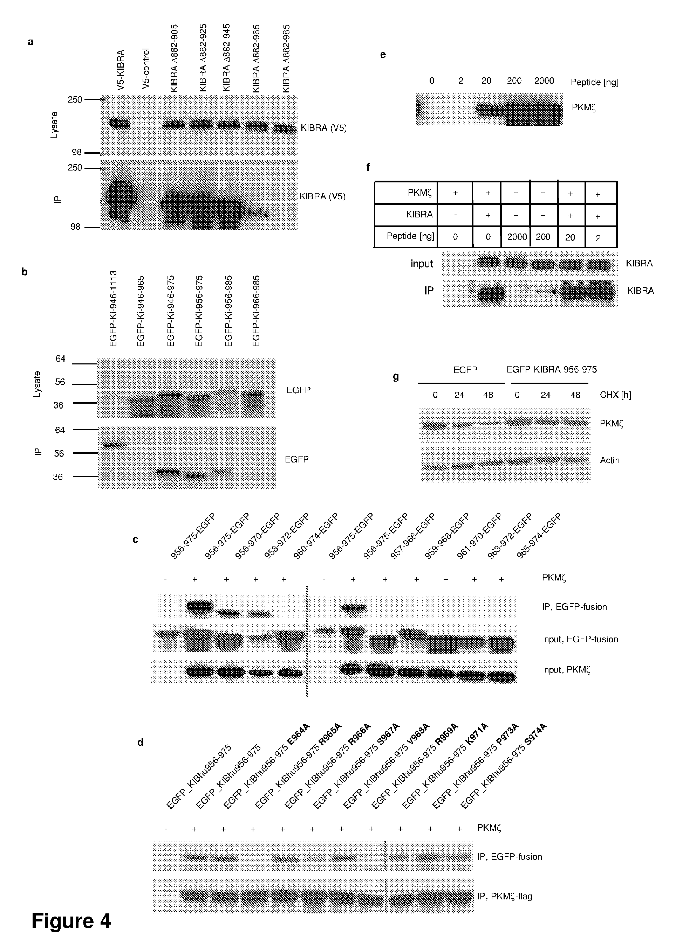

**Fig. 4** depicts that binding and protection of PKM zeta is dependent on a short motif near the KIBRA C-terminus **a,** Series of KIBRA deletion constructs for fine-mapping of the interaction site with PKM zeta. Starting from aa 882, increasing 20aa stretches of KIBRA were deleted and constructs were tested for interaction with FLAG-tagged PKM zeta. Very weak interaction was observed for Δ882-965, while Δ882-985 failed to interact at all, suggesting that the interaction site lies between aa946 and 985. **b,** The region between position 946 and 985 was investigated in a reverse approach by fusing overlapping stretches of 20 or 30 amino acids to EGFP and testing for PKM zeta-binding by co-immunoprecipitation. The data suggest that the binding motif lies between aa956 and 975, and that this stretch is sufficient to mediate binding **c,** Further deletion mapping indicates that the absolute minimal binding motif is from position 958 to 970. Only peptide fusions 956-970 and 958-972 are able to bind PKM zeta, indicating that the minimal sequence required for binding is amino acids 958 - 970. However, the interaction efficacy seems to drop with both short sequences in contrast to the 20mer **d,** Alanin mutation scan. PKM zeta-flag expression constructs were co-transfected with EGFP-fusion constructs containing the 956-975 PKM zeta binding motif. Amino acids from position 964 to 974 were mutated to Alanin. The arginine at position 965 is very important for binding **e,** A synthetic biotin-labeled peptide containing the binding motif (position 948-978, DSSTLSKKPPFVRNSLERRSVRMKRPSSVKS (SEQ ID NO: 32)) is able to bind PKM zeta expressed in COS cells as shown by co-immunoprecipitation using streptavidin beads **f,** The synthetic peptide 948-978 is able to disrupt a preformed KIBRA- PKM zeta complex. Increasing concentrations of the peptide were added to COS-lysates co-expressing KIBRA and FLAG- PKM zeta before pull-down with anti-FLAG beads. IC50 in this assay is estimated to be around 60 nM. **g,** The 20-amino acid binding motif is sufficient to mediate PKM zeta stability increase. Shown are PKM zeta levels after addition of CHX with co-expression of the EGFP-KIBRA-956-975 construct or an EGFP control vector.

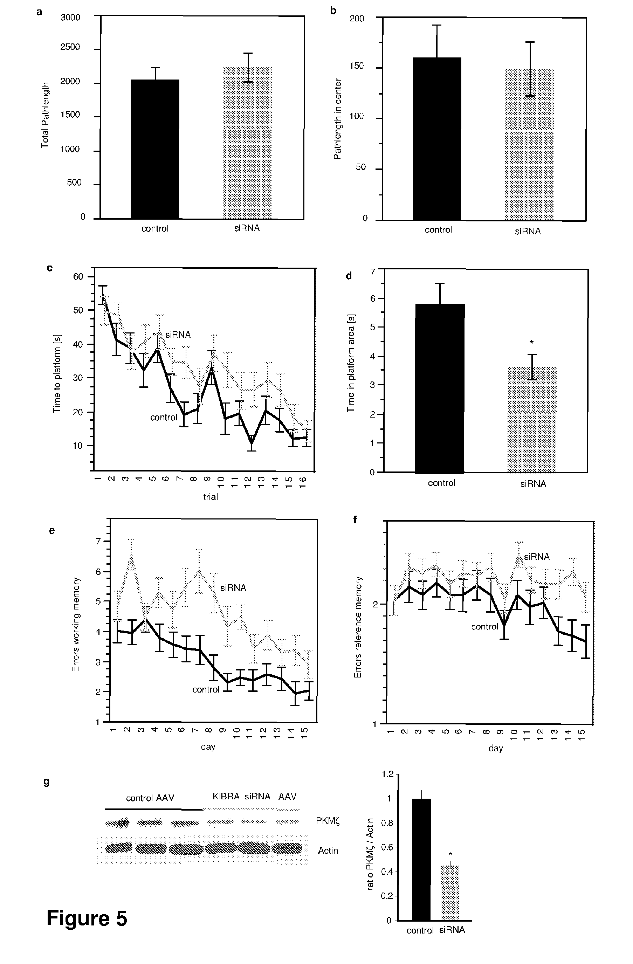

**Fig. 5** illustrates that hippocampal KIBRA knock-down by intrahippocampal delivery of KIBRA siRNA by AAV results in memory deficits **a,b,** Open field test reveals no difference in general motor behavior (**a,** total pathlength), and exploratory drive (**b,** pathlength in center area) **c,** Acquisition phase in the Morris watermaze is similar between groups **d,** In the probetrial after 24 h KIBRA knock-downs are significantly inferior to controls in recalling platform location (p<0.05) e, Analysis of working memory errors in the radial 8-arm maze reveals a difference in offset of the learning curves (p<0.05 by regression analysis), while the slopes are not significantly different **f,** Reference memory error reduction over 15 days is significantly inferior to controls (p<0.05 by regression analysis) **g,** Quantification of PKM zeta protein in the hippocampi of the siRNA or EGFP transduced rats reveals a significant reduction of PKM zeta levels in the KIBRA knock-downs.

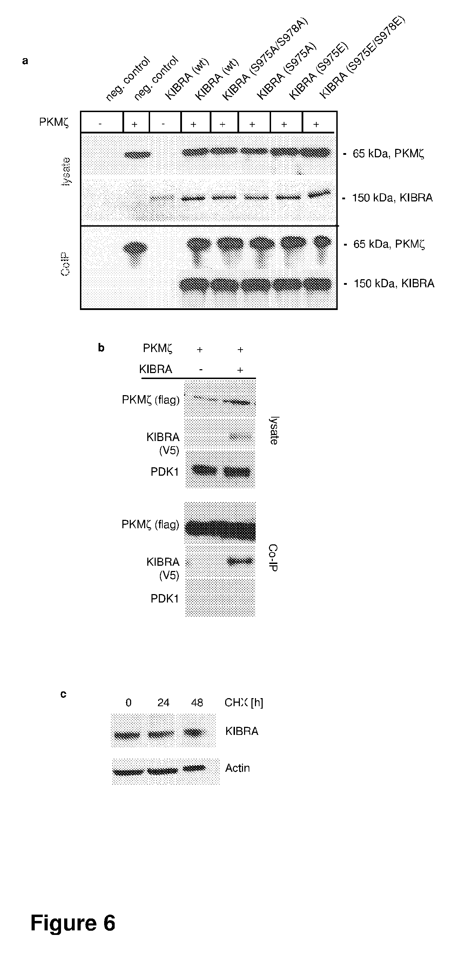

**Fig. 6** depicts the following: **a,** KIBRA C-terminal phosphorylation status does not alter interaction with PKM zeta. The C-terminal serine residues that can be phosphorylated by PKM zeta in vitro were mutated to alanine or glutamate. Neither of these alterations influenced the binding of PKM zeta to KIBRA, suggesting that the interaction is not mainly a kinase-target interaction **b,** PDK1 is not contained in the complex of KIBRA and PKM zeta. Shown are co-immunoprecipitation experiments with KIBRA and PKM zeta overexpressed in COS cells **c,** KIBRA protein levels remain stable over at least 48 h following translation inhibition with cycloheximide (CHX).

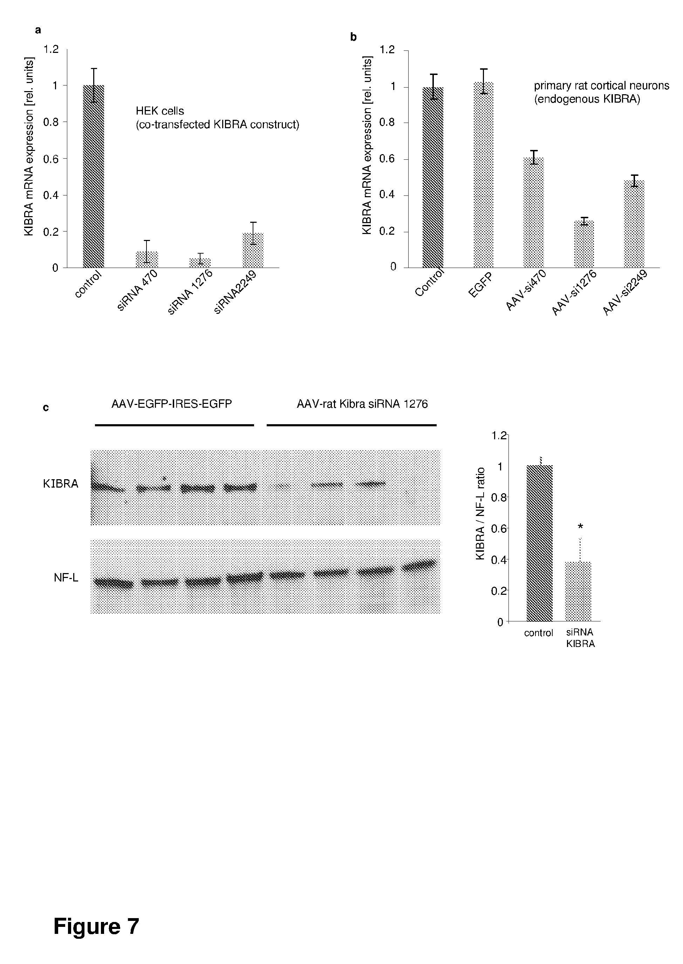

**Fig. 7** illustrates a characterization of siRNA knock-down of KIBRA. **a, b,** Cell culture knock-downs of KIBRA mRNA in Hek cells (a) or primary cortical neurons (b) shRNA1267 was used for the further experiments. In Hek cells, a KIBRA-expressing construct was co-transfected with the siRNA. c, In vivo knock-down of KIBRA in the rat hippocampus shown by Western blot and quantification of bands.

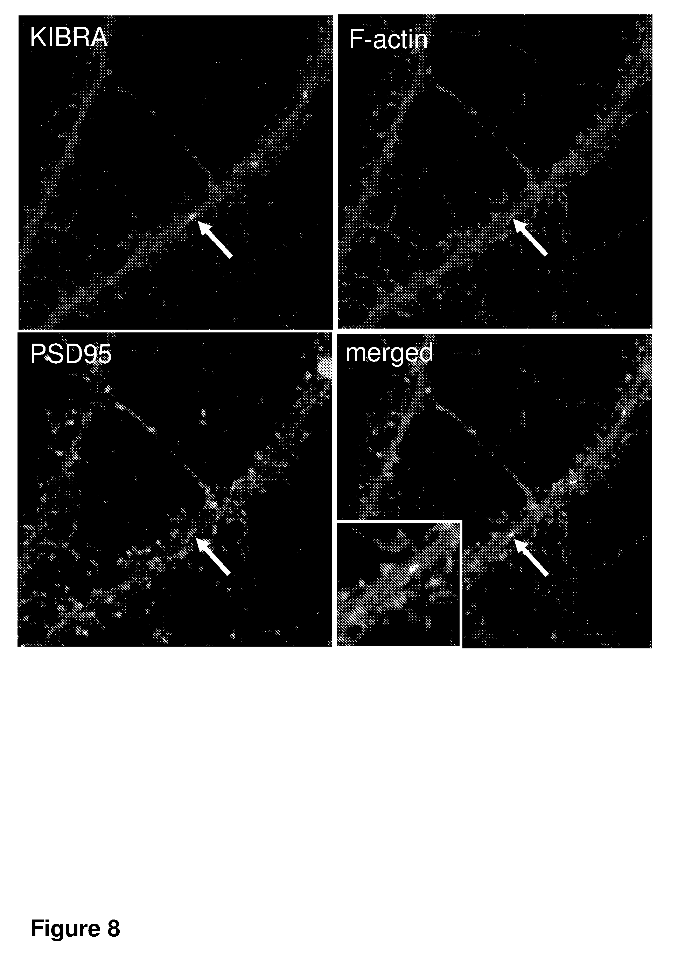

**Fig. 8** illustrates the localization of KIBRA in primary neurons in culture. Neurons were infected with a KIBRA-EGFP fusion constructs. KIBRA localizes differentially to different spines.

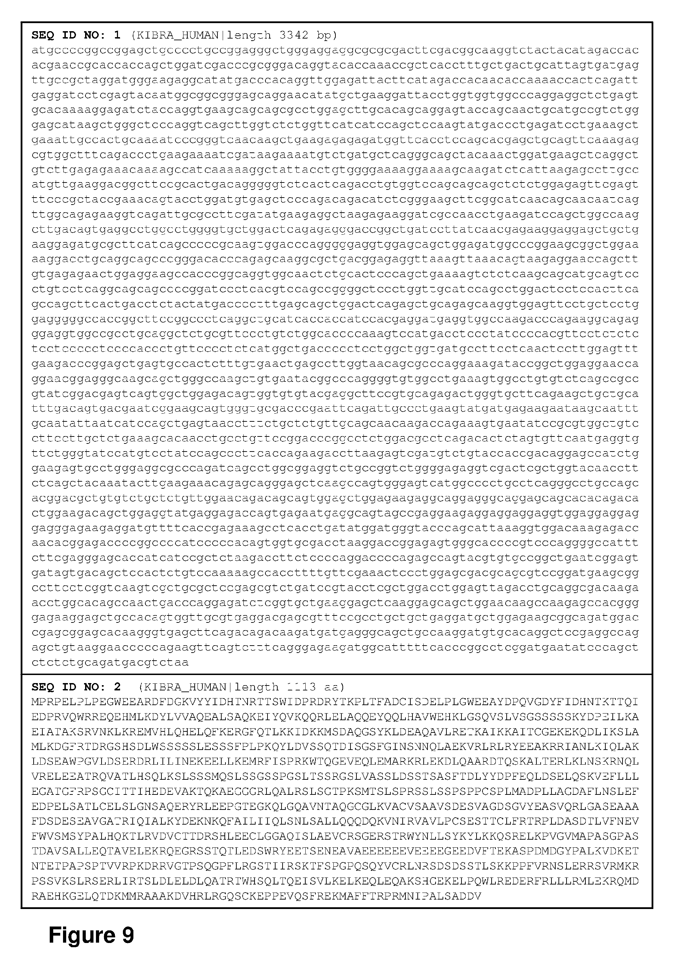

**Fig. 9** depicts a full-length human KIBRA DNA and protein sequence, which can also be found in SEQ ID NOs: 1 and 2 of the sequence listing.

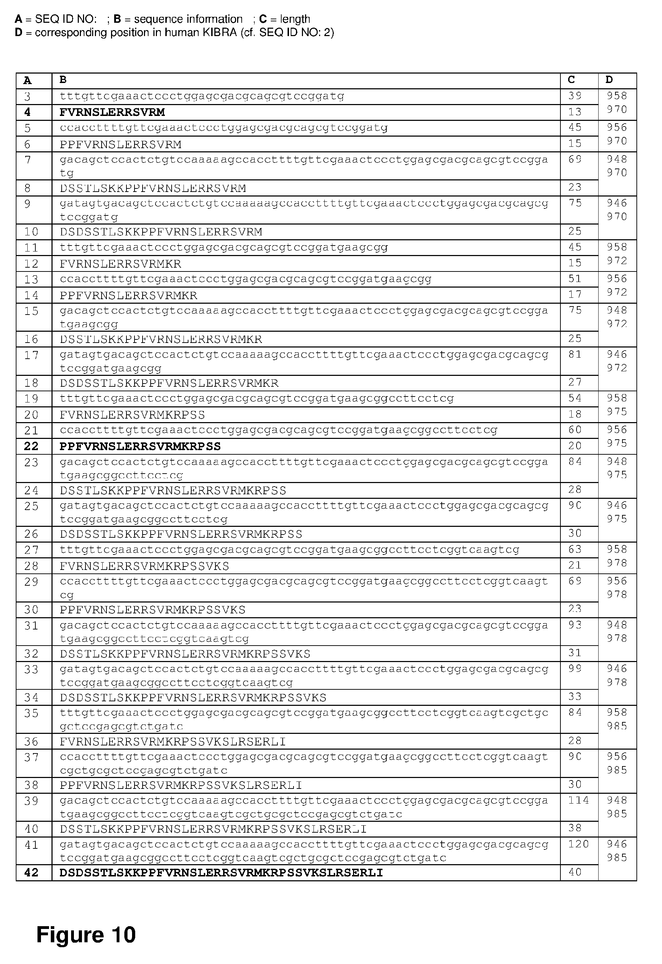

**Fig. 10** illustrates certain KIBRA sequences related to the present invention, i.e. the sequences that can also be found as SEQ ID NOs: 3-42 of the sequence listing

### DETAILED DESCRIPTION OF THE PREFERRED EMBODIMENTS

The various aspects of present invention are described hereinbelow and are defined in the claims.

Surprisingly, the present inventors have found that activated PKM zeta is stabilized by KIBRA by shielding the protein from degradation, particularly from proteasomal degradation. In addition, the present inventors have surprisingly found that said protection is the protection of activated, i.e. phosphorylated PKM zeta and that the protected form of PKM zeta retains its native activity, in particular, the shielded form of PKM zeta retains its kinase activity.

Moreover, the present inventors have surprisingly found that even a short peptide derived from the C-terminal section of KIBRA (i.e. a short motif near the KIBRA C-terminus) is sufficient for mediating said protein-protein interaction and for mediating such protection of PKM zeta. In addition, the present inventors have obtained surprising evidence that the interaction between KIBRA and PKM zeta is not, at least not primarily, a kinase-target interaction. Interestingly, as may be concluded from the present inventor's evidence, PKM zeta binds to the C-terminus of KIBRA and binding and protection are mediated by the same motif.

The present inventors have also shown that knock-down of KIBRA is accompanied by decreased PKM zeta protein levels. Without intending to be bound by theory, the present inventors consider that a key mechanism in memory maintenance is the stabilization of synaptic PKM zeta levels by binding to KIBRA or the KIBRA-derived peptides and the like of the present invention. In detail, the present inventors propose that KIBRA is a second component in the synaptic tag element constituted by PKM zeta. PKM zeta would be normally rapidly degraded after its initial local dendritic synthesis unless KIBRA is available for binding and preservation of this tag. Hence, KIBRA could be a regulator of the degree to which a particular spine, dendrite segment, or neuron is allowed to stabilize new input signal information or not. While the mode of regulation of KIBRA levels or its translocation is not fully understood at present, the present inventors already have evidence (not shown) that different learning paradigms can increase neuronal KIBRA levels. This model furthermore complements existing theories how PKM zeta level are maintained at spines for long time periods by positive feedback-loops, through which active PKM zeta would enhance its own translation by relief of a translational block *(Sacktor 2011).* Such a self-sustaining regulation is postulated to lead to a switch-like behavior stabilizing the activated state of the system after the critical threshold level has been reached. Depending on the way KIBRA itself is regulated, local KIBRA levels could either set the threshold of PKM zeta activity required to enter the positive feedback-loop leading to self-sustained activity, or act as a second component of the molecular decision process which spines to enhance.

These and the other findings herein open new pharmacological applications and approaches, which e.g. relate to cognition, learning and memory, such as to enhanced memory performance.

Accordingly, in a first aspect, the present invention relates to a method for identifying a compound capable of inhibiting degradation of PKM zeta.

Said method may e.g. be an in vivo method, ex vivo method or in vitro method. Accordingly, according to a preferred embodiment, said method may be performed in vitro. In some other embodiments, said method may be performed in vivo or ex vivo.

A "compound capable of inhibiting degradation of PKM zeta" as used herein is not particularly limited, as will be apparent from the following detailed descriptions. That is, in a first set of embodiments, said compound may be a compound that leads to a reduced degradation of PKM zeta in a test system. In a second set of embodiments, said compound may be a compound that is capable of interfering with PKM zeta. In a third set of embodiments, said compound may be a compound capable of interfering with the binding of certain peptides, peptide derivatives or peptide conjugates thereof to PKM zeta. Importantly, a given compound capable of inhibiting degradation of PKM zeta may fall under one, but also under more than one particular embodiment described herein.

In more detail, in a first set of embodiments of the first aspect, said method of the first aspect comprises the following steps a) providing a test system for monitoring the degradation of PKM zeta, and b) determining whether a given test compound leads to a reduced degradation of PKM zeta in said test system.

"Degradation of PKM zeta" as used herein refers to proteolysis of PKM zeta and preferably involves more than one proteolytic cleavage or breakage of a peptide bond, respectively, within PKM zeta. Preferably herein, degradation of PKM zeta refers to degradation of PKM zeta by the proteasome, i.e. to proteasomal degradation of PKM zeta.

Numerous ways of monitoring degradation of PKM zeta are possible. These include, but are not limited to monitoring the presence of PKM zeta protein over time for example via the use of antibodies (e.g. via Western Blotting). Other ways involve principles described in the Examples herein.

Accordingly, the term "test system for monitoring the degradation of PKM zeta" as used herein is not particularly limited and comprises in vitro, ex vivo and in vivo test systems. Accordingly, said system may e.g. involve a living cell.

As used herein, reduced degradation is preferably intended to mean that degradation is reduced by at least 10%, 15%, 20%, or 25%; preferably by at least 30%, 35%, 40%, 45%; preferably by at least 50%, 55%, 60%, 65%; preferably by at least 70%, 75%, 80%, 85%; preferably by at least 90%, 95%, 96%, 97%, 98%, 99% such as by about 100%.

Generally herein, a compound capable of inhibiting degradation of PKM zeta is preferably verified by means of a negative control in the absence of said test compound. The methods of the first set of embodiments of the first aspect, may thus include a step of measuring degradation of PKM zeta in the absence of a given test compound. Accordingly, reduced degradation may also mean that the amount of PKM zeta measured in the absence of the test compound at a given time point is at least 10%, 15%, 20%, or 25% less; preferably at least 30%, 35%, 40%, 45% less; preferably at least 50%, 55%, 60%, 65% less; preferably at least 70%, 75%, 80%, 85% less; preferably by at least 90%, 95%, 96%, 97%, 98%, 99% less, such as about 100% less than the amount of PKM zeta measured in the presence of the test compound.

Preferably, the test system employed in the present invention involves inhibiting protein translation. In other words, said method of the first aspect may involve the addition of an inhibitor of protein translation, which may also be referred to herein as a protein synthesis inhibitor. Numerous suitable inhibitors of protein translation - that work at various stages of translation of mRNA into proteins - are known to the skilled person and include, but are not limited to those selected from the group consisting of compounds inhibiting translation initiation, aminoacyl tRNA entry, peptidyl transfer, ribosomal translocation, and translation termination. Inhibitors of protein translation usually bind to the small or large subunit of the ribosome, which differ between prokaryotes and eukaryotes.

Particular examples of inhibitors of protein translation include, but are not limited to chloramphenicol, cycloheximide, diphtheria toxin, erythromycin, fusidic acid, linezolid, macrolid antibiotics, puromycin, streptogramin, tetracycline, tigecycline, and derivatives of the above.

Preferred examples of inhibitors of protein translation are inhibitors of eukaryotic protein translation and include, but are not limited to cycloheximide, diphtheria toxin and puromycin. Cycloheximide is e.g. produced by the bacterium *Streptomyces griseus.* It interferes with the translocation step in protein synthesis and, therefore, with translational elongation.

The term "test compound" is well known by a person skilled in the art. As used herein, test compounds are to be understood broadly and e.g. include all kinds of substances, which can be brought in contact with PKM zeta or cultured cells. Non-limiting examples of test compounds include low molecular weight compounds, peptides, polypeptides, nucleic acids, and the like, including derivatives and conjugates of all of the test compounds listed.

Generally herein, a test compound is not particularly restricted and may be natural compounds or synthetic compounds. In certain preferred embodiments, the test compound is selected from a peptide, polypeptide and protein or a derivative or conjugate thereof. As used herein, said peptide has from about 5 to about 40 amino acids, such as from about 10 to about 30 amino acids, such as from about 13 to about 20 amino acids. As used herein, said polypeptide may be any protein. Preferably, said polypeptide has from about 41 to about 2000 amino acids, such as from 45 to 1000 amino acids, from 50 to 500 amino acids, or from 100 to 300 amino acids. The peptide or polypeptide may be chimeric. The peptide or polypeptide may be an antibody. The peptide or polypeptide may also be a transcription factor. Peptide and polypeptide derivatives and conjugates are well known to the skilled person and may include any of the moieties described in relation to peptide derivatives and conjugates disclosed herein. Examples for nucleic acids also include aptamers, antisense nucleic acids and siRNA. Accordingly, in certain embodiments, the test compound is an siRNA.

In other preferred embodiments, the test compound is a low molecular weight compound and, preferably, not a peptide, polypeptide or protein or a derivative or conjugate thereof. Preferably, said low molecular weight compounds have a molecular weight of less than 1000 g/mol (or Da, respectively), such as preferably less than 800 g/mol, less than 750 g/mol, less than 700 g/mol, less than 600 g/mol, less than 500 g/mol, less than 400g/mol less than 300g/mol. Preferably, said low molecular weight compounds have a molecular weight of more than 50 g/mol.

A test compound herein may e.g. be identified starting from a collection of test compounds or a library containing such test compounds. Hence, the above identification may include a step of screening such collection or library. Numerous such collections and libraries, including customized collections and libraries, are available to the skilled person, such as commercially available.

In a second set of embodiments of the above methods of the first aspect comprises a step of determining whether a given test compound is capable of interfering with the proteasomal degradation of PKM zeta.

In preferred embodiments, said methods comprise a step of determining whether a given test compound is capable of i) inhibiting a E3-, E2-, E1-Ligase involved in proteasomal degradation of PKM zeta; or ii) stimulating deubiquitination of PKM zeta; or iii) inhibiting PKM zeta transport to or entry into the proteasome; or iv) inhibiting the assembly of the multiprotein complex involved in PKM zeta degradation.

As used herein, the term "a step of" or "the step of" is not intended to refer only to a single action or experimental step, but also includes reference to various actions or experimental steps. The exact nature of such actions or experimental steps will depend on the particular aspects and embodiments and will easily be understood by the skilled person.

Also, the present invention explicitly also relates to corresponding aspects and embodiments of all of the aspects and embodiments described herein, wherein the term "a step of" or "the step of" is not contained in the language describing said aspects and embodiments.

Accordingly, in a first particular type of embodiments of said second set of embodiments of the first aspect, the method of the first aspect comprises inhibiting an E3-, E2-, E1-Ligase involved in proteasomal degradation of PKM zeta. In a second particular type of embodiments of said second set of embodiments of the first aspect the method of the first aspect comprises stimulating deubiquitination of PKM zeta. In a third particular type of embodiments of said second set of embodiments of the first aspect said the method of the first aspect comprises inhibiting PKM zeta transport to or entry into the proteasome. In a fourth particular type of embodiments of said second set of embodiments of the first aspect the method of the first aspect comprises inhibiting the assembly of the multiprotein complex involved in PKM zeta degradation, by means of e.g. deneddylation of the cullin subunit of RING-finger ligases which facilitates the dissociation of CRL components.

Said compound capable of inhibiting according to i) and said compound capable of stimulating according to ii) may also referred to herein as compounds "capable of interfering with the ubiquitination of PKM zeta". Preferably, as used herein, a compound "capable of interfering with the ubiquitination of PKM zeta", a compound "inhibiting a E3-, E2-, E1-Ligase involved in proteasomal degradation of PKM zeta" and a compound "stimulating deubiquitination of PKM zeta" may each refer to a compound, the presence of which leads to reduced ubiquitination of PKM zeta. Preferably, such compounds are intended to refer to compounds that result in that ubiquitination of PKM zeta is reduced by at least 10%, 15%, 20%, 25%, 30%, more preferably by at least 35%, 40%, 45%, 50%, more preferably by at least 55%, 60%, 65%, 70%, more preferably by at least 75%, 80%, 85%; 90%, more preferably by at least 95%, 96%, 97%, 98%, or by at least 99%, such as by about 100%. As will be appreciated with the skilled person, such compounds may easily be identified by e.g. a method comprising a) providing a test system for monitoring ubiquitination of PKM zeta, and b) determining whether a given test compound leads to a reduced ubiquitination of PKM zeta in said test system. In certain embodiments, a compound "capable of interfering with the ubiquitination of PKM zeta" may interfere with or inhibit the action of an E1, E2 and/or E3 enzyme, preferably of an E3 enzyme, particularly of a PKM zeta specific E3 ligase.

Said inhibiting according to iii) and inhibiting according to iv) may also be referred to herein as compounds "capable of interfering with proteasome function".

Preferably, as used herein, a compound "capable of interfering with proteasome function", a compound inhibiting PKM zeta transport to or entry into the proteasome and a compound "inhibiting the assembly of the multiprotein complex involved in PKM zeta degradation" may each refer to a compound the presence of which leads to reduced proteasome function. As will be appreciated with the skilled person, such compounds may easily be identified, e.g. by techniques like BRET or FRET, capable of measuring protein distances if different proteins (ß1, ß2, ß5, AAA-Protein) of the complex are marked accordingly

In a third set of embodiments of the first aspect, said method comprises the step of determining whether a given test compound is capable of interfering with the binding of a peptide comprising the sequence FVRNSLERRSVRM and having a sequence selected from the group consisting of SEQ ID NOs 4, 6, 8, 10, 12, 14, 16, 18, 20, 22, 24, 26, 28, 30, 32, 34, 36, 38, 40, 42, or of a peptide derivative thereof, or of a peptide conjugate of the peptide or peptide derivative, to PKM zeta and/or PKC zeta. Accordingly, said peptide may have a sequence selected from the group consisting of SEQ ID NOs 4, 6, 8, 10, 12, 14, 16, 18, 20, 22, 24, 26, 28, 30, 32, 34, 36, 38, 40, 42. Likewise, said peptide may have a sequence as disclosed in FIG. 10. In a particular embodiment, said peptide has a sequence of 13 to 40, more preferably 15 to 40, such as 15 to 31 or 20 to 30, consecutive amino acids of SEQ ID NO: 42. Preferably, said peptide comprises the sequence FVRNSLERRSVRM (SEQ ID NO: 4). In some embodiments, said peptide comprises the sequence PPFVRNSLERRSVRMKRPSS (SEQ ID NO: 22). In another particular embodiment, said peptide has a sequence of n to 40 consecutive amino acids of SEQ ID NO: 42 and comprises a sequence selected from the group consisting of SEQ ID NOs 4, 6, 8, 10, 12, 14, 16, 18, 20, 22, 24, 26, 28, 30, 32, 34, 36, 38, 40, 42, wherein in each case, n corresponds to the total number of amino acids of the particular SEQ ID NO. Accordingly, said peptide may comprise or have a sequence of the amino acids from between residue 946, 948, 956 or 958 respectively, and residue 970, 972, 975 or 985, respectively of the human KIBRA. Individual preferred said peptides are e.g. those comprising or having a sequence of amino acids 946-970, 948-970, 956-970, 958-970, 946-972, 948-972, 956-972, 958-972, 946-975, 948-975, 956-975, 958-975, 946-985, 948-985, 956-985, 958-985 of (human) KIBRA. Preferably, said peptide is capable of binding to PKM zeta. Preferably, said peptide is capable of preventing PKM zeta from degradation, particularly from proteasomal degradation

Accordingly, the invention also concerns methods comprising determining whether a given test compound is capable of binding to the KIBRA binding site on PKM zeta.

As used herein, said "peptide conjugate of the peptide or peptide derivative" preferably refers to a peptide conjugate comprising said peptide or peptide derivative.

As used herein, a compound "capable of interfering with the binding" of certain other compounds is intended to refer to a compound the presence of which leads to reduced binding between said other compounds, i.e. between a peptide, peptide derivative or peptide conjugate according to the present invention and PKC zeta or PKM zeta. Numerous ways of binding of a peptide or peptide derivative to a protein such as PKM zeta are known to the skilled reader and include qualitative and quantitative assays (cf. hereinabove). Preferably, a compound "capable of interfering with the binding" of certain other compounds is intended to refer to a compound that results in that binding between said other compounds is reduced by at least 10%, 15%, 20%, 25%, 30%, more preferably by at least 35%, 40%, 45%, 50%, more preferably by at least 55%, 60%, 65%, 70%, more preferably by at least 75%, 80%, 85%; 90%, more preferably by at least 95%, 96%, 97%, 98%, or at least 99%. Generally herein, the order of addition of test compound and other components is not particularly limited. Hence, as one alternative, the test compound may be added to a combination of said other compounds. Alternatively, one of the other compounds may first be combined with the test compound, before the second other compound is added.

Various ways of determining whether a test compound is capable of interfering with the binding between peptides, polypeptides, proteins or the like are known to the skilled person. In preferred embodiments, the compounds are identified by an assay involving co-immunoprecipitation. That is, as a non-limiting example, co-immunoprecipitation of a peptide, peptide derivative or peptide conjugate disclosed herein, or the like, with PKM zeta or PKC zeta is assayed in the absence and presence of the test compound, or alternatively in the absence and presence of increasing amounts of the test compound. Further such methods and further details will be apparent to the skilled reader on the basis of the Examples disclosed herein. In a similar aspect, the present invention relates to a method for the identification of a compound being able to interact with PKC zeta and/or PKM zeta to identify a compound capable of inhibiting degradation of PKM zeta. In some embodiments, said method comprises the step of determining whether a given test compound is capable of interfering with the binding of a KIBRA protein or a derivative or conjugate thereof to PKC zeta and/or PKM zeta. Preferably, said compound being able to interact with PKC zeta and/or PKM zeta is a compound being able to bind to PKC zeta and/or PKM zeta.

Generally, as used herein, a "peptide" usually has from 5 to 40 amino acids, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 amino acids, and preferably from about 13 to about 35 amino acids, such as from about 15 to about 31 amino acids, such as from 20 to 30 amino acids, such as 25 amino acids. Generally, peptides can easily be obtained by the skilled person, such as by chemical synthesis, production in host cells, or combinations thereof.

As used herein, a "peptide derivative" may generally be derived from a given peptide disclosed herin in various manners. For example, it may be derived from said peptide in that it has a sequence that has a certain percentage of sequence identity with that of the corresponding peptide, comprises a sequence of said peptide, has certain residues in common with said peptide, and/or shares a certain activity with said peptide. A peptide derivative disclosed herein may itself be a peptide or may also be a polypeptide. The term "polypeptide" as used herein includes reference to a protein. Preferably herein, a "polypeptide", such as a polypeptide as an example of a peptide derivative disclosed herein, has at least 41, such as at least 42, 45, 50, 60, 70, 80, 90, particularly at least 100 amino acids. Preferably herein, a polypeptide does not contain more than 2000, such as not more than 500, 450, 400, 350, 300, 250, 200, or 150 amino acids. Peptide derivatives of the present invention may e.g. easily be obtained by the skilled person, e.g. by means of one or more amino acid substitution(s), addition(s) or deletion(s) or any combination thereof in amino acids of the peptides of the invention. The peptide derivative preferably comprises conservative substitutions or semi-conservative substitutions, i.e. substitutions by a member of a family of amino acids that are related in their side chains and chemical properties. Examples of such families, all of which are well-known to the skilled person, are families of amino acids with acidic side chains, basic side chains, non-polar aliphatic side chains, non-polar aromatic side chains, uncharged polar side chains, small side chains, bulky side chains and the like. Generally, a peptide derivative disclosed herein preferably contains from 13 to 500 amino acids, such as from 13 to 400, from 13 to 300, particularly from 13 to 200, from 20 to 200, from 13 to 100, from 20 to 100, more preferably from 13 to 50, from 15 to 50, from 20 to 50, from 13 to 45, from 13 to 40, even more preferably from 13 to 30, from 15 to 35, from 20 to 40, such as from 15 to 30, from 15 to 25 or from 17 to 23 amino acids ― or any range in between or combination between those numbers.

Preferably, said peptide derivative or peptide conjugate is generally selected from any one of the below A, B, C, D and E:
A) a peptide derivative,
   i) having a sequence that is at least 90 % identical to a sequence as defined herein; and/or
   ii) having a sequence of 13 to 200 amino acids that comprises a sequence as defined herein; and/or
   iii) having a sequence that is at least 70 % identical to a sequence as defined in herein and being capable of binding to PKM zeta; and/or
   iv) having a sequence of 13 to 500 amino acids that comprises a sequence as defined herein, and being capable of binding to PKM zeta; and/or
   v) having a sequence that is at least 80 % identical to a sequence as defined herein and comprising the amino acid residue E at the position corresponding to position 965 of the full-length sequence of human KIBRA (SEQ ID NO: 2) and/or
   vi) having a sequence that is at least 70 % identical to a sequence as defined herein and comprising the amino acid residues E, R, V at the positions corresponding to positions 965, 967, 969 of the full-length sequence of human KIBRA (SEQ ID NO: 2) and/or
   vii) having a sequence that is at least 60 % identical to a sequence as defined herein, comprising the amino acid residues E, R, V at the positions corresponding to positions 965, 967, 969 of the full-length sequence of human KIBRA (SEQ ID NO: 2), and being capable of binding to PKM zeta,
B) a peptide derivative,
   i) comprising D-amino acids having an inverse sequence of a sequence of a peptide as defined herein or of a sequence of peptide derivative according to A), and being capable of binding to PKM zeta; and/or
   ii) being a cyclic derivative of a peptide as defined herein or of a peptide derivative according to A); and/or
   iii) being a multimer of a peptide as defined herein or of a peptide derivative according to A),
C) a peptide derivative, particularly as defined in any of A) and B),
   comprising one or more covalent modification(s), particularly selected from the group consisting of acetylation, amidation, disulfide bond formation, formylation, glycosylation, methylation, phosphorylation, sulfatation, replacement of a peptide bond -(C=O)NH- by -(C=S)NH- and replacement of a peptide bond -(C=O)NH- by -(CH-CF₃)NH-,
D) a peptide conjugate comprising a peptide derivative as defined in any of A), B) and C),
   and/or
E) a peptide conjugate, particularly as defined in D), characterized in that it comprises
   i) a cell penetration moiety, particularly a moiety for crossing the blood brain barrier; and/or
   ii) a detectable label; and/or
   iii) a polymer; and/or
   iv) a membrane anchoring moiety; and/or
   v) a sequence interacting with the postsynaptic density, particularly with a PDZ motif.

In certain preferred embodiments, said compound capable of inhibiting degradation of PKM zeta is verified by means of a positive control in the presence of a peptide, peptide derivative or peptide conjugate as defined in the third set of embodiments of the first aspect or particularly in any of the above A, B, C, D and E. Also in this set of embodiments of the first aspect of the invention, said compound capable of inhibiting degradation of PKM zeta is preferably verified by means of a negative control in the absence of said test compound. Likewise, in some embodiments said compound capable of interfering with the binding of a given substance to PKC zeta and/or PKM zeta is verified by means of a negative control in the absence of said test compound.

Preferably, the methods of the invention may, for example, be performed in vivo, ex vivo or in vitro. Accordingly, a method of the invention may be an in vivo method, an ex vivo method or an in vitro method.

Percentages identity can easily be determined by the skilled person. As a non-limiting example, for a peptide of 20 amino acids, 70 % identity to said sequence according to the first aspect means that 14 out of 20 amino acids are identical.

Preferably, in a peptide derivative disclosed herein, a functionality of a peptide of the invention is maintained. For example, in certain preferred embodiments, a peptide derivative disclosed herein is capable of binding to PKM zeta. In certain preferred embodiments, a peptide derivative disclosed herein is capable of preventing PKM zeta from degradation, particularly from proteasomal degradation. As used herein, "a peptide being capable of binding to PKM zeta" is understood to also qualify as, and hence to also be, "a peptide being capable of binding to PKC zeta". Moreover, "a peptide being capable of binding to PKM zeta" is also understood to include "a peptide being capable of binding to PKM zeta and/or PKC zeta". Hence, in the embodiments herein, generally, the term "PKM zeta" may be replaced by "PKC zeta" and vice versa, or may be replaced by "PKM zeta and/or PKC zeta". Numerous ways of determining capability of binding of a peptide or peptide derivative to a protein such as PKM zeta are known to the skilled reader. Suitable qualitative and quantitative assays e.g. include, but are not limited those involving co-precipitation or surface plasmon resonance (SPR). Preferably, as used herein, a peptide that is capable of binding to PKM zeta is intended to mean a peptide that binds to PKM zeta with a dissociation constant K_{d}, where I/K_{d} is at least 10%, 15%, 20%, 25%, 30%, 35%, 40%, 45%, 50%, 55%, 60%, 65%, 70%, 75%, 80%, 85%; 90%, 95%, 96%, 97%, 98%, 99% of I/K_{d} of the K_{d} of a peptide according to the invention, particularly the K_{d} of a peptide corresponding to the respective peptide derivative or of the K_{d} of a peptide having SEQ ID NO: 22 (or KIBRA residues 956-975). In other embodiments, a peptide that is capable of binding to PKM zeta binds to PKM zeta even better than a peptide according to the invention, such as one having a sequence of SEQ ID NO: 4. Hence, a peptide that is capable of binding to PKM zeta may be a peptide that binds to PKM zeta with a dissociation constant K_{d} where I/K_{d} is at least 100%, 150%, 200%, 300%, 400%, 500%, 600%, 700%, 800%, 900% or 1000% of 1/K_{d} of the K_{d} of of a peptide according to the invention, particularly of the K_{d} of a peptide corresponding to the respective peptide derivative or of the K_{d} of a peptide having SEQ ID NO: 22.

In some preferred embodiments of this aspect, the peptide derivatives disclosed herein may alternatively or additionally be peptide derivatives i) comprising D-amino acids having an inverse sequence of a sequence of a peptide disclosed herein or of a sequence of a peptide derivative disclosed herein, and being capable of binding to PKM zeta; and/or ii) being a cyclic derivative of a peptide disclosed herein or of a peptide derivative disclosed herein,; and/or iii) being a multimer of a peptide disclosed herein or of a peptide derivative disclosed herein. Accordingly, the peptide derivative may also be derived from a peptide disclosed herein in that it has a similar three-dimensional structure or surface structure, respectively. Hence, the invention also employs a peptide derivative comprising D-amino acids having an inverse sequence of a sequence of a peptide disclosed herein or of a peptide derivative disclosed herein, and being capable of binding to PKM zeta. One example for such derivative is a peptide comprising, preferably consisting of, D-amino acids that have an inverse sequence of a sequence defined above. Hence, the peptide derivative disclosed herein may be characterized in that it consists of D-amino acids having an inverse sequence of a sequence defined herein, and is capable of binding to PKM zeta. As used herein, the latter peptide derivatives may also be referred to as a retro-inverso peptides / peptide derivatives of a peptide or peptide derivative disclosed herein or as a peptides / peptide derivatives having a retro-inverso form of a peptide or peptide derivative disclosed herein. Similarly, a "peptide derivative" herein may also be derived from a peptide disclosed herein e.g. in that it comprises a sequence of one or more peptides or peptide derivatives disclosed herein. The latter includes cyclic derivatives and multimers of a peptide disclosed herein and/or of a peptide derivative disclosed herein. Accordingly, also considered is a peptide derivative being a multimer of a peptide herein or of a peptide derivative described herein. Similarly, the invention also employs a peptide derivative being a cyclic derivative of a peptide disclosed herein or a peptide derivative disclosed herein.

Generally, the peptide, peptide derivative or peptide conjugate herein may be a chimeric peptide, chimeric peptide derivative or chimeric peptide conjugate. Chimeric peptides etc. are not particularly limited and are generally well-known to the skilled person. Also, the peptides, peptide derivatives and peptide conjugates disclosed herein may easily be obtained by the skilled person ― e.g. by methods involving chemical synthesis, production in host cells, or combinations thereof.

The peptides and peptide derivatives disclosed herein may comprise one or more covalent modification(s). Covalent modifications are well known to the skilled person and are not particularly limited. Preferably, said one or more covalent modification(s) are selected from the group consisting of acetylation, amidation, disulfide bond formation, formylation, glycosylation, methylation, phosphorylation, sulfatation, and isosteric replacement of a peptide bond, such as the replacement of -(C=O)NH- by -(C=S)NH- or the replacement of -(C=O)NH- by -(CH-CF₃)NH

The present invention may furthermore employ a peptide conjugate. Preferably, such peptide conjugate comprises a peptide or peptide derivative disclosed herein. As used herein, a "peptide conjugate" preferably refers to a conjugate that comprises a peptide or peptide derivative disclosed herein as well as an additional moiety. Said additional moiety is not particularly limited. Preferably, the additional moiety is covalently bound to a peptide or peptide derivative disclosed herein. Said additional moiety, for purposes herein, may also refer to more than one such moiety. Accordingly, peptide conjugate may also comprise more than one such moiety, such as two or more moieties of a given type of moieties and/or two or more moieties of different types of moieties. Said moieties are preferably selected from cell penetration moieties, such as moieties for crossing the blood brain barrier; detectable labels; polymers; membrane anchoring moieties; and sequences (such as peptide sequences) interacting with the postsynaptic density, such as sequences interacting with a PDZ motif. Accordingly, the peptide conjugate herein is preferably characterized in that it comprises: i) a cell penetration moiety; and/or ii) a detectable label; and/or iii) a polymer; and/or iv) a membrane anchoring moiety, and/or v) a sequence interacting with the postsynaptic density. The peptide conjugate disclosed herein may comprise any of the above i) to v) or any combination thereof.

Preferably, a peptide conjugate herein is characterized in that it comprises a cell penetration moiety. As used herein, a cell penetration moiety refers to a moiety that it is capable of entering into a cell. Preferably, it is capable of doing so even if comprised in a peptide conjugate disclosed herein. Said cell penetration moiety preferably is a cell penetration peptide (CPP) or a derivative thereof. Cell penetration peptides are well-known in the art. Non-limiting examples of preferred cell penetration peptides to be used in accordance with the present invention include cell penetration peptides having a sequence selected from the group consisting of the HIV-gp41 fusion sequence, the Kaposi FG signal sequence, the human integrin b3-signal sequence, RRRRRRR (SEQ ID NO: 43), RRRRRRRR (SEQ ID NO: 44), RRRRRRRRR (SEQ ID NO: 45), RRRRRRRRRR (SEQ ID NO: 46), a nuclear localization sequence, a HIV Tat-1-derived sequence, a SynB1 sequence, a buforin sequence, a magainin sequence, a sequence of the N-terminal repetitive domain of maize gamma-zein, a sequence of a lactoferrin-derived peptide, an antennapedia-derived sequence such as a penetratin sequence, the Isl-1 homeodomainm, KLALKLALKALKAALKLA (SEQ ID NO: 47), a transportan sequence, a chariot sequence, an MPG sequence like Pβ or Pα of gp41-SV40, a Pep-1 sequence (Trp-rich sequence of SV40), a Bac7 sequence (PRPLPFPRPG (SEQ ID NO: 48)), a VE-cadherin derived sequence like ₚVEC, a viral protein HSV-1 derived sequence like VP22, a protamine sequence, a PTD sequence including but not limited to PTD4 (YARAAARQARA (SEQ ID NO: 49)) and PTD5 (RRQRRTSKLMKR (SEQ ID NO: 50)), a PreS2-TLM sequence, a sequence of a calcitonin-derived peptide, an FAB fragment and an FC domain. Preferably, a cell penetration moiety herein is also capable of mediating trans-cellular transport such as the transport over the blood brain barrier. Preferably, it is capable of doing so even if comprised in a peptide conjugate disclosed herein. Hence, the cell penetration moiety herein preferably is a moiety capable of mediating trans-cellular transport. Hence, the cell penetration moiety herein preferably is enabling the peptide conjugate disclosed herein to cross the blood brain barrier. Such moiety may also be referred herein as "a moiety for crossing the blood brain barrier". Hence, preferably herein, the cell penetration moiety is a moiety for crossing the blood brain barrier.As used herein, said cell penetration moiety may also be a derivative of a cell penetration moiety, such as a derivative of a CPP. Said derivatives include but are not limited to i) a D-form of a cell penetration peptide, ii) a retro-inverso form of a cell penetration peptide (i.e. a CPP comprising, preferably consisting of, D-amino acids that have an inverse sequence of a sequence of a CPP), iii) a cell penetration peptide comprising one or more covalent modification(s) as described herein with relation to the peptides and peptide derivatives disclosed herein, iv) a small molecule mimicking a cell penetration peptide, e.g. a small molecule comprising a polyguanidine backbone.

In accordance with the invention, a peptide conjugate may comprise a detectable label. A detectable label is not particularly limited. Numerous detectable labels are well-known to the skilled person. Preferably, said detectable label is selected from the group consisting of the following tags: FLAG-tag, GST-tag, HA-tag, His-tag, V5-tag, myc-tag, Isopep-tag, BCCP, calmodulin-tag, maltose binding protein tag, Nus-tag, glutathione-S-transferase-tag, green fluorescent protein tag, S-tag, Softag 1, Softag 3, streptavidin tag, biotin tag, SBPtag, Ty tag, ACP-tag, CBP-tag, glutathione-S-transferase-tag (GST-tag), snap-tag, tandem affinity purification tag (TAP-tag), thioredoxin tag, GFP, YFP, and BFP. The detectable label may be a radioactive label or non-radioactive label. In accordance with the invention, a peptide conjugate may comprise a polymer. The polymer is preferably selected from the group consisting of PEG (polyethylene glycol), mPEG (methoxypolyethylene glycol), cyclodextrin, polyglutamate, polyaspartate and HPMA (N-(2-hydroxypropyl)methacrylamide). In accordance with the invention, a peptide conjugate may comprise a membrane anchoring moiety. The membrane anchoring moiety is preferably selected from the group consisting of a GPI-anchor, an isoprenyl-anchor (such as a farnesyl anchor or geranylgeranyl anchor), a myristoyl-anchor and a palmitoyl-anchor. In accordance with the invention, a peptide conjugate may comprise a sequence interacting with the postsynaptic density, such as a sequence interacting with a PDZ motif. The sequence interacting with the postsynaptic density is preferably selected from or comprises a PSD97 or PSD95 motif.

Generally, in certain preferred embodiments, the peptide, peptide derivative or peptide conjugate herein comprises the amino acid residue E at the position corresponding to position 965 of the full-length sequence of human KIBRA (SEQ ID NO: 2). In other preferred embodiments herein, the peptide, peptide derivative or peptide conjugate herein may alternatively or additionally comprise the amino acid residues R and V, respectively at the positions corresponding to positions 967 and 969, respectively, of the full-length sequence of human KIBRA (SEQ ID NO: 2 or Figure 9, respectively).

The native human KIBRA protein sequence referred to for purposes of the present invention, and sometimes abbreviated as "KIBRA" herein, is a full-length human KIBRA protein sequence, and is depicted in SEQ ID NO: 2 or Figure 9, respectively. Hence, reference to a sequence in SEQ ID NO: 2 also includes reference to a corresponding sequence in Figure 9.

In certain embodiments, the peptide derivative herein is not a full-length KIBRA protein, such as not a polypeptide of SEQ ID NO: 2 or Figure 9, respectively. In other embodiments, the peptide derivative herein is a full-length KIBRA protein, such as a polypeptide of SEQ ID NO: 2 or Figure 9, respectively.

In certain embodiments of the present invention, the peptide, peptide derivative and/or peptide conjugates disclosed herein, or a compound of the invention, do/does not consist of and/or comprise amino acids 919-978 and 897-1094, respectively, of the full-length mouse KIBRA sequence having a total of 1094 residues depicted in "Yoshihama et al., 2009". Likewise, in some embodiments, the peptide, peptide derivative and/or peptide conjugate disclosed herein, or a compound of the invention, does not consist of and/or comprise amino acids corresponding to amino acids 919-978 and 897-1094 of said full-length mouse KIBRA sequence.

These amino acids e.g. correspond to amino acids 927-986 and/or 905-1094 of KIBRA as disclosed in SEQ ID NO: 2 or Figure 9, respectively, as can easily be determined by the skilled person by means of a sequence alignment. Hence, similarly, in certain embodiments, the peptide, peptide derivative and/or peptide conjugate disclosed herein, or a compound of the invention, do not consist of and/or comprise amino acids 927-986 and/or 905-1094 of KIBRA. Similarly, in certain embodiments, the peptide, peptide derivative and/or peptide conjugate disclosed herein, or a compound of the invention, does not consist of and/or comprise amino acids corresponding to amino acids 927-986 and/or 905-1094 of KIBRA. Likewise, in certain embodiments, the peptide, peptide derivative and/or peptide conjugate disclosed herein, or a compound of the invention, does not consist of and/or comprise the amino acids a partial KIBRA sequence as disclosed in Yoshihama et al., 2009.

Generally herein, reference to a sequence in SEQ ID NO: 2 also includes reference to a corresponding sequence in Figure 9. Likewise, reference to a sequence in SEQ ID NO: 1 generally also includes reference to a corresponding sequence in Figure 9.

In a second aspect, the present invention relates to a compound capable of inhibiting degradation of PKM zeta, identified by a method according to the first aspect.

Particular embodiments of such compounds capable of inhibiting degradation of PKM zeta which have been identified by the present inventors are the compounds that have been described hereinabove in context with the methods of the third set of embodiments of the first aspect.

In a third aspect, the present invention relates to a compound of the second aspect for use in medicine.

In a fourth aspect, the present invention relates to a compound of the second aspect for use in a specific methods for treatment of the human or animal body by surgery or therapy or for use in a diagnostic method practiced on the human or animal body.

Preferably, said methods are selected from the group consisting of i) a method for improving cognition; ii) a method for improving learning; iii) a method for improving memory; iv) a method for improving motor learning and/or sensory motor recovery or for treating diseases which benefit from improved motor learning and/or improved sensory motor recovery; v) a method for treating or improving neuropsychological deficits after stroke; vi) a method for counteracting memory loss; vii) a method for improving or treating a memory deficit; viii) a method for improving or treating a dementia; ix) a method for improving or treating age-associated cognitive decline; x) a method for improving or treating mild cognitive impairment (MCI); xi) a method for improving or treating a brain trauma; and xii) a method for treating cancer.

The fourth aspect also includes the use of a compound of the invention in a method, particularly a non-therapeutic method, for improving cognition.

According to the fourth aspect, compounds of the invention are envisaged for use in any of the above methods on their own, or in any combination of said methods. Likewise, according to the fourth aspect, also any of the particular examples for the above methods described below are envisaged separately or in any combination thereof. As will be appreciated by the skilled person, herein, a treatment of a particular of the above methods does not exclude that the treatment simultaneously qualifies as a treatment of one or more other such methods, which may be due to certain (natural) overlaps.

Preferred embodiments / methods relating to the fourth aspect are described in the following:
Said method for improving memory preferably includes, but is not limited to a method for improving declarative memory; a method for improving episodic memory; a method for improving semantic memory; a method for improving non-declarative memory; and a method for improving long term memory.
Said method for improving motor learning and/or sensory motor recovery or for treating diseases which benefit from improved motor learning and/or improved sensory motor recovery preferably includes, but is not limited to a method for improving the treatment of or for treating diseases and conditions like Stroke and/or rehabilitation after stroke, spinal cord trauma and/or rehabilitation after spinal cord trauma, rehabilitation after major surgery, rehabilitation after orthopedic surgery, rehabilitation after brain surgery.
Said method for treating or improving neuropsychological deficits after stroke includes, but is not limited to method for treating or improving neuropsychological deficits such as agnosia, apraxia, anosognosia, prosopagnosia, hemineglect.
Said method for improving or treating a memory deficit includes, but is not limited to a method for improving or treating memory deficits following stroke (ischemic, hemorrhagic, subarachnoidal hemorrhage); a method for improving or treating memory deficits accompanying Parkinson's disease; a method for improving or treating memory deficits accompanying Huntington's disease; a method for improving or treating memory deficits accompanying Schizophrenia: a method for improving or treating memory deficits accompanying depression; a method for improving or treating memory deficits following radiation or chemotherapy to the brain; a method for improving or treating memory deficits following perinatal hypoxic conditions; a method for improving or treating memory deficits ensuing from infection of the brain (such as meningitis, viral encephalitis, limbic encephalitis, Hashimoto's encephalopathy, cerebral vasculitis); a method for improving or treating memory deficits accompanying tumours in the brain (such as lymphomas, metastatic tumours, gliomas); a method for improving or treating memory deficits accompanying hypothyroidism; a method for improving or treating memory deficits accompanying an encephalopathy; a method for improving or treating memory deficits accompanying Wilson's disease; a method for improving or treating memory deficits accompanying hydrocephalus (including normal pressure hydrocephalus); a method for improving or treating memory deficits following major surgery, especially neurosurgery, carotid surgery, heart surgery; a method for improving or treating memory deficits accompanying epilepsy, especially uncontrolled epilepsy and frequent seizures.
Said method for improving or treating a dementia deficit includes, but is not limited to a method for improving or treating alcohol dementia, Wernicke-Korsakoff Syndrome; a method for improving or treating dementia following hypothyroidism; a method for improving or treating dementia accompanying HIV-infection; a method for improving or treating vascular dementia; a method for improving or treating Lewy-body dementia; a method for improving or treating Morbus Binswanger; a method for improving or treating fronto-temporal dementia (FTD); a method for improving or treating Alzheimer's disease.
Said method for improving or treating a brain trauma also includes a method for improving or treating a mild (brain) trauma, e.g. boxer's syndrome (dementia pugilistica).
Said cancer preferably includes, but is not limited to carcinomas derived from epithelial cells (such as breast cancer, prostate cancer, lung cancer, pancreatic carcinoma, colon carcinoma), sarcomas derived from connective tissue (such as osteosarcoma, chondrosarcoma, liposarcoma, leiomyosarcoma, rhabdomyosarcoma), lymphomas and leukemias derived from hematopoietic (blood-forming) cells (such as Hogdkin lymphoma, multiple myeloma), germ cell tumors derived from pluripotent cells (such as seminoma), blastoma derived from immature cells or embryonic tissue (such as hepatoblastoma, medulloblastoma, nephroblastoma, neuroblastoma, pancreatoblastoma, pleuropulmonary blastoma, retinoblastoma).

As it is known to the skilled person, cognition is a term subsuming a broad variety of mental activities that are associated with the general processing of information. This information processing is central to consciousness, perception, learning and memory, associative thinking, reasoning based on rational judgment and deduction logic, problem-solving, conceptual thinking, knowing, planning, imagination, language, music, emotionality, humor, or motor learning. In other words, cognition refers to all higher functions of the central nervous system. The most intensely studied of these phenomena are learning and memory, partially owing to their amenability to animal studies. Memory itself can be sub-classified into different systems. One of those uses the time span of storage, and therefore propagates the existence of short-term and long-term memory. Other systems divide memory based on the type of information stored into non-declarative and declarative memory. An example for non-declarative memory is procedural memory, which stores information about motor execution (e.g. swimming). The term declarative or explicit memory refers to memories that can be consciously recalled, and is subdivided into episodic and semantic memory. Episodic memory is the memory for biographical events ordered into time and space. Semantic memory is the memory of facts about the outside.

To further describe particular methods in context with the fourth aspect, and their relation etc. to the present invention, the following is observed:
Agnosia is a neurological condition which results in an inability to know, to name, to identify, and to extract meaning from visual, auditory, or tactile impressions (Rhawn 2000). Apraxia is a movement disorder involving the control and sequencing of skilled fine and gross motor skills in the absence of impaired motor functioning or paralysis and is commonly associated with injuries to the parietal lobe (Rhawn, 2000). Anosognosia is a physiological rooted condition, like damage to the frontal or parietal lobe due to illness and disease, in which a person who suffers disability seems unaware of the existence of his or her disability. Around 10% - 18% of acute hemi paretic stroke patients suffer from anosognosia (Baier et al., 2005). Prosopagnosia, also called face blindness, is an impairment in the recognition of faces, which is often accompanied by other types of recognition impairments like place recognition, car recognition or facial expression of emotion, though sometimes it appears to be restricted to facial identity. Not surprisingly, prosopagnosia can create serious social problems. Patients suffering from prosopagnosia often have difficulty recognizing family members, close friends, and even themselves. Most of the documented cases are due to brain damage suffered after maturity from head trauma, stroke, and degenerative diseases (cf. www.faceblind.org/research/index.html). Hemineglect - also called spatial neglect or hemi-inattention - is a disorder of space-related behaviour. It is characterized by failure to explore the side of space contralateral to a brain lesion, or to react or respond to stimuli or subjects located on this side (Karnath, 2002)).

Morbus Binswanger or Binswanger's disease is a form of vascular dementia characterized by diffuse white matter lesions, accompanied with varying clinical findings which typically include a progressive dementia, depression and "subcortical" dysfunction such as gait abnormalities, rigidity and neurogenic bladder (Olsen, 1998). Morbus Alzheimer or Alzheimer's disease (AD) is a well-known, but incurable, neuro-degenerative disease characterized by the loss of neurons and synapses in the cerebral cortex and subcortical regions. Biochemically it is defined by accumulation of abnormally folded A-beta and tau proteins. Fronto-Temporal-Dementia (FTD) is an early-onset dementia caused by degeneration of the frontal lobe and may extend back to the temporal lobe. FTD is a member of the emerging group of proteinopathies (Haberland, 2010). Vascular dementia refers to a subtle, progressive decline in memory and cognitive functioning. It occurs when the blood supply carrying oxygen and nutrients to the brain is interrupted by a blocked or diseased vascular system. Vascular Dementia is the most common form of dementia in elderly in Asia and the second most common form of dementia in the US and Europe (cf. www.helpguide.org/elder/vascular_dementia.htm). Lewy-Body Dementia or Dementia with Lewy bodies (DLB) is the second commonest cause of neurodegenerative dementia in older people. It is part of the range of clinical presentations that share a neuritic pathology based on abnormal aggregation of the synaptic protein alpha-synuclein. (McKeith et al., 2004) Due to a loss of dopamine producing neurons - similar to Parkinson's disease - and a loss of acetylcholine producing neurons similar to that seen in Alzheimer's disease DLB patients suffer from cognitive-, emotional-, and motor disorders. Alcohol dementia, Wernicke-Korsakoff syndrome or alcoholic encephalopathy is a manifestation of thiamine (vitamin B₁) deficiency, usually secondary to alcohol abuse and characterized by confusion, nystagmus, ophthalmoplegia, anisocoria, ataxia, sluggish papillary reflexes and severe memory loss (cf. www.nlm.nih.gov/medlineplus/ency/article/000771.htm). As to dementia accompanying HIV-infection, it is observed that far in advance of end-stage acquired immunodeficiency syndrome (AIDS), HIV infection is associated with neurocognitive deficits and anatomic and functional brain abnormalities. Evidence supports a key role for the virus. Neurological dysfunction correlates with elevated levels of virus in the CSF. High viral load does not necessarily correlate with dementia, but monocytes or macrophages (which are derived from monocytes) also may be required. Thus, considerable evidence supports a necessary role for the virus in directly or indirectly mediating central nervous system disease (Major et al., 2000).

Mild Cognitive Impariment (MCI) is a syndrome defined as cognitive decline greater than expected for an individual's age and education level but that does not interfere notably with activities of daily life. The amnestic subtype of mild cognitive impairment has a high risk of progression to Alzheimer's disease (Gauthier, et al., 2006).

Age-associated cognitive decline - or normal (non-pathological, normative, usual) cognitive ageing - is an important human experience which differs in extent between individuals (Deary, et al., 2009).

Mild Brain Trauma is also referred to as concussion, is considered the most underreported, underdiagnosed and underestimated head trauma injury. Cumulative and repetitive head trauma caused by repetitive blows to the head over a long period of time often lead to dementia pugilistica (boxer's syndrome) a form of chronic traumatic brain injury mostly accompanied by some or all of the following symptoms: intolerance to loud noise, depression, confusion, unsteady walk, improper behavior, disorientation, double vision, poor concentration, speech problems, loss of memory, headache, dizziness (cf. www.depression-guide.com/dementia-pugilistica.htm).

Morbus Parkinson or Parkinson's disease (PD) is a progressive neurological disorder characterised by a large number of motor and non-motor features that can impact on function to a variable degree. The pathology of the disease is characterized by the accumulation of alpha-synuclein into inclusions (Lewy bodies) in neurons, and from insufficient formation and activity of dopamine in certain neurons in parts of the midbrain. Patients with PD are at almost six fold increased risk for dementia (Aarsland, et al., 2001). Morbus Huntington or Huntington's disease (HD) is a neurodegenerative disorder caused by an autosmal dominant mutation on either of an individual's two copies of the Huntingtin gene As the disease progresses, memory deficits tend to appear. Reported impairments range from short-term memory deficits to long-term memory difficulties, including deficits in episodic, procedural and working memory. (Montoya, et al., 2006). Schizophrenia is a chronic, severe, and disabling brain disease. Approximately 1 percent of the population develops schizophrenia during their lifetime. Developmental neurobiologists funded by the National Institute of Mental Health (NIMH) have found that schizophrenia may be a developmental disorder resulting when neurons form inappropriate connections during fetal development (NIH Publication No. 02-3517). Depression is a state of low mood and aversion to activity. Many people suffering from this chronic illness lose their appetites, their ability to sleep normally, their sex drive, and the very ability to enjoy the simplest pleasure. Also memory, one of the higher brain functions is badly affected by depression (cf. www.memorylossonline.com/summer2001/depression.html). Hypoxic conditions may cause HIE, hypoxic-ischemic encephalopathy, a serious condition that causes significant mortality and long-term morbidity. Hypoxic-ischemic encephalopathy is characterized by clinical and laboratory evidence of acute or subacute brain injury due to asphyxia, i.e., hypoxia or acidosis (cf. emedicine.medscape.com/article/973501-overview). Hypothyroidism is a condition in which the thyroid gland does not produce enough thyroid hormone Prenatal, the adequate functioning of both the maternal and fetal thyroid glands play an important role to ensure that the fetal neuropsycho-intellectual development progresses normally (Glinoer, 2000). The hallmark of encephalopathy is an altered mental state. Depending on the type and severity of encephalopathy, common neurological symptoms are progressive loss of memory and cognitive ability, subtle personality changes, inability to concentrate, lethargy, and progressive loss of consciousness. Other neurological symptoms may include myoclonus (involuntary twitching of a muscle or group of muscles), nystagmus (rapid, involuntary eye movement), tremor, muscle atrophy and weakness, dementia, seizures, and loss of ability to swallow or speak. Blood tests, spinal fluid examination, imaging studies, electroencephalograms, and similar diagnostic studies may be used to differentiate the various causes of encephalopathy (www.ninds.nih.gov/disorders/encephalopathy/encephalopathy.htm). Wilson's disease (WD) is a rare inherited disorder in which excessive amounts of copper accumulate in the body. The build-up of copper leads to damage in the kidneys, brain, and eyes. The primary consequence for most of those with WD is liver disease, but some Individuals may also experience clumsiness (ataxia) and loss of fine motor skills. If not treated, WD can cause severe brain damage, liver failure, and death (cf. www.ninds.nih.gov/disorders/wilsons/wilsons.htm). As to memory deficits accompanying epilepsy, uncontrolled epilepsy and frequent seizures, it is observed that attention is commonly affected in people with epilepsy and that epilepsy can reduce attentional speed or rate of information processing (cf. www.epilepsy.com/articles/ar_1063660416).

As to said methods of treating cancer, it is observed that recent studies suggested a role of PKC zeta in the preservation of normal cell polarization and cell differentiation. Whyte et al. 2001, demonstrated, that cells overexpressing a complete kinase-deficient PKC zeta display a cell polarizing deficit and that low level of PKC zeta mRNA expression is associated with poor outcome in a cohort of 295 breast cancer patients. In a model for the study of cellular differentiation, Kim et al., 2001, found, that the PMA induced monocyte/macrophage-like differentiation of human myelocytic leukemia cell line HL-60 also elevates the activity of PKC zeta. Furthermore PKC zeta deficient mice display an increased Ras-induced lung carcinogenesis suggesting a role for this kinase as a tumor suppressor by repression of the interleukin-6 promoter in vivo (Galvez et al., 2009). In summary, these findings that an increased PKC zeta/PKM zeta activity maintains normal cell differentiation and suppresses tumor and cancer formation suggest that an increased PKC zeta/PKM zeta activity maintains normal cell differentiation and therefore is an attractive approach to suppress formation of tumors and cancer. Accordingly, preventing PKC zeta/PKM zeta from degradation is considered to extend the availability of these peptide kinases and therefore to increase the activity of PKC zeta/PKM zeta and, hence, is considered to be an attractive approach to maintain cell differentiation and suppress cancer formation.

In case and as far as a method disclosed herein (and particularly in context with the fourth aspect) is not a method for the treatment of the human or animal body by surgery or therapy and/or is not a diagnostic method practiced on the human or animal body, the present invention preferably also relates to such method per se or to the use of a compound of the invention in such method - and not only to a compound of the invention for use in such method.

In certain other embodiments, the present invention explicitly involves methods for the treatment of a subject, such as a method for treating a subject in need thereof comprising administering to said subject a compound of the invention in an effective amount. Preferably, said method is selected from the preferred methods described in the context with the fourth aspect.

In an fifth aspect, the present invention relates to a composition comprising a compound of the invention, particularly of the second aspect. Said composition may be a pharmaceutical composition. Accordingly, said aspect preferably relates to a pharmaceutical composition comprising a compound of the second aspect, preferably together with a pharmaceutically acceptable carrier.

Preferably, said pharmaceutically acceptable carrier is selected from any of the carriers described hereinbelow ― likewise, the pharmaceutical compositions of the invention may be characterized by any of the following features or any combinations thereof.

For example, the pharmaceutical compositions of the invention may be contained in capsules, tablets, etc. all of which are readily known to the skilled person. Same may be coated e.g. with an EC comprising fatty acids, waxes, shellac, plastics, plant fibers, any of the EC described below, or any combination(s) thereof.

In detail as to the pharmaceutical compositions herein, e.g. depending on the route of administration, the uptake may benefit from varying carriers. Oral administration of protein drugs may be complicated by proteolytic degradation in the gastrointestinal tract. The proteolytic activity is commonly highest in the stomach and duodenum. In the stomach, pepsin cleaves peptide bonds between aromatic amino acids such as phenylalanine, tyrosine and tryptophan. This problem can be circumvented by delivering a peptide / protein solution in acid-resistant (gastro-resistant) capsules. An example are "DRcaps acid resistant hypromellose capsules" (Capsugel). Likewise, tablets containing the peptide can be coated by "enteric coating" (EC). EC can comprise or consist of fatty acids, waxes, shellac, plastics, and/or plant fibers.

Next to passive or active diffusion through the trans- or paracellular route in the intestine, peptides or proteins may also be taken up by specific receptor-mediated transport. Therefore, specific binding motifs to receptors present in the intestine can be fused to the peptide of interest. Accordingly, the compounds of the invention may be fused to such binding motifs. Corresponding conjugates are comprised by the compounds of the present invention.

Appropriate carrier formulations can be used in addition to EC that improve oral bioavailability of compounds such as peptides or proteins by protecting them from degradation, enhance uptake into the intestinal mucosa, and increase absorption across biological membranes. Examples for EC materials are: polyvinyl acetate phthalate (PVAP), cellulose acetate trimelitate (CAT), hydroxypropyl methylcellulose phthalate (HPMCP), hydroxypropyl methylcellulose acetate succinate (HPMCAS), methacrylic acid copolymer, Type C (Eudragit L100-55), Methacrylic acid copolymer dispersion (Eudragit L30D-55), methacrylic acid copolymer, Type A, (Eudragit L-100 and Eudragit L12,5), cellulose acetate phthalate (CAP) (Aquateric), methacrylic acid copolymer, type B, Eudragit S-100 and Eudragit S12,5, shellac (Marcoat 125 & 125 N). In addition to EC, polysaccharides, lectins (for example isolectin B4), chitosan, or other synthetic polymers are envisaged and may be of particular benefit for mucosal protein delivery systems.

Desired properties for a peptide carrier include the maintenance of the biological activity of the peptide of interest, a high biocompatibility, the ability to incorporate high peptide concentrations, the possibility to target specific cells or tissues, and release kinetics that can be adapted to reach desired pharmacokinetic properties. Examples for corresponding carriers are frequently in the form of nano- or microparticles, such as those with mean diameters ranging from 0.1 to 30 µm:
Nanosized polyelectrolyte complexes (PECs), which may be formed by self-assembly of proteins with natural and synthetic polymers by mixing oppositely charged drug and polymer that will interact by electrostatic-attraction. These can for example consist of chitosan, the peptide of interest, and other excipients such as tripolyphosphate (TPP), magnesium sulfate, sodium alginate or cyclodextrin (CD).
Poly(amidoamine)s (PAAs), which are cationic carriers. PAAs are synthetic tert-amino polymers obtained by stepwise polyaddition of primary or secondary aliphatic amines to bisacrylamides. These polymers are water-soluble, biodegradable and biocompatible, with lower cytotoxicity profiles than other polycationic vectors. Novel linear PAAs are available containing repetitive disulfide linkages in their backbone (so-called SS-PAAs). These SS-PAAs have the advantage of being relatively stable in the extracellular medium but are fast degraded in the reductive intracellular environment due to reduction of the disulfide bonds.

Lipid-based drug delivery systems, for example liposomes, mixed micelles, emulsions, micro-/nanoemulsions, self-emulsifying drug delivery systems (SEDDS), solid lipid nanoparticles (SLN), suspensions, or phospholipid-drug complexes. Liposomes can consist of distearoyl phosphadylcholine. Liposomes can be coated with mucoadhesive polymers like carbopol and chitosan. A detailed and comprehensive listing of all types and ingredients of lipid drug delivery formulations can be viewed at *"Fricker et al., (2010)",* which is hereby incorporated by reference in its entirety.

The following components may be used in different (lipid) drug delivery approaches: Acetylsalicylic acid drug/lecithin associates, alpha-tocopherol lipid emulsion, amphotericin B cochleate (solid lipid nanoparticles), camptothecin solid lipid nanoparticles, chloroproguanil, hydrochloride-dapsone-artesunate, curcumin, cyclosporin A, lecithin, ethanol, hydroxysafflor yellow A drug-phospholipid complex oil solution, phosphatidylcholine-conjugate, lipopolysaccharide; gydrogel systems, e.g. composed of a copolymer of methacrylic acid (MAA) with additions of polyethylene glycol (PEG), N-vinyl pyrrolidone, poly(itaconic acid) (PIA), glycogen, glycerol; polyester-based matrices, for example derived from poly(lactic acid) (PLA) and/or PLGA polymers; nanoparticles based on chitosan, poly(isobutylcyanoacrylate), acrylic acid-based copolymers. Nanoparticles and liposomes may also contain an add-on of a cell-penetrating peptide (CPP), such as listed elsewhere in this document. In addition, targeting moieties may be added to nanoparticles or liposomes, for example vitamin B12 or folic acid or transferrin.

Commonly used biodegradable excipients in all those approaches include: amylose α- 1,4 D-glucose, arabinogalactone β-1,4 & β-1,3 D- galactose, β-1,6 & β-1,3 D-arabinose and D-galactose, Natural pectin, hemicelluloses, chitosan, deacetylated β-1,4 N-acetyl D-glycosamine, deacetylated chitin, chondroitin sulfate B-1,3, D-glucoronic acid & N-acetyl D-glycosamine, mucosopolysaccharides containing sulphate ester group at 4 or 6 position, cyclodextrane α- 1,4 D-glucose, Dextran α- 1,6 D-glucose α- 1,3 D-glucose, Guar gum α- 1,4 D-mannose α- 1,4 D-galactose, galactomannan, pectin α- 1,4 D-galactouronic acid and 1,2 D- Rhamnose with D-galactose & D-arabinose side chains, xylan β-1,4 D-xylose with β-1,3 L-arabinose side chains.

Specific peptide / protein delivery systems are for example manufactured by Emisphere Technologies, Inc, and known as Eligen™ technology based on sodium N-[8-(2-hydroxybenzoyl)aminocaprylate] (SNAC). Other suitable proprietary systems include: CLEC, Bio-Oral, Oral-Lyn, HIM2, Oradel, Macrulin, Orasome.

All of the above described carriers, coatings, excipients, systems, etc., and any combination(s) thereof, are envisaged as embodiments for the pharmaceutical compositions and (medical) methods and uses described herein.

Generally, in the (medical) methods and uses described herein and for the medical indications described herein, such as those of the third, fourth and fifth aspects, the compounds of the invention, such as those contained in a pharmaceutical composition of the invention, may e.g. be administered orally such as in the form of pills, tablets, such as lacquered or sugar-coated tablets, granules, gelatin capsules having a soft or hard shell, solutions such as aqueous, alcoholic or oily solutions, syrups, emulsions or suspensions. Administration may also be carried out parenterally, for example via injection or infusion, and includes subcutaneous, intramuscular and intravenous injection or infusion. Other suitable administration modes are, for example, percutaneous, topical, rectal, via inhalation, via microcapsules, via implants or via rods. The latter include, but are not limited to ointments, tinctures, sprays, transdermal therapeutic systems, suppositories. Preferred administration forms will e.g. depend, on the disease to be treated and its severity and can easily be determined by the skilled practitioner.

In generally preferred embodiments herein, the compounds of he invention, such as those contained in a pharmaceutical composition of the invention, may e.g. be administered via a route including, but not limited to a route selected from parenteral (s.c., i.v.), oral, buccal, transdermal, pulmonary, intranasal, intraocular, rectal and vaginal routes. For systemic application or delivery to the brain the oral and nasal routes are the most promising alternatives to parenteral administration. Accordingly, oral, nasal and parenteral administration is particularly preferred.

In a sixth aspect, the present invention relates to a method for stabilizing PKM zeta comprising a step selected from the group consisting of i) a step of contacting PKM zeta with a compound capable of inhibiting degradation of PKM zeta; ii) a step of contacting PKM zeta with a compound capable of interfering with the binding of a peptide as defined herein or of a peptide derivative or peptide conjugate thereof disclosed herein to PKC zeta and/or PKM zeta; ii*) a step of contacting PKM zeta with a peptide, peptide derivative or peptide conjugate disclosed herein, iii) a step of contacting PKM zeta with a compound capable of interfering with ubiquitination of PKM zeta, particularly iii)1) a step of contacting PKM zeta with a compound capable of inhibiting a E3-, E2-, E1-Ligase involved in proteasomal degradation of PKM zeta, or iii)2) a step of contacting PKM zeta with a compound capable of stimulating deubiquitination of PKM zeta; iv) a step of contacting PKM zeta with a compound capable of inhibiting PKM zeta transport to or entry into the proteasome and v) a step contacting PKM zeta with a compound capable of inhibiting the assembly of the multiprotein complex involved in PKM zeta degradation.

Preferably, said compound has been identified by a method of the invention, particularly of the first aspect of the invention. Generally, the compounds of the second, third, fourth, fifth or sixth aspect have preferably been identified by a method according to the first aspect of the invention.

In certain embodiments, a compound of the invention, such as a compound as defined in the sixth aspect, is not a full-length KIBRA protein, such as not a polypeptide of SEQ ID NO: 2 or Figure 9, respectively. In other embodiments, the compound of the invention I not s a full-length KIBRA protein, such as a polypeptide of SEQ ID NO: 2 or Figure 9, respectively.

In certain embodiments of the present invention, a compound of the invention, do/does not consist of and/or comprise amino acids 919-978 and 897-1094, respectively, of the full-length mouse KIBRA sequence having a total of 1094 residues depicted in "Yoshihama et al., 2009". Likewise, in some embodiments, a compound of the invention does not consist of and/or comprise amino acids corresponding to amino acids 919-978 and 897-1094 of said full-length mouse KIBRA sequence.

These amino acids e.g. correspond to amino acids 927-986 and/or 905-1094 of KIBRA as disclosed in SEQ ID NO: 2 or Figure 9, respectively, as can easily be determined by the skilled person by means of a sequence alignment. Hence, similarly, in certain embodiments, a compound of the invention does not consist of and/or comprise amino acids 927-986 and/or 905-1094 of KIBRA. Similarly, in certain embodiments, a compound of the invention does not consist of and/or comprise amino acids corresponding to amino acids 927-986 and/or 905-1094 of KIBRA. Likewise, in certain embodiments, a compound of the invention does not consist of and/or comprise the amino acids of a partial KIBRA sequence as disclosed in Yoshihama et al., 2009.

Likewise, in related embodiments of the sixth aspect, the invention relates to a compound of the invention ― particularly a compound that has been identified by a method according to the first aspect of the invention ― for use in a method for stabilizing PKM zeta, particularly as further defined hereinabove in relation to the sixth aspect.

### EXAMPLES

Next, the present invention is further described by reference to the following, non-limiting examples.

### Example 1: Particular Materials and Methods

Animal experiments. Male Wistar rats (250g bodyweight) were used. They were housed at constant temperature (23°) and relative humidity (60%) with a fixed 12 h light / dark cycle and ad libitum access of food and water. AAV vectors are injected bilaterally at 4 sites per adult hippocampus. For each hippocampus a total volume of 4 µl was injected of a stock solution of 7 x 10⁹ number of particles per millilitre. Open field, Morris water maze, and 8-arm maze were monitored by proprietary camera setup and software (SYGNIS tracker). All animal experiments were conducted in a blinded and randomized fashion. Animal experiments were approved by the responsible authorities (Regierungsprasidium Karlsruhe, Germany).

**Plasmids/Constructs.** The expression plasmids (pExp_NterFlag_PKMζ_hu; pExp _Ntermyc_KIBRA_hu; pExp_nV5_KIBRA; pExp_NterFlag_KIBRA hu; pexp_nV5_PKMζ_hu; pExp_NterFlag_PKCζ_hu) were constructed by PCR with Phusion DNA polymerase (New England Biolabs). We modified the respective PCR products with attB1/2 recombination sites compatible for the Gateway recombination cloning system (Invitrogen). The amplified ORFs were recombined with a gateway donor vector to obtain a Gateway-compatible ENTRY clone. Another recombination of the ENTRY clones with the respective DEST vectors revealed the expression plasmids (CMV promoter). All Gateway related reagents and plasmids were from Invitrogen and Gateway cloning procedures were performed according to protocols provided by the manufacturer.

**AAV constructs and generation of AAV 1/2 virus.** Vectors intended for small hairpin RNA (shRNA) expression were based on the AAV2 ITR-flanked shRNA expression cassette pAM/U6-pl-CBA-hrGFP-WPRE-BGHpA described earlier *(Szumlinski, et al. 2005).* For knock-down of Kibra, a target sequence at position 1276 of the Kibra ORF (GAT CCG TTG AAG TTA AAC AGC AAG ATT CAA GAG ATC TTG CTG TTT AAC TTC AAC CTT TTT TGG AAA) (SEQ ID NO: 51) was used. HEK293 cells were used for the production of pseudotyped chimeric AAV 1/2 vectors (containing a 1:1 ratio of capsid proteins serotype 1 and 2) as described previously *(Szumlinski, et al. 2005).* The genomic titre of the viral solutions was determined by real-time PCR (light cycler, Roche diagnostics). In more detail, vectors intended for small hairpin RNA (shRNA) expression were based on the AAV2 ITR-flanked shRNA expression cassette pAM/U6-pl-CBA-hrGFP-WPRE-BGHpA described earlier *(Franich, et al. 2008),* which facilitates humanized renilla GFP reporter gene expression from a CBA hybrid promoter along with shRNA expression from a RNA polymerase III compatible human U6 promoter. For knock-down of Kibra transcript levels, a target sequence at position 1276 of the Kibra ORF (GAT CCG TTG AAG TTA AAC AGC AAG ATT CAA GAG ATC TTG CTG TTT AAC TTC AAC CTT TTT TGG AAA) (SEQ ID NO: 51) was identified with Invitrogen's BLOCK-iT™ RNAi Designer web tool, and complementary DNA oligonucleotides encoding a shRNA directed against this target sequence were generated using Ambion's pSilencer™ Expression Vectors Insert Design Tool. For the loop structure, sequence GTG AAG CCA CAG ATG was used as described previously by Zeng & Cullen *(Zeng et al., 2004).* Annealed oligos were then BamHI x HindIII subcloned into the polylinker site. The resulting vector was termed AAV-(rat)-KIBRA RNAi. AAV-eGFP is used as control vector. Generation of AAV eGFP was performed by subcloning the coding sequence of eGFP into the AAV2 backbone plasmid containing the chicken β-actin promoter and an IRES-eGFP sequence, flanked by AAV2 ITR sequences. HEK293 cells were used for the production of pseudotyped chimeric AAV1/2 vectors (containing a 1:1 ratio of capsid proteins serotype 1 and 2) as described previously *(Klugmann, et al. 2005).* Cultured cells (80% confluent) propagated in complete DMEM were transfected with the AAV construct and helper plasmids (pH21, pRV1 and pFΔ6) using calcium phosphate. 48h later, cells were harvested in PBS, centrifuged, and pellets from 5 plates were pooled in 25 mL of a buffer consisting of 150 mM NaCl, 20 mM Tris pH8, 1.25 mL of 10% sodium deoxycholate and 50 U/mL of benzonase. After an incubation of 1 hour at 37°C, 25 mL of 150 mL NaCl and 1.25 mL of 10% sodium deoxycholate were added and the solution was centrifuged. The supernatant was collected and filtered with 450 mM NaCl, 20 mM Tris pH 8 through a high affinity heparin column (1 mL HiTrap Heparin, Sigma) previously equilibrated with buffer (150 mM NaCl, 20 mM Tris pH 8), at a speed of 1 mL/min as described. The genomic titer of the viral solutions was determined by real-time PCR (light cycler, Roche diagnostics, Mannheim, Germany).

**Stereotactic injections**. AAV vectors were injected bilaterally into the adult hippocampus at 4 sites per hemisphere. For each hemisphere a total volume of 4 µl was injected of a virus solution containing 7 x 10⁹ virus particles /ml. The coordinates used for each hemisphere were 1) AP 5.8 mm from the lambda; ML 1.0 mm from the sagittal suture and DV 4.2 mm from the bregma, 2) AP 4.4 mm from the lambda; ML 2.9 mm from the sagittal suture and DV 3.9 mm from the bregma, 3) AP 3.8 mm from the lambda; ML 4.0 mm from the sagittal suture and DV 3.8 mm from the bregma, 4) AP 3.2 mm from the lambda; ML 4.6 mm from the sagittal suture and DV 6.2 mm from the bregma. Vector delivery was performed with a microprocessor controlled mini pump (World Precision Instruments, Sarasota, FA, USA) with 34xG beveled needles (World Instruments) in a stereotaxic frame (Kopf Instruments, Tujunga, CA, USA). One group (n=20) received the KIBRA knock down virus, control rats (n=20) were injected with equivalent volume of AAV-eGFP empty.

### Learning and memory testing

**Open field.** The open field consists of an opaque PVC box (90x90x90 cm³). To evaluate locomotor activity the arena was divided into 4x4 equal squares and the 4 inner squares were defined as the center. For testing the rat is placed in the lower right corner of the box facing the wall. Exploration is analyzed automatically for 5 min using SYGNIS Tracker software. Time and pathlength in the center and in the periphery is calculated.

**Morris water maze (MWM).** The water maze is a circular water tank (diameter 1.70 m, depth 0.60 m) partially filled with water. Non toxic colour is added to the water preventing the animals from seeing a submerged transparent plexiglas platform that is placed slightly below the water. The platform is left in this position for the duration of the experiment. External visual cues are placed in the surrounding environment of the water maze. These cues are visible from the pool and serve as spatial orientation for the rats. A camera placed over the center of the pool connected to a video recorder tracks the behavior of the animals. To ensure that the rats learned the spatial task efficiently they first performed a non-spatial pretraining where the platform is made visible by placing cues directly over the platform. During non-spatial training cues positioned outside the maze are hidden by surrounding the pool with black curtains. In the spatial training phase rats have 4 trials per day, one trial has a maximal time of 60 s. Animals were released from the SW, S, SO and O points of the pool in a randomized but balanced manner such that the start point frequency and the distance to the platform were distributed equally across the test. After climbing onto the platform, rats are allowed to remain on the platform for 30 s before being returned to the cage for a 30 s inter-trial interval. In cases where the rat was unable to find the platform, it was guided by the experimenter to the target and was allowed to remain there for 30 s. After training animals underwent a probe trial on the 5^{th} day in which the platform was removed and animals allowed to search for the platform for 120 s. Latency and pathlength to find the platform are calculated using proprietary software. Additional parameters measuring the proximity (time and distance in the area of the platform) are calculated.

**Radial 8-arm maze.** The maze consisted of 8 equally spaced arms radiating from a central platform, which the rat has to enter in order to retrieve a food pellet placed in 4 of the arms. The central platform was 25 cm in diameter, each arm measured 50 cm x 10 cm. Walls consisted of clear plexiglas walls with 20 cm height. The rat is randomly placed in one of the unbaited arms (random start arm) and is allowed to enter the baited arms to pick up the pellet. The animal will acquire a working memory relating to arms that were baited as well as arms already entered. In addition, the animal will learn to avoid unbaited arms during the testing phase (reference memory). Testing time was 300 s with 3 trials per day over 3 weeks (15 days). Animals were food deprived for the test until a 10% drop in intial bdy weight was reached, and were kept at that weight during the trial. Correct choices and errors were recorded using proprietary analysis software.

**Electroporation of COS1 cells.** COS1 cells were cultured in Dulbecco's modified eagle medium (DMEM) high glucose supplemented with 10% fetal calf serum (FCS). Cells were transfected with appropriate plasmids as indicated in the result section. For electroporation, COS-1 cells were harvested and resuspended in electroporation (EP)-buffer (50 mM K₂HPO₄, 20 mM CH₃COOK, 20 mM KOH, pH 7.4) at a densitiy of 3.5x10⁶ cells per transfection. After addition of 25 mM MgSO₄, cells were mixed with 10 µg of plasmid-DNA (ratio of pulled down plasmid to co-expressed plasmid was 1:4; pure co-expression of two plasmids was performed in a ratio of 1:2) and transferred to an electroporation cuvette. Electroporation was performed in a Biorad Gene Pulsar II electroporator at 500 µF capacitance and 230 V, and cells were transferred into DMEM supplemented with 10% FCS and plated immediately. 48 h after transfection, cells were harvested for Co-IP. To perform western blot analysis, cells were stimulated 24 h after transfection for the indicated time points with cycloheximide and MG-132, respectively.

**Culture of primary hippocampal cultures.** Ten to twelve hippocampi were dissected from Wistar rat embryos E18. The tissue was dissociated using 10 mg/ml trypsin (Chemicon, Hofheim, Germany), 5 mg/ml EDTA/DNAse (Roche Diagnostics, Mannheim, Germany) in HBSS (BioWhitakker). The digestion was stopped after 20 min using MEM without L-Glutamine (Invitrogen, Karlsruhe, Germany) supplemented with 10% FCS, 0.45% D(+)-Glucose, 1 mM sodium pyruvate, 0.25 µM glutamate and 50000 U Pen/Strep. Cells were plated at a density of 10000 cells per well of a 24-well-plate on glass cover slips coated with poly-L-lysine. Following an incubation of three hours, the medium was replaced by neurobasalmedium containing 1xB27 supplement (Invitrogen, Karlsruhe, Germany) mixed with 50% (final concentration) conditioned medium. For viral infection, cells were cultured for 14 DIV and then virus was given to the culture. After another seven days, primary neurons were harvested for RNA preparation or protein extraction.

**Immunocytochemistry.** After two weeks of culture virus was given and another week of culture followed. After 21DIV the cells were fixed in Histofix (Roth, Karlsruhe, Germany) and stored in PBS at 4 °C until processed. Spines were labeled by F-actin staining using Phalloidin-TRITC (1:200; Sigma-Aldrich, Taufkirchen, Germany) and nuclei were visualized by DAPI (1:10.000) staining. Coverslips were embedded on slides in a blinded manner.

**Microscopy.** Using a LEICA confocal LASER scanning microscope images were taken with 63x Oil-Objective and digital zoom frames of 7x were set. Two images per cell were analyzed and areas close to the soma including the first bifurcation were chosen. Images for EGFP and TRITC-Emission were taken blinded under the same settings for one group of pictures.

**Data analysis.** The analysis was taken place blinded. Using the ImageJ software EGFP-positive processes were traced for their length using the NeuronJ plugin. The amount of spines belonging to those processes was calculated with ImageJ's Particle Analyze-tool. The images showing the TRITC labeling of spines were analyzed by the following procedure: The brightness for all pictures in one set was adjusted to the same level. The image was then transformed into a binary picture and fused particles were separated using the "watershed" tool. All particles were counted (= No of spines). The total Number of spines was related to the total length of processes in each picture. After unblinding the measurements groups were compared and t-Tests undertaken.

**Western blotting.** Cells were washed once with icecold PBS and scraped off the plate after adding lysis buffer (50 mM Tris/HCl pH 7.4, 150 mM NaCl, 1 mM EDTA, 1% NP40, 0.2 mM PMSF, Protease Inhibitor Cocktail (Roche Diagnostics, Mannheim, Germany)). Lysates were frozen in liquid nitrogen and stored afterwards at -20°C. After syringing the lysate 5x, lysate was spun down at 13000 rpm at 4 °C for 10 min and the protein concentration of the supernate was determined (BCA-Test, Pierce). 1/5 volume of 5x sample-buffer (with β-Mercaptoethanol) was added and samples were denatured at 95 °C for 5 min. 50 µg of protein was run on a SDS-PAGE and proteins were transferred to nitrocellulose membranes using an iBlot™ Dry Blotting System (Invitrogen, Karlsruhe, Germany). Blots were blocked with 5% milk powder in PBS/ 0.2% Tween20, washed three times with PBS/ 0.2% Tween20, and incubated with the primary antibody (anti-flag 1:10000, mouse monoclonal (Sigma, Taufkirchen, Germany); anti-V5 1:5000, mouse monoclonal (Sigma, Taufkirchen, Germany); anti-Rock1 1:250, rabbit polyclonal, CellSignaling (NEB, Frankfurt, Germany); anti-Rock2 1:1000, rabbit polyclonal, CellSignaling (NEB, Frankfurt, Germany); anti- p190 RhoAGAP 1:1000, rabbit polyclonal, CellSignaling (NEB, Frankfurt, Germany); anti-CamKII, 1:500 rabbit polyclonal, CellSignaling (NEB, Frankfurt, Germany); anti-RhoA (67B9) 1:1000, rabbit polyclonal, CellSignaling (NEB, Frankfurt, Germany); anti-Actin 1:10 000, mouse monoclonal (Millipore, Schwalbach, Germany); anti-PKC zeta (C-20) 1:200, rabbit polyclonal (Santa Cruz, Heidelberg, Germany); p-PKCζ-T410 1:200, rabbit polyclonal (Santa Cruz, Heidelberg, Germany) over night at 4°C. After washing, the blots were incubated with the secondary antibody (anti-rabbit- or anti-mouse-HRP-conjugatedantiserum) for 1 h at room temperature. Signals were detected using the supersignal chemiluminescence system (Pierce, Rockford, IL, USA) and exposed to CL-Xposure film (Pierce). For quantification of scanned autoradiographs Windows ImageJ (NIH, Bethesda, MD,USA) was used.

**Co-immunoprecipitation.** 2d after electroporation, cells were washed twice with ice-cold 1xPBS, and lyesd (50 mM Tris/HCl pH 7.4, 150 mM NaCl, 0.5% NP40, 20 mM NaF, 2 mM EDTA, 2 mM EGTA, 2 mM orthovanadate, protease inhibitor cocktail) at 4°C. Lysate was incubated with anti-flag beads (Sigma) at 4 °C for 2h, 5µl of peptide solution was added as indicated. Protein was released by 1x SDS loading buffer (without reducing reagents) (95 °C for 5min).

In more detail, 2d after electroporation, COS cells were washed twice with ice-cold IxPBS, and protein extraction was carried out by lysis of cells with lysis-buffer (50 mM Tris/HCl pH 7.4, 150 mM NaCl, 0.5% NP40, 20 mM NaF, 2 mM EDTA, 2 mM EGTA, 2 mM orthovanadate, protease inhibitor cocktail) at 4 °C. After incubation for 20 min at 4°C, the lysate was spun down at 13500 rpm at 4 °C for 10 min. One part of the supernatant was kept for direct Western analysis (lysate). The other part was incubated with prepared anti-flag beads (ANTI-FLAG M2 Affinity Gel, Sigma, Taufkirchen, Germany) at 4 °C for 2 h. After precipitation of the anti-flag-antigen-complex, the beads were washed 3x with TBS (50 mM Tris/HCl, 150 mM Nacl). To release the flag-bound-complex from the beads, 1x SDS loading buffer (without reducing reagents) was added and incubated at 95°C for 5min. After spinning down the beads, supernatant (IP) was analyzed by performing SDS-PAGE and transferring proteins onto nitrocellulose membranes (iBlot^{TM} Dry Blotting System, Invitrogen, Carlsbad, CA, USA). Blots were blocked with 5% milk powder and incubated overnight at 4 °C with the primary antibody (anti-flag M2 Monoclonal Antibody, 1:10000 (Sigma, Taufkirchen, Germany) and anti-V5 Antibody, 1:5000 (Sigma, Taufkirchen, Germany), respectively). After washing, the blots were incubated with the secondary antibody (anti-mouse antiserum HRP-coupled, 1:8000 (Dianova, Hamburg, Germany) for 1h at room temperature. Signals were detected using the supersignal chemiluminescence system (Pierce, Rockford, IL, USA) and exposed to CL-Xposure film (Pierce, Rockford, IL, USA)

**Quantitative PCR.** KIBRA-overexpressing primary neuronal cells were harvested 5 d after transduction of KIBRA by AAV for RNA preparation using the Qiagen RNeasy Mini Kit (Qiagen, Hilden, Germany) following the manufacturers recommendations cDNA was synthesized from 2 µg total RNA using oligo-dT primers and superscript II reverse transcriptase (Invitrogen, Karlsruhe, Germany) according to standard protocols. Quantitative RT-PCR was performed using the Lightcycler system (Roche Diagnostics, Mannheim, Germany) with SYBR-Green staining of double-stranded DNA. The following primer pairs were used: KIBRA sp-1 2982mm "GAA GGA GCT GAA GGA GCA TTT" (SEQ ID NO: 52), KIBRA asp-1 3219mm "CCT GAA AGA CTG CAC TTC TGG" (SEQ ID NO: 53); PKC zeta 5'fwd "CGC TCAC CCTC AAG TGG GTG GAC AG" (SEQ ID NO: 54), PKC zeta and PKM zeta 3'rev "GGC TTG GAA GAG GTG GCC GTT GG" (SEQ ID NO: 55); PKM zeta 5'fwd "CCA CCC GGG CCT GGA GAC ATG" (SEQ ID NO: 56). Cycling conditions were as follows: 10min at 95°C; 5s at 95°C, 10s at 60°C, 30s at 72°C, and 10s at 84 for 45 cycles for KIBRA, and 10min at 95°C; 5s at 95°C, 10s at 67°C, 30s at 72°C, and 10s at 83 for 45 cycles for PKC/M zeta. Melting curves were determined using the following parameters: 95 °C cooling to 50 °C; ramping to 99 °C at 0.2 °C/ sec. Specificity of product was ensured by melting point analysis and agarose gel electrophoresis cDNA content of samples was normalized to the expression level of Cyclophilin (primers: ,,cyc5" ACC CCA CCG TGT TCT TCG AC (SEQ ID NO: 57); "acyc300" CAT TTG CCA TGG ACA AGA TG (SEQ ID NO: 58)) . Relative regulation levels were derived after normalization to native cortical neurons.

**Proteasome activity assay.** For assaying the influence of KIBRA on proteasome activity in COS1 cells, a 20S Proteasome activity assay (#APT280, Millipore, Germany) was used. In this assay, a labeled substrate (LLVY-7-Amino-4-methylcoumarin (AMC)) is cleaved and fluorescence of the free AMC fluorophore can be quantified using a 380/ 460 nm filter set. COS cells were transfected with pExp_nV5_KIBRA_hu, pExp_NterFlag_PKMzeta_hu or with the respective V5-/Flag- control-plasmids. 2d after transfection, cells were harvested for the assay using a lysis-buffer containing 50 mM HEPES (pH 7.5), 5 mM EDTA, 150 mM NaCl and 1% Triton X-100. Cell extraction and proteasome activity assay were performed according to the manufacturer's instructions, including a proteasome positive control and Lactacystin as proteasome inhibitor. Fluorescence data was collected by using a BMG FLUOstar plate reader (BMG, Ortenberg, Germany) using 340 nm excitation and 450 nm emission filters.

**Kinase assays.** CREB phosphorylation assays were performed using the SignaTECT Protein Kinase C Assay System from Promega and 33^{P}-ATP (10 µCi /µl) provided by Perkin Elmer following the manufacturer's instructions. Briefly, after expression in COS cells with or without co-expression of KIBRA, FLAG-tagged PKC zeta was harvested as described under "Co-immunoprecipitation" in lysis-buffer containing 1% NP40. Harvested protein samples were quantified by Western blotting, and equal amounts were applied in the phosphorylation assay. Bead suspensions were diluted 1:2 in 0.1 mg/ml BSA / 0.05% Triton. 5 µl of bead-coupled protein or 9 ng of recombinant PKC zeta provided by Upstate / Millipore (positive control) and added to 20 µl PKC reaction mix containing 1x PKC activation buffer, 1.2 µM biotinylated CREB peptide substrate, 0.1 mM ATP and 1 µM 33P yATP (1 µCi/ sample). Samples were incubated for 5 minutes at 30°C. The reaction was stopped with termination buffer as supplied and 10 µl of terminated reactions were spotted onto the SAM membrane. Membranes were washed as described in 200 mM NaCl and 2M NaCl / 1% H₃P0₄ and aqua dest.. Analysis was performed by phosphoimaging (Fuji scanner FLA 2000).

**Statistics.** Two- or more group comparisons were done using ttest or ANOVA. Time series were analyzed using regression analyses. Analyses were done with JMP 8.02 (SAS Institute), and p-values < 0.05 were considered significant.

### Example 2:

As described above, PKM zeta is identical to the C-terminal half of the atypical Protein kinase C ζ (PKC zeta) lacking its N-terminal auto-inhibitory domain and generated by an independent promoter within the gene *(Hernandez et al., 2003).* PKM zeta mRNA is stored locally in dendrites and translated after sufficient synaptic stimulation *(Osten et al., 1996; Muslimov et al., 2004).* PKM zeta is constitutively active after phosphorylation by PDK1 *(Kelly et al., 2007).* The present inventors initially confirmed interaction of PKM zeta and PKC zeta with KIBRA in pulldown assays from transfected COS cells **(****Fig. 1a****).** The binding was very robust and reproducible under multiple lysis conditions and detergents (not shown). This interaction was also relevant in a cellular context as co-expression of a myristoylated PKC zeta redirected a KIBRA-GFP fusion protein completely towards the cell membrane **(****Fig. 1b****).** The present inventors have previously demonstrated that PKM zeta can phosphorylate - at least in vitro - two serine residues near the KIBRA C-terminus *(Büther et al., 2004).* Mutating those residues to alanine or glutamate had no influence on the interaction observed **(****Fig. 6a****).**

The very stable nature of the interaction and the absence of any influence of phosphorylation at sites in KIBRA suggests that the nature of the interaction is not primarily a kinase-target interaction. During co-expression experiments the present inventors noted that PKC zeta and PKM zeta protein levels increased dramatically in presence of KIBRA **(****Fig. 1c****).** Interestingly, expression of PKM zeta is much weaker than PKC zeta expressed from the same plasmid vector. This increase in protein levels could also be observed in endogenously expressed PKC zeta in primary cortical neurons infected with a KIBRA-overexpressing adeno-associated virus (AAV1/2) **(****Figure 1d****).** The effect of KIBRA on PKC/PKM zeta levels appeared specific, as levels of a number of other protein kinases overexpressed in COS cells were not influenced, such as CamKII, Rock1 or Rock 2 **(****Figs. 1e****-f).**

### Example 3:

mRNA levels of PKM zeta were monitored to determine whether this enhancement in levels was caused by influences of KIBRA on the mRNA level, for example on mRNA stability. Endogenous PKC zeta or PKM zeta mRNA levels were not influenced by increased presence of KIBRA in primary cortical neurons (Fig. 2a) or in other cells (not shown). The present inventors therefore asked whether KIBRA presence led to a stabilization of PKM zeta on the protein level. Expression of PKM zeta in the presence of the protein translation inhibitor cycloheximide (CHX) in COS cells led to a rapid decrease of the kinase levels within 48 h **(****Fig. 2b****),** while co-expression of KIBRA stabilized kinase levels, suggesting that KIBRA interferes with protein degradation of PKM zeta. PKM zeta levels were dependent on input KIBRA amounts in an approximately linear relationship **(****Fig. 2c****).** The degradation appeared proteasome-mediated, as PKM zeta was ubiquitinylated in the presence of overexpressed ubiquitin **(****Fig. 2d****),** or by endogenous ubiquitin after inhibition of proteasomal degradation by MG-132 exposure of the cells **(****Fig. 2e****).** Moreover, treatment of COS or SHSY-5Y cells with the proteasome inhibitor MG132 prevented the time-dependent decrease in kinase levels after blocking further translation of PKM zeta mRNA by cycloheximide in **(****Fig. 2f,g**), while lysosomal inhibitors had no effect (not shown). To determine whether KIBRA potentially acts as a more general inhibitor of proteasome activity proteasome activity assays were performed that did not show any influence of KIBRA nor PKM zeta presence on proteasome activity **(****Fig. 2h****).**

### Example 4:

The constitutive kinase activity of PKM zeta is thought to be crucial in its role in maintaining long-term memory storage *(Sacktor 2008).* The present inventors therefore asked whether binding to KIBRA altered PKC/ PKM zeta kinase activity. PKC zeta was immunoprecipitated from COS cells in absence or presence of KIBRA, and phosphorylation of a CREB target peptide was determined by a radioactive filter assay in comparison to in vitro synthesized and purified PKC zeta. Equal amounts of PKC zeta determined by Western blot were entered into the assay. Presence of KIBRA did not decrease the kinase activity of PKC zeta **(****Fig. 3a****).** Next, it was determined whether activation status of PKM zeta influenced binding to KIBRA. Mutating the activation loop Threonin to Alanin at position 227, the site phosphorylated by PDK as the only required activation step (corresponding to position 410 in PKC zeta) (Le Good, Ziegler et al. 1998), abolished binding to KIBRA **(****Fig. 3b****).** Notably, basal expression of this mutant kinase was very weak, suggesting that inactive PKM zeta is subject to enhanced degradation. Likewise, mutating the highly conserved lysine residue responsible for orientation of the α- and (3-phosphates of ATP (K98W) also essential for kinase activity resulted in a highly unstable protein that did not bind KIBRA **(****Fig. 3c****).** Finally, deleting the C-terminal stretch of the kinase (Δ371-409) containing the turn motif autophosphorylation site (T377) and the hydrophobic motif (392-397) necessary for kinase activity *(Steinberg 2008)* fully abolished KIBRA binding, while both wt and PKM zeta (Δ404-409) did bind (Fig. 3d). The present inventors consider that previous activation by PDK1 may be important for KIBRA to bind and protect PKM zeta from degradation, and that the bound kinase retains its activity towards other proteins. PDK1 itself is not contained in the complex, fitting to the assumption of a prior phosphorylation event and subsequent KIBRA binding **(****Fig. 6b****).**

### Example 5:

Crucial for a role as long-term stabilizer of PKM zeta would be that KIBRA itself is highly stable. Indeed, KIBRA amounts remain stable after 48 h CHX exposure of transfected cells **(****Fig. 6c****).** Next, the binding motif(s) in KIBRA responsible for binding were determined. By deletion series, it was determined that a region in the C-terminal third was important for binding (data not shown). Fine deletion mapping indicated amino acids 946 - 985 as important **(****Fig. 4a****).** EGFP-fusion of different stretches within that region indicated a 20 aa motif (PPFVRNSLERRSVRMKRPSS (aa 956 - 975), SEQ ID NO: 22) is sufficient for PKM zeta binding **(****Fig. 4b****).** Further deletion mapping indicates that the absolute minimal binding motif is from position 958 to 970, however, with some loss of binding activity compared to the 20mer sequence **(****Fig. 4c****).** An alanine scan from position 964 to 974 showed that the arginine at position 965 is very important for binding within the binding motif, while all other single point mutants retained binding activity to different degrees (wherein the amino acid residues at positions 967 and 969 also appear to be somewhat important for binding) **(****Fig. 4d****).** A synthetic peptide containing the binding motif (from aa 948-978) was also sufficient for binding **(****Fig. 4e****),** and was able to displace KIBRA from a pre-formed complex with PKM zeta in a concentration-dependent manner **(****Fig. 4f****).** Most importantly, co-expressing of the EGFP-peptide-fusion construct with PKM zeta was sufficient for stabilization of the kinase **(****Fig. 4g****).**

### Example 6:

In another experiment, consequences of reducing KIBRA expression in the rat hippocampus by AAV-mediated siRNA delivery were studied siRNA-treatment produced an overall reduction of KIBRA expression by almost 50% on the protein level **(****Fig.** 7). The initial open field test revealed no significant differences between siRNA-treated and control animals in parameters such as overall distance travelled **(****Fig. 5a****),** mean velocity, or time or distance spent in center **(****Fig. 5b****).** In the Morris water maze, the acquisition phase over 16 trials using random choices of 4 start positions yielded similar performances of both groups with a slight disadvantage for the siRNA group that resulted in the same run time reached at trial 16 **(****Fig. 5c****).** However, animals with downregulated KIBRA levels in the hippocampus demonstrated significantly worse performance in the recall of the platform location after 24 h, both analyzed for cumulative distance to platform location (p = 0.008) or for time spent in the target area defined as a circle around the original platform center with a radius 4 times larger than the original platform (p = 0.014, **Fig. 5d****).** As a learning paradigm not associated with aversive stimuli, the radial 8-arm maze with 4 baited arms was chosen. SiRNA-treated rats displayed more working memory **(****Fig. 5e****)** and reference memory errors **(****Fig. 5f****)** than controls. Multiple regression analysis revealed that KIBRA downregulation influenced working memory performance equally over the duration of the trial, while the slope behavior of reference memory performance was changed significantly by treatment, indicating that the speed of reference memory acquisition was altered. In conclusion, KIBRA downregulation impairs spatial learning and memory performance in two paradigms.

Inhibiting PKM zeta activity is unambiguously linked to a decrease in the ability to maintain long-term memories, while a simple increase in PKM zeta levels enhances the capabilities for memory storage *(Shema et al., 2007; Serrano et al., 2008; Shema et al.,* 2011). Hippocampal PKM zeta levels in rats where KIBRA levels have been downregulated by siRNA were determined. Here, PKM zeta levels were decreased by over 50% in the hippocampi of those animals **(****Fig. 5g****).** The present inventors consider the mechanism observed in cell culture to obviously also be active in vivo, and to be sufficient to explain the influence of altered KIBRA levels on learning and memory performance.

### Example 7:

Moreover, it was shown that KIBRA overexpressed by AAV in primary neurons localizes to different spines in different amounts, suggesting preferential localization to spines in a certain, yet unknown state of plasticity (cf. **Fig. 8****).**

### Example 8:

Screening approach targeting protein stabilisation of PKM zeta. Such a screen may be performed to monitor enhanced protein stability of PKM zeta mediated by KIBRA or specific fragments of the KIBRA protein (i.e. KIBRA peptides or derivatives thereof) or any other test compound. For example, KIBRA or the levels of its fragments may be elevated by co-expression, stimulation with specific, KIBRA inducing compounds or oligopeptides that enter the cells. To measure PKM zeta protein levels, PKM zeta may be expressed under control of a minimal promoter (or a CMV-promoter) as a fusion protein with a reporter protein, such as luciferase, or PKM zeta will be expressed with a small peptide tag, such as the flag-,V5 or myc-epitope. Alternatively cells are transfected with a PKM zeta-reporter construct only.

To monitor stabilization of PKM zeta by KIBRA, KIBRA peptides, peptide derivatives or peptide conjugates, or any other test compounds, e.g. SH-SY5Y cells (or other immortalized cell lines such as COS- or HEK293-cells) may be transfected with a plasmid containing the ORF of PKM zeta. In such assays, cells may for example be co-transfected with a plasmid harboring the ORF of KIBRA or a fragment etc. thereof, or another test compound may be introduced. To determine the impact on degradation of the PKM zeta protein, cells may be treated 1d after transfection with cycloheximide (CHX, 10µM) for e.g. 24 h and 48 h to inhibit de novo protein synthesis. Alternatively cells may be transfected with a PKM zeta-reporter construct only. E.g. 1d after transfection KIBRA peptides, substances of interest, test compounds or the like are transferred / introduced into the cells. De novo protein synthesis may be stopped on day 2 after transfection / introduction using e.g. cycloheximide. If a reporter such as luciferase is to be detected, a luciferase assay using a plate reader will be employed. For detecting the small peptide tag, such as the flag-, V5- or myc-epitope, an ELISA can be performed using an antibody targeted against the tag for capturing PKM zeta. Detection of the kinase may be done using an anti-PKC/M zeta antibody. Read-outs can be statistically analyzed, and hits identified. In detail, in a particular assay, 15.000 COS-1 cells per well were seeded in 96 well plates. One day after seeding, cells were transfected using Lipofectamine2000 (Invitrogen) following the recommended instructions with the following plasmids: flagPKM zeta-ORF under control of a minimum promoter (pTAmin) and CMV-promoter driven KIBRA- or LacZ-ORF or flagPKM zeta-ORF under control of a minimum promoter (pTAmin) and CMV-promoter driven expression of EGFP-fusions of KIBRA protein fragments and mutations thereof (amino acids 946-965 or 956-975 of human KIBRA-protein and e.g. single mutation of amino acid R965 to A965 in the fragment 956-975). Cells were treated with cycloheximide (CHX, 10µM). 0 h, 24 h and 48 h after stimulation, cells lysates were prepared and frozen until processed further. For cell lysis medium was removed and cells were washed in 1xPBS, pH 7,6. 50µl lysis buffer was added (1%Triton X100, 50 mM Tris/HCl, pH 7,6, 150 mM NaCl, 1 mM EDTA, PMSF (1:1000) and incubated for 5 minutes. Lysates of two wells were pooled. 50µl of cell lysate was added per well of a precoated ANTI-FLAG High Sensitivity 96-well plate (SIGMA-Aldrich) and incubated at 4°C, over night. Plates were washed 3 times with 1xPBS/0,5% Tween-20, then the detection antibody was added (rb-a-PKC zeta, C20 (Santa Cruz), 1:3000 in 1xPBS/0,5% Tween-20/ 3%Milkpowder) and incubated for 1,5h at room temperature. Plates were then washed as above and incubation with HRP-linked anti rabbit Antibody (1:1000 in 1xPBS/0,5% Tween-20/ 3%Milkpowder) followed (45min, room temperature). Plates again were washed as above and 100µl TMB-Substrate () was added. After 15min at room temperature the reaction was stopped using 2N H₂S0₄.

Optical densities were measured at 450 nm using a Fluostar plate reader. Background levels of untransfected cells were subtracted from read out values and PKM zeta stability was calculated comparing the OD450 nm at 0 h, 24 h and 48 h after CHX treatment.

### Example 9:

On the basis of the findings of the present inventions, it may be screened for molecules (such as small molecules or oligopeptides) that have the ability to bind to PKM zeta at the position where binding of a peptide disclosed herein interferes with degradation of the kinase - and to thus identify compounds capable of inhibiting degradation of PKM zeta. Such screen can yield molecules with similar or higher affinity to PKM zeta than native KIBRA or a peptide of the invention. These molecules can be further derivatized and optimized for medical uses.

To this end, human KIBRA or peptide fragments, such as a 20mer containing amino acids 956-975 of KIBRA, and PKM zeta may be expressed in SHSY5Y-, COS- or HEK293-cells (or other immortalized cell lines, or primary cells such as neurons) with different labels (e.g. EGFP or luciferase or tag sequences such as flag-, myc- or V5-tag). During the assay, test compounds such as small molecules are added to the KIBRA peptide and PKM zeta to compete with their interaction. In one implementation of the assay a small molecule library may be screened for binders that displace the native KIBRA or KIBRA peptide, such as the 20mer. In detail, a flag-tagged PKM zeta construct may be overexpressed in a cell system (like e.g. COS, HEK, SHSY5Y), immunoprecipitated using flag-beads (e.g. agarose beads) under stringent conditions, released from the beads, and bound to ELISA plates harbouring an immobilized anti- FLAG -antibody. Alternatively, PKM zeta generated in a cell-free system may be used (e.g. from a reticulocyte or wheat germ lysate translation system). E.g. a biotinylated KIBRA or KIBRA peptide may then be added to the system, and will bind after incubation of a short time period (e.g. 1 h) to PKM zeta. The test compound (or e.g. small molecule library) is then added in concentrations of typically 1 or 10 µM. After incubation of a short time period, the ELISA plates can be washed under relatively mild conditions, and the label (here: biotin moiety) of KIBRA or the KIBRA peptide is detected e.g. using Streptavidin coupled to a fluorophore. The fluorophore intensity may be quantitated in an adequate reader, and wells will be identified with a significantly diminished fluorophore intensity, indicating displacement of the KIBRA or KIBRA peptide by the respective test compound (such as a small molecule). Instead of using a small molecule library, a peptide library or the like may be used. In another particular example of this assay, flag-tagged PKM zeta and a KIBRA peptide luciferase fusion protein may be expressed. These two proteins will form a complex within the cells, and this complex will be isolated by immunoprecipitation using flag-beads. After this step, the above procedure can be used without the need for adding KIBRA peptide. The readout system may be more versatile in this approach, as either luciferase, or a protein-encoded fluorophore such as EGFP or YGFP may be used. If the test compound, such as a small molecule, carries a tag itself, this may be measured along the lines to KIBRA-detection, as well. An increase of the signal will give information if the molecule itself binds PKM zeta.

### Example 10:

To obtain / arrive at further peptide derivatives of the peptides described herein or compounds capable of inhibiting degradation of PKM zeta, a phage display library may be employed (e.g. available form New England Biolabs, Kit Components: Ph.D.^{TM}-12 Phage Display Peptide Library, E. coli K12 ER2738, Biotin, Streptavidin, lyophilized). A common vector used in such assay is the bacteriophage M13. The phage library displays random peptide sequences of seven or 12 amino acids, where also longer sequences may be used. The randomized peptide sequences in the NEB libraries are expressed at the N-terminus of the minor coat protein pIII, resulting in a valency of five copies of the displayed peptide per virion. In this approach, PKM zeta may again be immobilized in wells, and the phages may be added to the well. Phages with appropriate sequences would bind, and remain bound after the washing step. Bound phages are then eluted, a suitable bacterial host (such as *Escherichia coli* bacterial cells, including e.g. as TG1, SS320, ER2738, or XI,1-Blue) is infected, and phages are amplified. After e.g. three washing and enrichment steps (often called "panning"), the sequence preference can be determined when sequencing the DNA of all remaining phages. Preferably, a modified assay may test for displacement of KIBRA or of a KIBRA peptide of the invention, to avoid the use of binders not related to the KIBRA-interacting site on PKM zeta. Also, in a similar fashion phage libraries can be employed that present antibody structures on their surface in order to identify antibodies that could displace the KIBRA peptide. In accordance with such assays, it is considered feasible to obtain / arrive at peptide derivatives which can potentially be improved in binding affinity and thus protection of PKM zeta from degradation. In addition, optimized peptides for medical use may be derived from the most preferred sequence identified.

### Example 11:

Screening approach targeting ubiquitination of PKMzeta: As PKM zeta is degraded by the Ubiquitin-Proteasome-System (UPS), one approach to screen for substances that influence the degradation of the kinase is to determine the amount of ubiquitinated PKM zeta protein. To this end, the kinase may be expressed as a fusion protein with a small peptide tag, such as the flag- or myc-epitope. SHSY5Y cells (or other immortalized cell lines such as COS- or HEK293-cells) may be co-transfected with a plasmid containing the ORF of PKM zeta and with a plasmid harboring the ORF of KIBRA or a fragment thereof, or another test compound may be introduced. Expression may occur under control of a CMV-promoter or a minimal promoter. 1d after transfection, cells may be treated with MG-132 (5 µM) for a few hours to inhibit proteasomal degradation and to enrich ubiquitinated proteins. An ELISA may be performed capturing PKM zeta with an antibody targeted against the small peptide tag. The amount of ubiquitinated PKM zeta will be determined by monitoring ubiquitin with an anti-ubiquitin antibody. Alternatively, an ELISA will be performed where a plasmid harboring the V5-tagged ubiquitin may be co-transfected with the PKM zeta- and KIBRA-fragment-expressing plasmids described before. 1d after transfection, PKM zeta capturing will be done with an antibody targeted against the small peptide tag. For quantification of ubiquitination, an anti-V5-antibody will be used.

### Example 12:

Screening approach using FRET: Another approach to investigate ubiquitination of PKM zeta and possible inhibition of this process, is to monitor ubiquitination in vivo by FRET (Fluorescence Resonance Energy Transfer) using multiphoton fluorescence lifetime imaging microscopy (FLIM) technique. The FLIM-FRET technique allows the characterization of the spatial organization and quantification of protein interactions in living cells. Binding of two proteins (one harbouring a donor fluorophore and the other fused to an acceptor fluorophore) leads to the transfer of energy as the two molecules are brought into close proximity. Energy transfer is detectable by a decreased donor fluorescence lifetime. PKM zeta may be expressed as a fusion protein with a reporter tag, such as EGFP (donor fluorophore), and ubiquitin may be expressed fused to an acceptor fluorophore such as mcherry. Other examples of fluorophore tags that can function as FRET pair are CFP (donor) and YFP (acceptor). FRET can likewise occur between combinations of GFPs and synthetic dyes such as Cy3 and Cy5. SHSY5Y-, COS- or HEK293-cells (or other immortalized cell lines or primary cells such as hippocampal neurons) may be transfected with a plasmid containing the ORF of PKM zeta fused to a donor fluorophore and with a plasmid harbouring the ORF of ubiquitin fused to an acceptor fluorophore. To analyze the impact of KIBRA (or any test compound) on the ubiquitination of PKM zeta, e.g. a plasmid containing the ORF of KIBRA or fragments thereof may be co-transfected with the PKM zeta- and ubiquitin-fluorophore-fusion plasmids (or the test compound may be introduced). 1d after transfection, fluorescent signals occurring after ubiquitination of PKM zeta may be measured with excitation at 488 nm (GFP) and time-resolved fluorescence at the emission wavelength of mcherry at 610 nm (measurements will be done for the other FRET-pairs such as CFP and YFP with the respective wavelengths required for excitation and emission). Potential inhibitors of the ubiquitination reaction will decrease the fluorescent signal. Another possibility to monitor polyubiquitination of PKM zeta is to perform the reaction *in vitro.* In this assay, the fluorescence donor (europium chelate (Eu)) is attached to Ubiquitin, and the acceptor (allophycocyanin (APC)) may be coupled to streptavidin. Ubiquitination of biotinylated PKM zeta will occur in the presence of the enzymes E1, E2, E3, ATP and Eu-Ubiquitin. After interaction of biotin with streptavidin-APC, Eu-Ubiquitin may be brought into close proximity to APC when ubiquitination of PKM zeta occurs. Fluorescent signals may be detected during ubiquitination and after adding KIBRA-peptides to the ubiquitination reaction, inhibitors may be identified by measuring decreased fluorescence.

### Example 13:

Alternatively, e.g. for assays involving interaction of KIBRA (or of a peptide, peptide derivative of peptide conjugate of the invention) with PKM zeta, such assays may also be performed with sections of PKM zeta including the KIBRA binding site. That is, specificity of such assays might be further improved if only the part of PKM zeta is used therein that binds KIBRA or the like. With such modified assays, further compounds being able to interact with PKM zeta and/or PKC zeta may be identified. To this end, several approaches may be employed. PKM zeta can be divided in smaller protein stretches by using PCR-based subcloning. These stretches can be incubated with a KIBRA, KIBRA peptide, etc. and probed for interaction, using methods described above. In a complementary approach, the structure of PKM zeta bound to e.g. the 20mer KIBRA peptide may be determined. This may routinely be done using X-ray crystallography or nuclear magnetic resonance (NMR). Protein nuclear magnetic resonance is usually performed highly purified protein in aqueous solution. The protein concentration may e.g. be in the range 0.1 - 3 mM, in a total buffer volume of 300 to 600 µl. The NMR sample is usually prepared in a thin walled glass tube. The pure protein may be produced in an expression system such as mammalian cells (e.g. COS-cells), baculovirus, yeast (e.g. pichia), bacteria (e.g. *E.coli).* Alternatively, the protein may be produced in a cell-free system, such as reticulocyte lysate or wheat germ extract. The protein may commonly be labelled with carbon-13 and nitrogen-15. NMR spectra can then be used to determine the 3D-structure of the proteins examined, including e.g. the KIBRA binding site of PKM zeta.

### Example 14:

The determination of the binding pocket in PKM zeta for the KIBRA peptide is considered to allow modeling of test compounds (such as small molecule structures) into the binding pocket that allow to mimic the protective function of the KIBRA peptide. That way, further compounds being able to interact with PKM zeta and/or PKC zeta may be identified.

### REFERENCES

Aarsland, D., K. Andersen, et al. (2001). "Risk of dementia in Parkinson's disease: a community-based, prospective study." Neurology 56(6): 730-6.
Baier, B. and H. O. Karnath (2005). "Incidence and diagnosis of anosognosia for hemiparesis revisited." J Neurol Neurosurg Psychiatry 76(3): 358-61.
Büther, K., C. Plaas, et al. (2004). "KIBRA is a novel substrate for protein kinase Czeta." Biochem Biophys Res Commun 317(3): 703-7.
Deary, I. J., J. Corley, et al. (2009). "Age-associated cognitive decline." Br Med Bull 92: 135-52.
Franich, N. R., H. L. Fitzsimons, et al. (2008). "AAV vector-mediated RNAi of mutant huntingtin expression is neuroprotective in a novel genetic rat model of Huntington's disease." Mol Ther 16(5): 947-56.
Fricker et al., Phospholipids and Lipid-Based Formulations in Oral Drug Delivery, Pharm Res (2010) 27:1469-1486; DOI 10.1007/s11095-O10-0130-x,
Galvez AS, Duran A, et al. (2009). "Protein kinase Czeta represses the interleukin-6 promoter and impairs tumorigenesis in vivo." Mol Cell Biol. Jan; 29(1):104-15. Epub 2008 Oct 27. Gauthier, S., B. Reisberg, et al. (2006). "Mild cognitive impairment." Lancet 367(9518): 1262-70.
Glinoer, D. (2000). "Potential repercussions for the progeny of maternal hypothyroxinemia during pregnancy." Thyroid 10(1): 59-62.
Haberland, C. (2010) "Frontotemporal dementia or frontotemporal lobar degenerationoverview of a group of proteinopathies." Ideggyogy Sz 63(3-4): 87-93
Hernandez, A. I., N. Blace, et al. (2003). "Protein kinase M zeta synthesis from a brain mRNA encoding an independent protein kinase C zeta catalytic domain. Implications for the molecular mechanism of memory." J Biol Chem 278(41): 40305-16.
Johannsen, S., K. Duning, et al. (2008). "Temporal-spatial expression and novel biochemical properties of the memory-related protein KIBRA." Neuroscience 155(4): 1165-73.
Karnath, Hans-Otto, "The cognitive and neural bases of spatial neglect", Oxford Univ. Press: 2002
Kelly, M. T., J. F. Crary, et al. (2007). "Regulation of protein kinase Mzeta synthesis by multiple kinases in long-term potentiation." J Neurosci 27(13): 3439-44.
Kim MS, Lim WK, et al. (2001)."The activation of PI 3-K and PKC zeta in PMA-induced differentiation of HL-60 cells". Cancer Lett. Sep 28;171(1):79-85
Klugmann, M., C. W. Symes, et al. (2005). "AAV-mediated hippocampal expression of short and long Homer 1 proteins differentially affect cognition and seizure activity in adult rats." Mol Cell Neurosci 28(2): 347-60.
Kraus LM, Sacktor TC, Francis JT (2010) Erasing Sensorimotor Memories via PKMζ Inhibition. PLoS ONE 5(6)
Le Good, J. A., W. H. Ziegler, et al. (1998). "Protein kinase C isotypes controlled by phosphoinositide 3-kinase through the protein kinase PDK1." Science 281(5385): 2042-5.
Major, E. O., D. Rausch, et al. (2000). "HIV-associated dementia." Science 288(5465): 440-2
McKeith, I., J. Mintzer, et al. (2004). "Dementia with Lewy bodies." Lancet Neurol 3(1): 19-28.
Montoya, A., B. H. Price, et al. (2006). "Brain imaging and cognitive dysfunctions in Huntington's disease." J Psychiatry Neurosci 31(1): 21-9.
Muslimov, I. A., V. Nimmrich, et al. (2004). "Dendritic transport and localization of protein kinase Mzeta mRNA: implications for molecular memory consolidation." J Biol Chem 279(50): 52613-22.
Olsen CG, Clasen ME. "Senile dementia of the Binswanger's type". Am Fam Physician. 1998 Dec;58(9):2068-74.
Osten, P., L. Valsamis, et al. (1996). "Protein synthesis-dependent formation of protein kinase Mzeta in long-term potentiation." J Neurosci 16(8): 2444-51.
Papassotiropoulos, A., D. A. Stephan, et al. (2006). "Common Kibra alleles are associated with human memory performance." Science 314(5798): 475-8.
Rhawn Joseph; "Neuropsychiatry, Neuropsychology, Clinical Neuroscience"; Academic Press, New York, 2000
Sacktor, T. C. (2008). "PKMzeta, LTP maintenance, and the dynamic molecular biology of memory storage." Prog Brain Res 169: 27-40.
Sacktor, T. C. (2011). "How does PKMzeta maintain long-term memory?" Nat Rev Neurosci 12(1): 9-15.
Sacktor, T. C., P. Osten, et al. (1993). "Persistent activation of the zeta isoform of protein kinase C in the maintenance of long-term potentiation." Proc Natl Acad Sci U S A 90(18): 8342-6.
Schneider, A., M. J. Huentelman, et al. (2010). "KIBRA: A New Gateway to Learning and Memory?" Front Aging Neurosci 2: 4.
Serrano, P., E. L. Friedman, et al. (2008). "PKMzeta maintains spatial, instrumental, and classically conditioned long-term memories." PLoS Biol 6(12): 2698-706.
Shema, R., S. Haramati, et al. (2011). "Enhancement of consolidated long-term memory by overexpression of protein kinase Mzeta in the neocortex." Science 331(6021): 1207-10. Shema, R., T. C. Sacktor, et al. (2007). "Rapid erasure of long-term memory associations in the cortex by an inhibitor of PKM zeta." Science 317(5840): 951-3.
Steinberg, S. F. (2008). "Structural basis of protein kinase C isoform function." Physiol Rev 88(4): 1341-78.
Szumlinski, K. K., K. D. Lominac, et al. (2005). "Homer2 is necessary for EtOH-induced neuroplasticity." J Neurosci 25(30): 7054-61.
Whyte J, Thornton L, et al. (2010)."PKCzeta regulates cell polarisation and proliferation restriction during mammary acinus formation." J Cell Sci. Oct 1;123(Pt 19):3316-28
Xiao, L., Y. Chen, et al. (2011). "KIBRA Regulates Hippo Signaling Activity via Interactions with Large Tumor Suppressor Kinases." J Biol Chem 286(10): 7788-96.
Yoshihama, Y., T. Hirai, et al. (2009). "KIBRA Co-localizes with protein kinase Mzeta (PKMzeta) in the mouse hippocampus." Biosci Biotechnol Biochem 73(1): 147-51.
Zeng, Y. and B. R. Cullen (2004). "Structural requirements for pre-microRNA binding and nuclear export by Exportin 5." Nucleic Acids Res 32(16): 4776-85.

## Claims

1. A method for identifying a compound capable of inhibiting degradation of PKM zeta.

2. The method of claim 1 comprising the following steps
a) providing a test system for monitoring the degradation of PKM zeta, and
b) determining whether a given test compound leads to a reduced degradation of PKM zeta in said test system.

3. The method according to claim 2, wherein the test system involves inhibiting protein translation.

4. The method of any one of claims 1 and 2 comprising a step of determining whether a given test compound is capable of interfering with the proteasomal degradation of PKM zeta.

5. The method of claim 4 comprising a step of determining whether a given test compound is capable of
i) inhibiting a E3-, E2-, El-Ligase involved in proteasomal degradation of PKM zeta; or
ii) stimulating deubiquitination of PKM zeta; or
iii) inhibiting PKM zeta transport to or entry into the proteasome; or
iv) inhibiting the assembly of the multiprotein complex involved in PKM zeta degradation.

6. The method of claim 1 comprising the step of determining whether a given test compound is capable of interfering with the binding of
a peptide or a peptide derivative thereof or a peptide conjugate of the peptide or peptide derivative
to PKM zeta and/or PKC zeta,
wherein said peptide comprises the sequence FVRNSLERRSVRM, and wherein said peptide has a sequence selected from the group consisting of:
FVRNSLERRSVRM (SEQ ID NO: 4);
PPFVRNSLERRSVRM (SEQ ID NO: 6);
DSSTLSKKPPFVRNSLERRSVRM (SEQ ID NO: 8);
DSDSSTLSKKPPFVRNSLERRSVRM (SEQ ID NO: 10);
FVRNSLERRSVRMKR (SEQ ID NO: 12);
PPFVRNSLERRSVRMKR (SEQ ID NO: 14);
DSSTLSKKPPFVRNSLERRSVRMKR (SEQ ID NO: 15);
DSDSSTLSKKPPFVRNSLERRSVRMKR (SEQ ID NO: 18);
FVRNSLERRSVRMKRPSS (SEQ ID NO: 20);
PPFVRNSLERRSVRMKRPSS (SEQ ID NO: 22);
DSSTLSKKPPFVRNSLERRSVRMKRPSS (SEQ ID NO: 24);
DSDSSTLSKKPPFVRNSLERRSVRMKRPSS (SEQ ID NO: 26);
FVRNSLERRSVRMKRPSSVKS (SEQ ID NO: 28);
PPFVRNSLERRSVRMKRPSSVKS (SEQ ID NO: 30);
DSSTLSKKPPFVRNSLERRSVRMKRPSSVKS (SEQ ID NO: 32);
DSDSSTLSKKPPFVRNSLERRSVRMKRPSSVKS (SEQ ID NO: 34);
FVRNSLERRSVRMKRPSSVKSLRSERLI (SEQ ID NO: 36);
PPFVRNSLERRSVRMKRPSSVKSLRSERLI (SEQ ID NO: 38);
DSSTLSKKPPFVRNSLERRSVRMKRPSSVKSLRSERLI (SEQ ID NO: 40);
DSDSSTLSKKPPFVRNSLERRSVRMKRPSSVKSLRSERLI (SEQ ID NO: 42);
and a sequence of 13 to 40 consecutive amino acids of SEQ ID NO: 42.

7. The method of claim 6, wherein the peptide derivative or peptide conjugate is selected from any of the below A, B, C, D and E:
A) a peptide derivative,
i) having a sequence that is at least 90 % identical to a sequence as defined in claim 6; and/or
ii) having a sequence of 13 to 200 amino acids that comprises a sequence as defined in claim 6; and/or
iii) having a sequence that is at least 70 % identical to a sequence as defined in claim 6 and being capable of binding to PKM zeta; and/or
iv) having a sequence of 13 to 500 amino acids that comprises a sequence as defined in claim 6, and being capable of binding to PKM zeta; and/or
v) having a sequence that is at least 80 % identical to a sequence as defined in claim 6 and comprising the amino acid residue E at the position corresponding to position 965 of the full-length sequence of human KIBRA (SEQ ID NO: 2) and/or
vi) having a sequence that is at least 70 % identical to a sequence as defined in claim 6 and comprising the amino acid residues E, R, V at the positions corresponding to positions 965, 967, 969 of the full-length sequence of human KIBRA (SEQ ID NO: 2) and/or
vii) having a sequence that is at least 60 % identical to a sequence as defined in claim 6, comprising the amino acid residues E, R, V at the positions corresponding to positions 965, 967, 969 of the full-length sequence of human KIBRA (SEQ ID NO: 2), and being capable of binding to PKM zeta,
B) a peptide derivative,
i) comprising D-amino acids having an inverse sequence of a sequence of a peptide as defined in claim 6 or of a sequence of peptide derivative according to A), and being capable of binding to PKM zeta; and/or
ii) being a cyclic derivative of a peptide as defined in claim 6 or of a peptide derivative according to A); and/or
iii) being a multimer of a peptide as defined in claim 6 or of a peptide derivative according to A),
C) a peptide derivative, particularly as defined in any of A) and B),
comprising one or more covalent modification(s), particularly selected from the group consisting of acetylation, amidation, disulfide bond formation, formylation, glycosylation, methylation, phosphorylation, sulfatation, replacement of a peptide bond -(C=O)NH- by -(C=S)NH- and replacement of a peptide bond -(C=O)NH- by -(CH-CF₃)NH-,
D) a peptide conjugate comprising a peptide derivative as defined in any of A), B) and C), and/or
E) a peptide conjugate, particularly as defined in D), **characterized in that** it comprises
i) a cell penetration moiety, particularly a moiety for crossing the blood brain barrier; and/or
ii) a detectable label; and/or
iii) a polymer; and/or
iv) a membrane anchoring moiety; and/or
v) a sequence interacting with the postsynaptic density, particularly with a PDZ motif.

8. The method of claim 6 or 7, wherein said compound capable of inhibiting degradation of PKM zeta is verified by means of a positive control in the presence of a peptide, peptide derivative or peptide conjugate as defined in claim 6 or 7.

9. The method according to any one of claims 1 to 8, wherein said compound capable of inhibiting degradation of PKM zeta is verified by means of a negative control in the absence of said test compound.

10. The method according to any one of claims 1 to 9, wherein said method
i) is performed *in vivo,*
ii) is performed *ex vivo* or
iii) is performed *in vitro.*

11. A compound capable of inhibiting degradation of PKM zeta, identified by a method according to any one of claims 1 to 10,
particularly wherein the compound is not KIBRA.

12. A compound of claim 11 for use in medicine.

13. A compound of claim 11 for use in a method selected from the group consisting of
i) a method for improving cognition
ii) a method for improving learning;
iii) a method for improving memory;
iv) a method for improving motor learning and/or sensory motor recovery or for treating diseases which benefit from improved motor learning and/or improved sensory motor recovery;
v) a method for treating or improving neuropsychological deficits after stroke;
vi) a method for counteracting memory loss;
vii) a method for improving or treating a memory deficit;
viii) a method for improving or treating a dementia;
ix) a method for improving or treating age-associated cognitive decline;
x) a method for improving or treating mild cognitive impairment (MCI);
xi) a method for improving or treating a brain trauma;
xii) a method for treating cancer.

14. A pharmaceutical composition comprising the compound of claim 11.

15. A method for stabilizing PKM zeta comprising a step selected from the group consisting of
i) a step of contacting PKM zeta with a compound capable of inhibiting degradation of PKM zeta;
ii) a step of contacting PKM zeta with a compound capable of interfering with the binding of a peptide, peptide derivative or peptide conjugate as defined in claim 6 or 7 to PKC zeta and/or PKM zeta;
ii*)a step of contacting PKM zeta with a peptide, peptide derivative or peptide conjugate as defined in claim 6 or 7,
iii)a step of contacting PKM zeta with a compound capable of interfering with ubiquitination of PKM zeta, particularly iii)1) a step of contacting PKM zeta with a compound capable of inhibiting a E3-, E2-, E1-Ligase involved in proteasomal degradation of PKM zeta, or iii)2) a step of contacting PKM zeta with a compound capable of stimulating deubiquitination of PKM zeta;
iv)a step of contacting PKM zeta with a compound capable of inhibiting PKM zeta transport to or entry into the proteasome
and
v) a step contacting PKM zeta with a compound capable of inhibiting the assembly of the multiprotein complex involved in PKM zeta degradation.
particularly wherein said compound has been identified by a method according to any one of claims 1 to 10,
especially wherein the compound is not KIBRA.
